(19) Europäisches Patentamt
European Patent Office
Office européen des brevets

(11) **EP 2 283 163 B1**

(12) **EUROPEAN PATENT SPECIFICATION**

(45) Date of publication and mention
of the grant of the patent:
**08.07.2015 Bulletin 2015/28**

(51) Int Cl.:
***C12Q 1/68*** (2006.01)

(21) Application number: **09755566.8**

(22) Date of filing: **17.04.2009**

(86) International application number:
**PCT/US2009/040919**

(87) International publication number:
**WO 2009/146204 (03.12.2009 Gazette 2009/49)**

(54) **POLYMORPHISMS ASSOCIATED WITH AGE-RELATED MACULAR DEGENERATION AND METHODS FOR EVALUATING PATIENT RISK**

MIT ALTERSBEDINGTER MAKULADEGENERATION ASSOZIIERTE POLYMORPHISMEN UND VERFAHREN ZUR BEWERTUNG VON PATIENTENRISIKEN

POLYMORPHISMES ASSOCIÉS À LA DÉGÉNÉRESCENCE MACULAIRE LIÉE À L ÂGE ET PROCÉDÉS D ÉVALUATION DU RISQUE POUR UN PATIENT

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **18.04.2008 US 46083 P**
**27.08.2008 US 92317 P**

(43) Date of publication of application:
**16.02.2011 Bulletin 2011/07**

(73) Proprietors:
• **Tufts Medical Center**
**Boston, MA 02111 (US)**
• **Massachusetts General Hospital**
**Boston, MA 02114 (US)**

(72) Inventors:
• **SEDDON, Johanna, M.**
**Boston, MA 02108 (US)**
• **DALY, Mark**
**Boston, MA 02114 (US)**

(74) Representative: **Murphy, Colm Damien et al**
**Venner Shipley LLP**
**200 Aldersgate**
**London EC1A 4HD (GB)**

(56) References cited:
**WO-A1-2007/044897       US-A1- 2007 020 647**
**US-B2- 7 206 438**

• **DATABASE EMBL [Online] 23 May 2007 (2007-05-23), "Macaca mulatta clone CH250-436O9, WORKING DRAFT SEQUENCE, 2 ordered pieces.", XP002648131, retrieved from EBI accession no. EM_HTG:AC204005 Database accession no. AC204005**
• **DATABASE EMBL [Online] 12 August 2002 (2002-08-12), "hd33a02.y1 Human Retina cDNA (Un-normalized, unamplified): hd/he Homo sapiens cDNA clone hd33a02 5', mRNA sequence.", XP002648132, retrieved from EBI accession no. EM_EST:BQ639229 Database accession no. BQ639229**
• **DATABASE EMBL [Online] 8 January 2005 (2005-01-08), "Bos taurus clone CH240-35C19, WORKING DRAFT SEQUENCE, 4 unordered pieces.", XP002648133, retrieved from EBI accession no. EM_HTG:AC155043 Database accession no. AC155043**
• **FAGERNESS ET AL.: "Variation near complement factor I is associated with risk of advanced AMD.", EUR J HUM GENET., vol. 17, no. 1, 6 August 2008 (2008-08-06) , pages 100-104, XP55002160,**
• **DATABASE GENBANK WISTOW ET AL.: 'Expressed sequence tag analysis of human retina for the NEIBank Project: Retbindin, an abundant, novel retinal cDNA and alternative splicing of other retina-preferred gene transcripts.', XP008145205 Database accession no. BQ639229 & MOLECULAR VISION vol. 8, 15 June 2002, pages 196 - 204**

- DATABASE NCBI 26 May 2006 'Single Nucleotide Polymorphism. Reference SNP Cluster Report: rs13117504.' Database accession no. rs13117504
- DATABASE NCBI 15 July 2002 'hd33a02.y1 Human Retina cDNA (Un-normalized, unamplified): hd/he Homo sapiens cDNA clone hd33a02 5-, mRNA sequence.', XP002648132 Database accession no. BQ639229.1.

- FAGERNESS ET AL.: 'Variation near complement factor I is associated with risk of advanced AMD.' EUR J HUM GENET. vol. 17, no. 1, 06 August 2008, pages 100 - 104, XP055002160

**Description**

GOVERNMENT SUPPORT

This invention was made with government support under EY011309 awarded by the National Institutes of Health. Additional funding was provided by the National Eye Institute (N01-EY-0-2127) and grant U54 RR020278 from the National Center for Research Resources. The government may have certain rights in the invention.

BRIEF DESCRIPTION OF THE DRAWINGS

FIG. 1 is a plot showing sensitivities and specificities for a variety of risk score cutpoints and ROC curves for prediction of advanced age-related macular degeneration among younger and older age groups.

FIG. 2 are plotted histograms for advanced age-related macular degeneration risk scores for cases and controls among the original sample (above) and replication sample (below) based on all genetic variants as well as demographic and environmental variables.

FIG. 3 are sequences showing alleles at polymorphic sites: rs2230199 (SEQ ID NO:1), rs1061170 (SEQ ID NO:2), rs10490924 (SEQ ID NO:3), rs9332739 (SEQ ID NO:4), rs641153 (SEQ ID NO:5), rs1410996 (SEQ ID NO:6) and rs2230203 (SEQ ID NO:7).

FIGS. 4.1 through 4.27 show the complement factor markers that were examined for their association with AMD.

BACKGROUND

Age-related macular degeneration (AMD) is the most common geriatric eye disorder leading to blindness. Macular degeneration is responsible for visual handicap in what is conservatively estimated to be approximately 16 million individuals worldwide. Among the elderly, the overall prevalence is estimated between 5.7% and 30% depending on the definition of early AMD, and its differentiation from features of normal aging, a distinction that remains poorly understood.

Histopathologically, the hallmark of early neovascular AMD is accumulation of extracellular drusen, basal laminar deposit (abnormal material located between the plasma membrane and basal lamina of the retinal pigment epithelium) and basal linear deposit (material located between the basal lamina of the retinal pigment epithelium and the inner collageneous zone of Bruch's membrane). The end stage of AMD is characterized by a complete degeneration of the neurosensory retina and of the underlying retinal pigment epithelium in the macular area. Advanced stages of AMD can be subdivided into geographic atrophy and exudative AMD. Geographic atrophy is characterized by progressive atrophy of the retinal pigment epithelium. In exudative AMD the key phenomenon is the occurrence of choroidal neovascularisation (CNV). Eyes with CNV have varying degrees of reduced visual acuity, depending on location, size, type and age of the neovascular lesion. The development of choroidal neovascular membranes can be considered a late complication in the natural course of the disease possibly due to tissue disruption (Bruch's membrane) and decompensation of the underlying longstanding processes of AMD.

Many pathophysiological aspects as well as vascular and environmental risk factors are associated with a progression of the disease, but little is known about the etiology of AMD itself as well as about the underlying processes of complications like the occurrence of CNV. Family, twin, segregation, and case-control studies suggest the involvement of genetic factors in the etiology of AMD. The extent of heritability, number of genes involved, and mechanisms underlying phenotypic heterogeneity, however, are unknown. The search for genes and markers related to AMD faces challenges-onset is late in life, and there is usually only one generation available for studies. The parents of patients are often deceased, and the children are too young to manifest the disease. Generally, the heredity of late-onset diseases has been difficult to estimate because of the uncertainties of the diagnosis in previous generations and the inability to diagnose AMD among the children of an affected individual. Even in the absence of the ambiguities in the diagnosis of AMD in previous generations, the late onset of the condition itself, natural death rates, and small family sizes result in underestimation of genetic forms of AMD, and in overestimation of rates of sporadic disease. Moreover, the phenotypic variability is considerable, and it is conceivable that the currently used diagnostic entity of AMD in fact represents a spectrum of underlying conditions with various genetic and environmental factors involved.

Early detection of AMD would reduce the growing societal burden due to AMD by targeting and emphasizing modifiable habits earlier in life and recommending more frequent surveillance for those highly susceptible to the disease. Treatment trials will also benefit from such information when enrolling participants. There remains, therefore, a strong need for improved methods of diagnosing or predicting AMD or a susceptibility to AMD in subjects, as well as for evaluating and developing new methods of treatment.

SUMMARY

Described herein are methods and compositions that allow for improved diagnosis of AMD and susceptibility to AMD. The compositions and methods are directed to the unexpected discovery of genetic markers and causative polymorphisms in genes associated with the complement pathway. These markers and polymorphisms are useful for diagnosing AMD or a susceptibility to AMD, for use as drug targets, for identifying therapeutic agents, and for determining the efficacy of and a subject's responsiveness to a therapeutic treatment.

One embodiment is directed to a method for diagnosing age related macular degeneration or a susceptibility to age related macular degeneration according to claim 1. The polymorphic site is a single nucleotide polymorphism (*e.g.*, selected from the group consisting of: rs13117504 (SEQ ID NO:8), wherein the guanidine allele is indicative of age related macular degeneration or susceptibility to age related macular degeneration; rs10033900 (SEQ ID NO:9), wherein the guanidine allele is indicative of age related macular degeneration or susceptibility to age related macular degeneration, and the polymorphic site of SEQ ID NO:10, wherein the guanidine allele is indicative of age related macular degeneration or susceptibility to age related macular degeneration). In a particular embodiment, the presence or absence of a particular allele is detected by a hybridization assay. In a particular embodiment, the presence or absence of a particular allele is determined using a microarray. In a particular embodiment, the presence or absence of a particular allele is determined using an antibody. In a particular embodiment, the method further comprises detecting one or more genetic markers associated with age related macular degeneration and a gene other than complement factor I (*e.g.*, C3, C5 or CFH).

One example is directed to a purified polynucleotide comprising the polymorphic site and at least about six or more contiguous nucleotides of one or more of the sequences given as SEQ ID NOS:8, 9 or 10, wherein the minor allele is present at the polymorphic site.

One example is directed to a diagnostic array comprising one or more polynucleotide probes that are complementary to a polynucleotide described herein.

One embodiment is directed to a diagnostic system comprising according to claim 8 comprising: a diagnostic array, an array reader, an image processor, a database having data records and information records, a processor, and an information output; wherein the system compiles and processes patient data and outputs information relating to the statistical probability of the patient developing AMD.

One embodiment is directed to a method of determining the risk of a subject for developing age related macular degeneration according to claim 9. The single nucleotide polymorphism is selected from the group consisting of: rs13117504 (SEQ ID NO:8), wherein the guanidine allele is indicative of age related macular degeneration or susceptibility to age related macular degeneration; rs10033900 (SEQ ID NO:9), wherein the guanidine allele is indicative of age related macular degeneration or susceptibility to age related macular degeneration, and the polymorphic site of SEQ ID NO:10, wherein the guanidine allele is indicative of age related macular degeneration or susceptibility to age related macular degeneration. In a particular embodiment, the one or more environmental risk factors are selected from the group consisting of: obesity, smoking, vitamin and dietary supplement intake, use of alcohol or drugs, poor diet and a sedentary lifestyle.

One example is directed to a method of evaluating a treatment for age-related macular degeneration, comprising: determining a level, or an activity, or both said level and said activity, of at least one transcription and/or translation products or fragments of SEQ ID NOS:1-10 (Example 3 and FIG. 3) is compared to a reference value representing a known disease or health status, thereby evaluating the treatment of AMD.

One example is directed to a method of making a diagnostic array of the disclosure comprising: applying to a substrate at a plurality particular address on the substrate a sample of the individual purified polynucleotide compositions comprising SEQ ID NOS:1-10.

One example is directed to a kit and its use for diagnosis, or prognosis of age-related macular degeneration, or for determination of increased risk of developing AMD, or for monitoring a progression of age-related macular degeneration in a subject, or for monitoring success or failure of a therapeutic treatment of said subject.

DETAILED DESCRIPTION

Described herein is to the unexpected discovery that particular alleles at polymorphic sites associated with genes coding for proteins involved in the complement pathway are useful as markers for AMD and susceptibility to AMD. The compositions and methods described herein refer in particular to markers associated with complement factor I (CFI) alone or in combination with other markers associated with AMD or a susceptibility to AMD.

As used herein, "gene" is a term used to describe a genetic element that gives rise to expression products (*e.g.*, pre-mRNA, mRNA and polypeptides). A gene includes regulatory elements and sequences that otherwise appear to have only structural features, *e.g.,* introns and untranslated regions.

The genetic markers are particular "alleles" at "polymorphic sites" associated with particular complement factors, *e.g.,* CFI. A nucleotide position at which more than one nucleotide can be present in a population (either a natural population or a synthetic population, e.g., a library of synthetic molecules), is referred to herein as a "polymorphic site". Where a polymorphic site is a single nucleotide in length, the site is referred to as a single nucleotide polymorphism ("SNP"). If at a particular chromosomal location, for example, one member of a population has an adenine and another member of the population has a thymine at the same genomic position, then this position is a polymorphic site, and, more specifically, the polymorphic site is a SNP. Polymorphic sites can allow for differences in sequences based on substitutions, insertions or deletions. Each version of the sequence with respect to the polymorphic site is referred to herein as an "allele" of the polymorphic site. Thus, in the previous example, the SNP allows for both an adenine allele and a thymine allele.

A reference sequence is typically referred to for a particular genetic element, *e.g.,* a gene. Alleles that differ from the reference are referred to as "variant" alleles. The reference sequence, often chosen as the most frequently occurring allele or as the allele conferring a typical phenotype, is sometimes referred to as the "wild-type" allele.

Some variant alleles can include changes that affect a polypeptide, *e.g.,* the polypeptide encoded by a complement pathway gene. These sequence differences, when compared to a reference nucleotide sequence, can include the insertion or deletion of a single nucleotide, or of more than one nucleotide, resulting in a frame shift; the change of at least one nucleotide, resulting in a change in the encoded amino acid; the change of at least one nucleotide, resulting in the generation of a premature stop codon; the deletion of several nucleotides, resulting in a deletion of one or more amino acids encoded by the nucleotides; the insertion of one or several nucleotides, such as by unequal recombination or gene conversion, resulting in an interruption of the coding sequence of a reading frame; duplication of all or a part of a sequence; transposition; or a rearrangement of a nucleotide sequence. Alternatively, a polymorphism associated with AMD or a susceptibility to AMD can be a synonymous change in one or more nucleotides (*i.e.,* a change that does not result in a change to a codon of a complement pathway gene). Such a polymorphism can, for example, alter splice sites, affect the stability or transport of mRNA, or otherwise affect the transcription or translation of the polypeptide. The polypeptide encoded by the reference nucleotide sequence is the "reference" polypeptide with a particular reference amino acid sequence, and polypeptides encoded by variant alleles are referred to as "variant" polypeptides with variant amino acid sequences.

A genetic marker is "associated" with a genetic element or phenotypic trait, for example, if the marker is co-present with the genetic element or phenotypic trait at a frequency that is higher than would be predicted by random assortment of alleles (based on the allele frequencies of the particular population). Association also indicates physical association, *e.g.,* proximity in the genome or presence in a haplotype block, of a marker and a genetic element.

Haplotypes are a combination of genetic markers, *e.g.,* particular alleles at polymorphic sites. The haplotypes described herein are associated with AMD and/or a susceptibility to AMD. Detection of the presence or absence of the haplotypes herein, therefore is indicative of AMD, a susceptibility to AMD or a lack thereof. The haplotypes described herein are a combination of genetic markers, *e.g.,* SNPs and microsatellites. Detecting haplotypes, therefore, can be accomplished by methods known in the art for detecting sequences at polymorphic sites.

The haplotypes and markers disclosed herein are in "linkage disequilibrium" (LD) with preferred complement pathway gene(s), *e.g.,* CFI, and likewise, AMD and complement-associated phenotypes. "Linkage" refers to a higher than expected statistical association of genotypes and/or phenotypes with each other. "LD" refers to a non-random assortment of two genetic elements. If a particular genetic element (*e.g.,* an allele at a polymorphic site), for example, occurs in a population at a frequency of 0.25 and another occurs at a frequency of 0.25, then the predicted occurrence of a person's having both elements is 0.125, assuming a random distribution of the elements. If, however, it is discovered that the two elements occur together at a frequency statistically significantly higher than 0.125, then the elements are said to be in LD since they tend to be inherited together at a higher frequency than what their independent allele frequencies would predict. Roughly speaking, LD is generally correlated with the frequency of recombination events between the two elements. Allele frequencies can be determined in a population, for example, by genotyping individuals in a population and determining the occurrence of each allele in the population. For populations of diploid individuals, *e.g.,* human populations, individuals will typically have two alleles for each genetic element (*e.g.,* a marker or gene).

The disclosure is also directed to markers identified in a "haplotype block" or "LD block". These blocks are defined either by their physical proximity to a genetic element, *e.g.,* a complement pathway gene, or by their "genetic distance" from the element. Other blocks would be apparent to one of skill in the art as genetic regions in LD with the preferred complement pathway gene, *e.g.,* CFI. Markers and haplotypes identified in these blocks, because of their association with AMD and the complement pathway, are encompassed by the disclosure. One of skill in the art will appreciate regions of chromosomes that recombine infrequently and regions of chromosomes that are "hotspots", *e.g.,* exhibiting frequent recombination events, are descriptive of LD blocks. Regions of infrequent recombination events bounded by hotspots will form a block that will be maintained during cell division. Thus, identification of a marker associated with a phenotype, wherein the marker is contained within an LD block, identifies the block as associated with the phenotype. Any marker identified within the block can therefore be used to indicate the phenotype.

Additional markers that are in LD with the markers of the disclosure or haplotypes are referred to herein as "surrogate" markers. Such a surrogate is a marker for another marker or another surrogate marker. Surrogate markers are themselves markers and are indicative of the presence of another marker, which is in turn indicative of either another marker or an associated phenotype.

The Complement Pathway

Among other complement pathway members, complement pathway genes were selected genes for evaluation. Tag SNPs were selected across complement factors 3 and 5 and made particular inclusion of SNP rs2230199 in C3, which was reported to have a p = 2.8 $\times$ 10$^{-5}$ in single marker tests available on the NIH dbGAP database in a genome-wide association of 400 AMD cases and 200 controls. Several allelic markers were found in close association with CFI (Example 3), significantly including those described by SEQ ID NOS:8-10. Genotyping was performed as part of experiments using the Illumina GoldenGate assay and Sequenom iPLEX system. The study population consisted of 2,172 unrelated Caucasian individuals 60 years of age or older diagnosed based on ocular examination and fundus photography (1,238 cases of both dry and neovascular (wet) advanced AMD and 934 controls).

The role of epistasis between rs2230199 and five variants previously was also evaluated. Specifically, two variants at CFH (1061170- SEQ ID NO:2 and 10490924- SEQ ID NO:3), two variants at the CFB/C2 locus (9332739- SEQ ID NO:4 and 641153- SEQ ID NO:5), and one at the LOC387715/HTRA1 locus (1410996- SEQ ID NO:6) (see FIG. 3 for sequences) were established as unequivocally associated to AMD risk in this cohort. Using logistic regression, no statistically significant interaction terms between any pair of these SNPs, the two Factor B rare protective SNPs as a category or the three haplotypes formed by the two different CFH SNPs was identified. While weak interactions cannot be excluded, this result suggests that despite targeting the same pathway, these variants largely confer risk in an independent, log-additive fashion.

This associated Arg102Gly variant (SEQ ID NO:1) has previously been established as the molecular basis of the two common allotypes of C3: C3F (fast) and C3S (slow) (so named due to a difference in electrophoretic motility). The C3F variant has been previously reported as associated to other immune-mediated conditions such as IgA nephropathy and glomerular nephritis. The variant has also been reported to influence the long term success of renal transplants, where C3S homozygote recipients had much better graft survival and function when receiving a donor kidney with a C3F allotype than a matched homozygote C3S donor. More generally, deficiencies in both C3 and complement factor H (CFH) have been associated to the immune-mediated renal damage in membranoproliferative glomerulonephritis (MPGN), and the AMD-associated Y402H variant has also been shown to be significantly associated with MPGN underscoring a deep connection in the etiology of these two disorders (Li, M. et al., Nat. Genet., 38:1049-1054, 2006).

Case-control association studies for AMD in several genomic regions continued, yielding a SNP just 3' of Complement Factor I (CFI) on chromosome 4 with significant association (p < 10$^{-7}$). Sequencing was performed on coding exons in linkage disequilibrium with the detected association. No obvious functional variation was discovered that could be the proximate cause of the association, suggesting a non-coding regulatory mechanism. Subsequent studies with this marker, alone or in combination with other AMD-associated markers established the efficacy of detecting specific CFI-associated allelic markers for diagnosing AMD or a susceptibility to AMD.

Diagnostic Gene Array

Polynucleotide arrays provide a high throughput technique that can assay a large number of polynucleotide sequences in a single sample. This technology can be used, for example, as a diagnostic tool to assess the risk potential of developing AMD, *e.g.,* by detecting one or more AMD-associated allelic markers, *e.g.,* markers associated with CFI. Polynucleotide arrays (for example, DNA or RNA arrays), are known in the art for use as diagnostic or screening tools. Such arrays include regions of usually different sequence polynucleotides arranged in a predetermined configuration on a substrate, at defined x and y coordinates. These regions (sometimes referenced as "features") are positioned at respective locations ("addresses") on a substrate. The arrays, when exposed to a sample, exhibit an observed binding pattern. This binding pattern can be detected upon interrogating the array. All polynucleotide targets (for example, DNA) in the sample can be labeled, for example, with a suitable label *(e.g.,* a fluorescent compound) that allows for the detection of specific sample-array interactions. The observed binding pattern is indicative of the presence and/or concentration of one or more polynucleotide components of the sample.

Arrays can be fabricated by depositing previously obtained biopolymers onto a substrate, or by in situ synthesis methods. The substrate can be any supporting material to which polynucleotide probes can be attached, including glass, nitrocellulose, silicon, and nylon. Polynucleotides can be bound to the substrate by either covalent bonds or by non-specific interactions, such as hydrophobic interactions. The *in situ* fabrication methods include those described in U.S. Pat. No. 5,449,754 for synthesizing peptide arrays, and in U.S. Pat. No. 6,180,351 and WO 98/41531 and the references cited therein for synthesizing polynucleotide arrays. Further details of fabricating biopolymer arrays are described in U.S. Pat. No. 6,242,266; U.S. Pat. No. 6,232,072; U.S. Pat. No. 6,180,351; U.S. Pat. No. 6,171,797; EP No. 0 799 897;

PCT No. WO 97/29212; PCT No. WO 97/27317; EP No. 0 785 280; PCT No. WO 97/02357; U.S. Pat. Nos. 5,593,839; 5,578,832; EP No. 0 728 520; U.S. Pat. No. 5,599,695; EP No. 0 721 016; U.S. Pat. No. 5,556,752; PCT No. WO 95/22058; and U.S. Pat. No. 5,631,734. Other techniques for fabricating biopolymer arrays include known light directed synthesis techniques. Commercially available polynucleotide arrays, such as Affymetrix GeneChip™, can also be used. Use of the GeneChip™, to detect gene expression is described (Lockhart, D. et al., Nat. Biotech., 14:1675-1680, 1996; Chee, M. et al., Science, 274:610-614, 1996; Hacia, J. et al., Nat. Genet., 14:441-447, 1996; and Kozal, M. et al., Nat. Med., 2:753-759, 1996). Other types of arrays are known in the art, and are sufficient for developing an AMD diagnostic array of the present disclosure.

To create the arrays, single-stranded polynucleotide probes, for example, can be spotted onto a substrate in a two-dimensional matrix or array. Each single-stranded polynucleotide probe can comprise at least 6, 7, 8, 9, 10, 11, 12, 13, 14, 15, 16, 17, 18, 19, 20, 25 or 30 or more contiguous nucleotides selected from the nucleotide sequences shown in SEQ ID NO:1-10. In array fabrication, the probes formed at each feature are usually expensive. Additionally, sample quantities available for testing are usually also very small and it is therefore desirable to simultaneously test the same sample against a large number of different probes on an array. These conditions make it desirable to produce arrays with large numbers of very small (for example, in the range of tens or one or two hundred microns), closely spaced features (for example many thousands of features).

Samples can be assayed directly for the presence or absence of one or more AMD-associated markers. Samples can also be processed, for example, to isolate nucleic acids or to amplify specific nucleic acids. Tissue samples from a patient suspected of being at risk for developing AMD, for example, can be treated to isolate single-stranded polynucleotides, for example by heating or by chemical denaturation, as is known in the art. The single-stranded polynucleotides in a tissue sample can be labeled and hybridized to the polynucleotide probes on the array. Detectable labels that can be used include radiolabels, biotinylated labels, fluorophors, and chemiluminescent labels. Double-stranded polynucleotides, comprising the labeled sample polynucleotides bound to polynucleotide probes, can be detected once the unbound portion of the sample is washed away. Detection can be visual or with computer assistance. Preferably, after the array has been exposed to a sample, the array is read with a reading apparatus (such as an array "scanner") that detects the signals (such as a fluorescence pattern) from the array features. Such a reader would have a very fine resolution (for example, in the range of five to twenty microns) for a array having closely spaced features.

The signal image resulting from reading the array can be digitally processed to evaluate which regions of read data belong to a given feature as well as to calculate the total signal strength associated with each of the features. The foregoing steps, separately or collectively, are referred to as "feature extraction" (U.S. Pat No. 7,206,438, for example, describes an apparatus and method of enhancing feature extraction, e.g., processing one or more detected signal images each acquired from a field of view of an array reader). Using any of the feature extraction techniques so described, detection of hybridization of a patient derived polynucleotide sample with one of the AMD markers on the array given as SEQ ID NO:1-10 identifies that patient as having a genetic risk factor for AMD, as described above.

Also encompassed by the herein is a system for compiling and processing patient data, and presenting a risk profile for developing AMD. A computer aided medical data exchange system is preferred. The system can be designed to provide high-quality medical care to a patient by facilitating the management of data available to care providers. The care providers include, for example, physicians, surgeons, nurses, clinicians, various specialists, and so forth. It should be noted, however, that while general reference is made to a clinician in the present context, the care providers may also include clerical staff, insurance companies, teachers and students, and so forth. The system provides an interface, which allows the clinicians to exchange data with a data processing system. The data processing system is linked to an integrated knowledge base and a database. System, and the database draw upon data from a range of data resources. The database may be software-based, and includes data access tools for drawing information from the various resources as described below, or coordinating or translating the access of such information. In general, the database will unify raw data into a useable form.

The integrated knowledge base is intended to include one or more repositories of medical-related data. The data itself may relate to patient-specific characteristics as well as to non-patient specific information, as for classes of persons, machines, systems and so forth. Examples of patient-specific clinical data include patient medical histories, patient serum and cellular antioxidant levels, and the identification of past or current environmental, lifestyle and other factors that predispose a patient to develop AMD. These include various risk factors such as obesity, smoking, vitamin and dietary supplement intake, use of alcohol or drugs, poor diet and a sedentary lifestyle.

Use of the present system involves a clinician obtaining a patient sample, and evaluation of the presence of a genetic marker in that patient indicating a predisposition for AMD, such as SEQ ID NO:1-10. The clinician or their assistant also obtains appropriate clinical and non-clinical patient information, and inputs it into the system. The system then compiles and processes the data, and provides output information that includes a risk profile for the patient, of developing AMD. Particular illustrations of this process will depend on the specific information collected and the specific operations of the system, which are believed to be routine given the teachings provided herein.

Described herein are certain allelic markers, e.g., polynucleotide sequences, that have been correlated to AMD, compositions based on these markers, methods for using these markers, and kits and systems for practicing the methods using these markers. These markers are useful as diagnostics for identifying patients who have AMD, are at risk for developing AMD or who have a susceptibility to developing AMD. The markers described herein can be used alone or in conjunction with other diagnostic methods, e.g., methods using other markers or environmental risk factors.

EXEMPLIFICATION

Example 1.

Several candidate genes have screened negatively for association with AMD (Haddad, S. et al., Surv. Ophthalmol., 50:306-363, 2006). The list includes TIMP3 (Tissue inhibitor of metalloproteinases-3), IMPG2, the gene encoding the retinal interphotoreceptor matrix (IPM) proteoglycan IPM 200, VMD2 (the bestrophin gene), ELOVL4 (elongation of very long chain fatty acids), RDS (peripherin), EFEMP1 (EGF-containing fibulin-like extracellular matrix), BMD (bestrophin). One gene has been shown to have variations in the coding regions in patients with AMD, namely, GPR75 (a G protein coupled receptor gene). Others have shown a possible association with the disease in at least one study; PON1 the (paraoxonase gene), SOD2 (manganese superoxide dismutase, APOE (apolipoprotein E) for which the $\varepsilon 4$ allele has been found to be associated with the disease in some studies and not in others; and CST3 (cystatin C) for which one study has suggested an increased susceptibility for ARMD in CST3 B/B homozygotes. There are conflicting reports regarding the role of the ABCR (ABCA4) gene with regard to AMD. Allikmets and colleagues first reported an association with the disease.

Genetic variants associated with AMD have been identified. There is also an association to a region containing several tightly linked genes on chromosome 10 (LOC387715, HTRA1) although the function of those genes and variants is not fully understood. Using the database described herein, a previously unrecognized common, non-coding variant in CFH and other complement factor genes w was identified that substantially increases the influence of this locus on AMD, and strongly replicated the associations of four other published common alleles in three genes (p values ranging from 10-12 to 10-70), including the first confirmation of the BF/C2 locus.

*Complement Pathway is involved in AMD:* Genetic variants and environment play a role in AMD development and pathogenesis. Therefore, it is desirable to take both into account when determining an individual's risk. To date, the Y402H variant of complement factor H (CFH) is the most replicated and studied of several variants associated with AMD, conferring an estimated 7-fold increased risk in patients with the homozygous condition. The Y402H single nucleotide polymorphism (SNP) is within the CFH binding site for heparin and C-reactive protein (CRP). Altered binding to these sites can lead to loss of function; *e.g.,* decreased ability to bind to targets and/or interact with CRP, thereby giving rise to excessive complement activation. Because the initiation of complement activation can occur on cell surfaces as well as in the fluid phase, the activation of complement is one of the earliest events that can be detected.

When classical pathway activation occurs through the binding and activation of C1 to antibodies, C4 is cleaved, producing C4a and C4b. C4a is released locally and is circulated. It can be detected by a commercially available ELISA kits *(e.g.,* Pharmingen OPT-EIA) in ng/mL quantities. A similar event occurs when the lectin pathway is activated through binding of mannose binding lectin (MBL) to a carbohydrate-covered bacterial surface and the mannan-binding lectin-associated serine protease (MASP) enzymes cleave C4. C4a thus serves as a marker for activation of both the classical and lectin pathways. Many charged surfaces on microbes or other particulates including aggregates of multiple classes of immunoglobulins have been shown to activate the alternative complement pathway. The first split product released in this pathway is Bb from the cleavage of factor B. Bb can be measured in plasma by a commercial ELISA kit *(e.g.,* Quidel) in $\mu$g/mL quantities. As complement pathways can interact with one another, measuring components of each pathway may be important for diagnosis or prediction of complement-associated disease, *e.g.,* AMD.

If activation by any of the pathways continues, C3 is the next major protein to produce measurable fragments. C3 is initially split into two pieces: C3a is a small fragment that has anaphylatoxin activity, interacting through a specific C3a receptor found on many cell types, and C3b is a large fragment that has the property of binding covalently to nearby surfaces or molecules through an active thioester bond. The latter is produced by a conformational change in the molecule when the C3 convertase cleaves it. This covalent attachment leads to permanent deposits of C3b (or its subsequent cleavage fragments) on surfaces in the vicinity of complement activation. These deposits and subsequent cleavage fragments interact with C3 receptors (CR1, CR2, CR3, CR4) that are found on many cell types. This leads to immune adherence and provides a transport mechanism for the clearance of immune complexes, bacteria, viruses or whatever the C3b has become attached to. C5a and C5b-9 (membrane attack complex (MAC)) are markers of the terminal activation pathway as well.

CFH dampens the alternative pathway by three actions: 1) it prevents binding of factor B to C3; b) it binds to C3bBb (the alternative pathway C3 convertase), displacing the Bb enzymatic subunit; and 3) it provides cofactor activity for factor I (CFI), which can then cleave C3b, producing the inactive form, iC3b. Some iC3b is in the fluid-phase in concentrations normally below 30 $\mu$g/mL in plasma, with low variability. When elevated, it may provide an indirect indication that CFH is functioning to inactivate C3b. Inhibition of CFH with antibody reduces the cleavage of C3b to iC3b as measured by Western blot. To determine the function of CFH in inactivating C3b, it would be desirable to measure C3b and iC3b. C3b assays, however, show substantial variability. C3, which reflects certain disease states, is therefore measured, and the ratio of iC3b/C3 is analyzed as another possible indicator of AMD risk.

Factor B provides the enzymatic subunit, Bb, of the C3 convertase, contributing to the amplification loop of the alternative pathway and formation of C5 convertase. Whereas CFH dampens the alternative pathway, properdin stabilizes C3 and C5 convertases of the alternative pathway, thus serving to promote formation of the MAC instead of inactivation of C3b. Whereas variants of CFH increase the risk of AMD, variations in the genes encoding factor B were found to reduce the risk of AMD. Both factors B and C3 are important in the development of laser-induced choroidal neovascularization in mouse models.

In addition to genetic considerations, environmental factors play a role in AMD risk and may affect complement levels. Smoking is an independent risk factor for AMD and has been reported to activate complement and to increase factor B levels. Smokers have been reported to have reduced CFH levels. Plasma levels of CFH are reported to vary widely in the general population (110-615 $\mu$g/mL) and the measurement of CFH may not differentiate normal from variant CFH. To identify at-risk patients, therefore, other possible biomarkers associated with AMD are measured- biomarkers that may also be affected by environmental factors strongly associated with increased risk of AMD. Based on the pathways, it would be anticipated that iC3b (or iC3b/C3) would be most elevated in non-smokers with the CFH Y402H TT genotype and with low BMI (anticipated to have stage 1), and undetectable in CC smokers with high BMI and with advanced AMD. For CC smokers with stage 1, it would be expected that factor B levels would be lower than in those with advanced AMD (with the possible caveat of patients with protective variants of factor B). Bb, a fragment of factor B produced by activation of the alternative pathway, is a reliable marker of alternative pathway activation. Ratios of Bb to B are informative with respect to the activation rate and extent of the alternative pathway, and analysis of these factors in conjunction with C3 measures provides insight into the processes ongoing in the inflammatory lesions.

*Genetic approach to AMD:* AMD falls into the category of complex, late-onset diseases similar to type II diabetes, Alzheimer's disease, cardiovascular disease, hypertension, etc. where the genetic contributions do not necessarily manifest with straightforward Mendelian inheritance. Instead, it is presumed that these and other complex diseases are the result of complex interaction between environmental factors and susceptibility of multiple alleles of multiple genes and that these factors only cause disease when, in combination, a threshold of susceptibility is reached. Two major hypotheses are commonly explored to search for these genetic risk factors - the "common disease/common variant hypothesis" *(e.g.,* as suggested by the association of the APOE4 allele with Alzheimer's disease) and the hypothesis that rarer, more penetrant variants at multiple genes may explain the genetic component of multifactorial disease. While there is not general agreement, and limited empirical data, to suggest which hypothesis will bear more fruit in any individual disease, it seems most likely that complex diseases with involvement of many genes may quite naturally have contributions from both common and rare variation.

To detect common, low-penetrance variation, the association study is the design of choice- as made evident by both theoretical considerations and a proven track-record of detecting common genetic variants for multifactorial disease. Common variation has been conclusively determined to play a substantial role in the heritability of AMD. Previous efforts, however, have focused almost exclusively on polymorphisms that are already known to result in changes in the coding and regulatory regions of genes. A limited knowledge of the genome, limited ability to recognize many forms of potentially functional variation from sequence context alone, and lack of true understanding of causal pathways, has therefore limited the ability to apply these techniques (which remain quite costly and unproven). These hurdles have been overcome and recent results indicate that successful identification and replication of low-penetrance alleles can be convincingly achieved.

Plasma biomarkers in the complement system are associated with AMD and AMD progression, and these associations differ according to genotype, controlling for environmental factors.

Baseline plasma levels of the complement factors are measured in patients who are genotyped and phenotyped for AMD to determine if these markers predict risk of AMD given environmental risk factors. The study population includes: 1) Discordant sibling pairs (from families and DZ twins) with one sibling grade 3b, 4, and 5 and one sibling with grade 1 (N = 100 pairs, with 200 siblings), and 2) Progressors among the siblings with transition from grades 1-4 to grades 3b, 4, and 5 or grade 4 to 5 over time (total sample 620 of whom 214 have progressed). There will be additional progressors over time and the total sample expected for this aim is approximately 1000 subjects. All subjects have stored plasma samples that have never been thawed, and were collected in a manner that can be used for these lab analyses. Plasma data can be coupled with risk factor data as described above, including smoking, body mass index (BMI) and serum high-sensitivity CRP from a different aliquot of blood drawn on the same day as the proposed plasma complement assays (for the discordant pairs). Serum CRP and plasma complement factors (from aliquots drawn on the same day at baseline) can also be measured for subjects in the progression aspect of the study for the prospective analyses.

Complement assays: CFH, factor B, factor I, C3 and C5 levels are measured primarily with radial immunodiffusion, using polyclonal antisera specific for the components, according to the procedures followed by the Complement Laboratory at NJC. Split products C3a, iC3b, C5a and C4a, along with the terminal complement complex (SC5b-9), are measured by ELISA using kits produced by Pharmingen BD or Quidel. Ratios (iC3b:C3 and C3a:C3) can be calculated with these data. The normal ranges for these components are given in Table 1.

Table 1

| Component | Normal Range (mean $\pm$ 2 standard deviations) |
|---|---|
| Factor H | 160 - 412 $\mu$g/mL |
| Factor I | 29 - 58 $\mu$g/mL |
| Factor B | 127.6 - 278.5 $\mu$g/mL |
| C3 | 66 - 162 mg/dL |
| C5 | 55 - 113 $\mu$g/mL |
| C4 | 11 - 39 mg/dL |
| C3a | 98 - 857 ng/mL |
| iC3b | 0 - 30.9 $\mu$g/mL |
| Bb | 0-0.83 $\mu$g/mL |
| SC5b-9 | 0 - 179 ng/mL |
| C4a | 101 -745 ng/mL |

In the clinical laboratory, anything outside of three standard deviations is considered abnormal. Given that some of the patients may have low native components (C3, FB and C4), the ratio of the levels to the split products are predicted to be more useful than absolute amounts. Comparison of the results from the disease cohorts with the controls is extremely useful for further studies in terms of identifying the appropriate biomarkers for AMD patients. All complement split products are evaluated in specimens that have been collected in EDTA tubes, processed to obtain the EDTA-plasma rapidly after blood collection, and stored frozen in liquid nitrogen freezers. Each specimen is tested for all proteins on the first thaw, since repeated freeze-thaw cycles can produce false positive results.

Methods- CFH, factor I, factor B, C5: Radial immunodiffusion is performed by preparing 1% agarose gels containing an appropriate amount of specific antibody for the component to be measured. Wells are cut in the gel and filled with a measured amount of each test serum or plasma, control serum or plasma, and a series of at least three standards with known concentration of the component measured. After incubation of the filled gels for 72 hours at 4°C, the diameter of the precipitin ring formed by combination of the antibody with its antigen (the component being tested) is measured and the area of the precipitin ring is calculated. Using the areas of the rings formed by the standards, the concentrations of the component present in the test samples are calculated by linear regression.

C3a, C4a: ELISA method using OptEIA kits from Pharmingen-BD (San Diego).

iC3b, Bb, SC5b-9: these markers are measured using kits from Quidel (San Diego, CA). Three controls are run with each set of test samples, and the specimens are all tested in duplicate.

C-reactive protein (CRP) binds to CFH at the CCP7 where the Y402H CFH polymorphism exists. Serum CRP was found to be elevated in patients with AMD compared to controls. CRP may also increase the risk of AMD in patients carrying at least one allele of the CFH variant. While not being bound by a particular theory, it has been proposed that CFH binds CRP and counter-arrests alternative pathway activation induced by damaged tissue.

Analyses: For the case-control comparison, conditional logistic regression was used to determine the likelihood of having advanced AMD given levels of the various complement factors and CRP values within categories of genotype, while assessing and adjusting for pack year history of smoking, BMI and cardiovascular disease. Effect modification between complement factors versus CRP and complement factors versus genotype is also determined. Risk factor data is available within the existing database and analyzed. Additional analyses are performed to assess associations between genotype and complement factors using the general linear model. For progression, Cox regression analyses is applied to assess whether complement levels are associated with AMD progression, controlling for genotype, smoking, BMI, CRP, etc. Interactions and effect modification are assessed to determine if complement factors are more or less related to AMD within certain genotypes, or whether these associations vary depending on smoking status, level of BMI, etc. Power for the discordant pair analyses is adequate to detect an effect size (i.e., mean difference between groups/sd) = 0.40 with 80% power based on a comparison of 100 cases and 100 controls. Power is even larger for the progression study where there are 214 progressors out of 620 subjects. Regarding multiple testing, the different complement factors tend to be highly correlated and a Bonferroni type correction would be inappropriate.

Example 2. Prediction Model for Advanced Atrophic and Neovascular Age-Related Macular Degeneration Based on Genetic, Demographic, and Environmental Variables.

Context: Six single nucleotide polymorphisms (SNPs) in five genes are associated with age-related macular degeneration (AMD). Described herein are the joint effects of genetic and environmental variables leading to predictive models for potential screening for AMD.

Design, Setting, and Participants: Caucasian participants in the multi-center Age-Related Eye Disease Study with advanced AMD and visual loss (n = 509 cases) or no AMD (n = 222 controls) were evaluated. Advanced AMD was defined as geographic atrophy, neovascular disease. Risk factors including smoking and BMI were assessed, and DNA specimens were genotyped for the six variants in five genes: *CFH, LOC387715/HTRA1, CFB, C2,* and *C3.* Unconditional logistic regression analyses were performed. Receiver operating characteristic (ROC) curves were calculated.

Outcome Measures: Prevalence of advanced dry and neovascular AMD and predictive ability of risk scores based on sensitivity and specificity to discriminate between cases and controls.

Results: *CFH Y402H, CFH* rs1410996*, LOC387115 A69S, C2 E318D, CFB R32Q,* and C3 *R102H* polymorphisms are each independently related to advanced AMD, controlling for demographic factors, smoking, BMI, and vitamin/mineral treatment assignment. Multivariate odds ratios (OR's) were 3.5 (95% confidence interval (CI) 1.7-7.1) for *CFH Y402H; 3. 7* (95% CI 1.6-8.4) for *CFH rs1410996;* 25.4 (95% CI 8.6-75.1) for *LOC387715 A69S;* 0.3 (95% CI 0.1-0.7) for *C2* E318D; 0.3 (95% CI 0.1-0.5) for *CFB;* and 3.6 (95% CI 1.4-9.4) for *C3 R102H,* comparing the homozygous risk/protective genotypes to the referent genotypes. Genetic plus environmental risk scores provided C statistics ranging from 0.803 to 0.859, which were replicated in an independent sample of 452 cases and 317 controls.

Six genetic variants, as well as smoking and BMI are independently related to advanced AMD causing visual loss, with excellent predictive power.

***Methods****: Phenotypic Data*

The Age-Related Eye Disease Study (AREDS; Arch. Ophthalmol., 119:1417-1436, 2001; Arch. Ophthalmol., 125:1225-1232; Arch. Ophthalmol., 125:671-679, 2007; Arch. Ophthalmol., 112:533-539, 2005) included a randomized clinical trial to assess the effect of antioxidant and mineral supplements on risk of AMD and cataract, and a longitudinal study of AMD. Based on ocular examination and AREDS reading center photographic grading of fundus photographs, Caucasian participants in this study were divided into two main groups representing the most discordant phenotypes: no AMD with either no drusen or nonextensive small drusen (n = 222), or advanced AMD with visual loss (n = 509). Non-Caucasians were excluded since the distribution of advanced AMD in that population differs considerably compared with Caucasians. The advanced form of AMD, groups 3 and 4 in the original AREDS classification that include non-central and central atrophy, neovascular disease, as well as visual loss, was then reclassified into the two subtypes as either non-central or central geographic atrophy (n = 136) or neovascular disease (n = 373), independent of visual acuity level using the Clinical Age-Related Maculopathy Grading System, to determine whether results differed between these two (advanced dry and wet) phenotypes. Another comparison was made between unilateral or bilateral advanced AMD according to the AREDS system. Demographic and risk factor data, including education, smoking history, and BMI, were obtained at the baseline visit from questionnaires and height and weight measurements. Antioxidant status was defined as taking antioxidants (antioxidants alone or antioxidants and zinc) or no antioxidants (placebo or zinc alone) in the clinical trial.

***Methods: Genotyping***

DNA samples were obtained from the AREDS Genetic Repository. The following six SNPs were evaluated: 1) Complement Factor H *(CFH) Y402H* (rs1061170) in exon 9 of the *CFH* gene on chromosome 1q31, a change 1277T>C, resulting in a substitution of histidine for tyrosine at codon 402 of the *CFH* protein, *2) CFH* rs1410996 is an independently associated single nucleotide polymorphism (SNP) variant within intron 14 of *CFH,* 3) *LOC387715 A69S* (rs10490924 in the LOC387715/HTRA1 region of chromosome 10), a non-synonymous coding SNP variant in exon 1 of *LOC387715,* resulting in a substitution of the amino acid serine for alanine at codon 69, 4) Complement Factor 2 or *C2 E318D* (rs9332739), the non-synonymous coding SNP variant in exon 7 of C2 resulting in the amino acid glutamic acid changing to aspartic acid at codon 318, and 5), Complement Factor B or *CFB R32Q* (rs641153), the non-synonymous coding SNP variant in exon 2 of *CFB* resulting in the amino acid glutamine changing to arginine at codon 32, and 6), Complement Factor 3 or *C3 R102H* (rs2230199), the non-synonymous coding SNP variant in exon 3 of *C3* resulting in the amino acid glycine to arginine at codon 102. Genotyping was performed using primer mass extension and MALDI-TOF MS analysis by the MassEXTEND methodology of Sequenom (San Diego, CA).

*Methods: Statistical Analysis*

Individuals with advanced AMD, as well as the separate subtypes of dry, wet and bilateral advanced AMD, were compared to the control group of Caucasian persons with no AMD, with regard to genotype and risk factor data. Multivariate unconditional logistic regression analysis was performed to evaluate the relationships between AMD and all of the genotypes plus various risk factors, controlling for age (70 or older, younger than 70), gender, and education (high school or less, more than high school), cigarette smoking (never, past, current), and BMI, which was calculated as the weight in kilograms divided by the square of the height in meters (<25, 25-29.9, and ≥30). The AREDS assignment in the randomized clinical trial was also added to the multivariate model (taking a supplement containing antioxidants or taking study supplements containing no antioxidants). Tests for multiplicative interactions between each of the genotypes versus smoking and BMI respectively, were calculated using cross product terms according to genotype and the individual risk factors. In addition, similar analyses were performed to assess gene-gene interactions for each combination of genes. Odds ratios and 95% CIs were calculated for each risk factor and within the three genotype groups. Tests for trend for the number of risk alleles for each genetic variant (0, 1, 2) were calculated. Sensitivities and specificities for a variety of risk score cut-points were evaluated to assess the optimal use of the model for individual risk prediction, *e.g.,* sensitivities and specificities of at least 80%. The method for calculation of the AMD risk score based on all genetic, demographic and behavioral factors is explained in Table 2. The areas under the receiver operating characteristic (ROC) curves were obtained separately for the age groups 50-69 and 70+ years. In addition, an age-adjusted concordant or "C" statistic based on the ROC curves was calculated for different combinations of genes and environmental factors to assess the probability that the risk score based on the group of risk factors in that model from a random case was higher than the corresponding risk score from a random control within the same 10 year age group. To test the reproducibility of the risk prediction model, a separate replication sample consisting of 452 cases and 317 controls was obtained from the AMD study databases using the same grading system based on ocular photographs, and computed the C statistic using the risk score derived from the original sample. ROC curves were obtained for the replication sample.

*Results*

The mean ages (±SD) of cases and controls were 69.1 (±5.2) and 66.8 (±4.2) respectively. Females comprised 58% of cases and 54% of controls. Table 3 displays the relationship between genotype and covariate data among controls. There were no statistically significant associations between any of the genetic variants and the demographic, behavioral, or treatment variables. There was a non-significant trend toward an association between age and the C3 variant, with a somewhat higher proportion of the younger individuals with one or two risk alleles, or the GC or GG genotypes.

Relationships between pairs of genes were also evaluated. Table 4 displays multivariate adjusted associations between advanced AMD and demographic and behavioral factors controlling for all genetic variants, as well as associations between AMD and genetic factors adjusting for the environmental factors. There were positive associations between the two independent *CFH* variants and the combined advanced AMD group *(Y402H,* OR=3.5, 95%CI 1.7-7.1, p trend = 0.0003); *CFH* rs1410996 (OR = 3.7, p trend = 0.0003). There were positive associations between AMD and the *LOC388715 A69S* variant (OR = 25.4, p trend < 0.0001) and *C3* (OR = 3.6, p trend = 0.001). There were protective associations between *C2* (OR = 0.3, p = 0.003) and *CFB* variant (OR = 0.3, p < 0.0001). There were positive independent associations with age (OR = 2.8, p < 0.0001), current smoking (OR = 3.9, p = 0.001), and past smoking (OR = 1.9, p = 0.004). There was a protective effect of higher education (OR = 0.6, p = 0.01). A borderline positive association with BMI was present (OR = 1.5, p = 0.11) and no significant association with gender or antioxidant treatment was seen. In general, similar associations between genes and AMD were seen for all subtypes of AMD, including unilateral and bilateral advanced AMD and dry and wet types of advanced AMD, although associations varied slightly for specific types of advanced AMD.

Interactions between each genotype versus smoking (ever/never) and BMI (25+/ <25), were evaluated (Table 5). No significant interactions were found between any of the genotypes and smoking or BMI. There was a trend for a smaller effect of BMI on those with genotype *CFH Y402H* TT and an adverse effect of BMI for those with a risk allele (the CC and CT genotypes). Furthermore, within a given genotype, smoking and higher BMI increased risk of advanced AMD. For example, for the homozygous GG risk genotype for *C3,* the OR for advanced AMD was 3.3 (1.0-10.9) for "never smokers", and increased to 9.8 (2.0-47.5) for individuals who had ever smoked, indicating that there are main effects of both smoking and *C3* genotype but no interaction effect.

Table 6 shows C statistics for models with different combinations of genetic, demographic, and environmental variables. The C statistic for model 1 based on the two previously reported genes, *CFH Y402H* and *LOC 387715 A69S,* (ref) and age, gender, education, and antioxidant treatment was 0.803 ± 0.018. There was a significant improvement in the C statistic upon adding smoking and BMI as additional risk factors in model 2 with a C statistic of 0.822 ± 0.017 (model 1 versus 2, p = 0.027). For model 3, the model including all six variants was considered, together with age, gender, education and antioxidant treatment. A C statistic of 0.846 $\pm$ 0.016, which was a significant improvement over the corresponding two gene model (model 1 versus 3, p < 0.001). When smoking and BMI were added to the basic six genetic variant model 3, the C statistic increased to 0.859 $\pm$ 0.015, and this was a significant improvement compared with the corresponding two gene model (model 2 versus 4, p = 0.001). There was a modest improvement as well with the addition of the environmental variables to the model with the six variants (model 3 versus 4, p = 0.037). It should be noted that these C statistics are higher than the Framingham risk score prediction model results for coronary heart disease (CHD).

The AMD risk score was tested in a separate replication sample of 452 cases and 317 controls that were not used in constructing the algorithm. The mean ages ($\pm$SD) were 76 $\pm$ 6.6 for cases and 72 $\pm$ 4.4 for controls, of which 49% and 53% were male, respectively. The C statistic was 0.810 $\pm$ 0.016, which indicates excellent discrimination between cases and controls. This C statistic was calculated with adjustment for age, gender, education, smoking and BMI. For this analysis, antioxidant status was assigned as "no" since participants were not taking AREDS supplements at the time of enrollment, and, in a separate analysis, no subjects were consuming high quantities of these antioxidants in their diets. The C statistic for both the original and replication samples are comparable to or exceed the C statistic for the Framingham risk score for prediction of CHD.

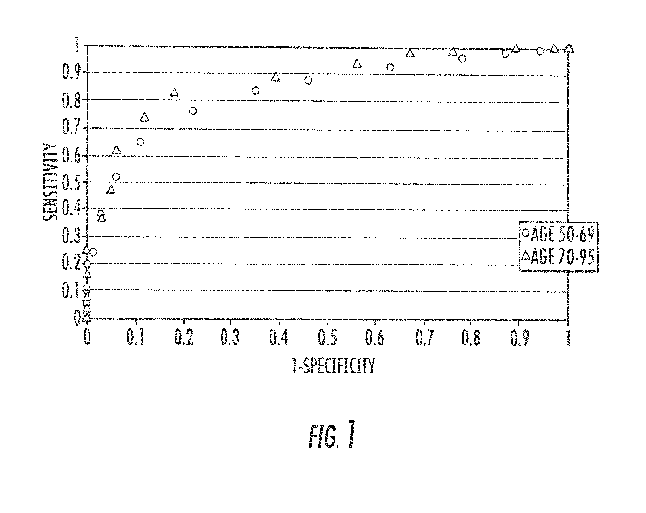

The sensitivity and specificity of model 4 was calculated using different cut points to denote potential screen positive criteria separately for each age group, as described in Table 2. The corresponding ROC curves are presented in FIG. 1. A cutpoint where both the sensitivity and specificity would be at least 80% was identified for the older age group (risk score $\geq$ 3 is screen positive, < 3 is screen negative), which yielded a sensitivity of 83% and specificity of 82%. Risk prediction for the younger age group was good; for a cut point of screen positivity of 2.5, the sensitivity was 76% and the specificity was 78%. The risk prediction was better for the older age group (FIG. 1).

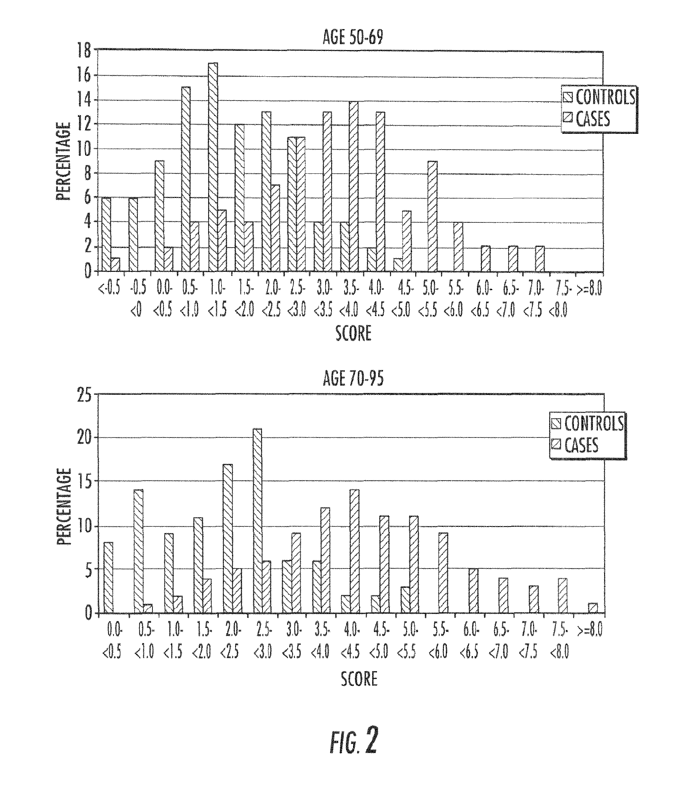

Risk score distributions within a given age group appeared to be substantially different with case scores tending to be higher than controls although there was some overlap. The risk scores for the replication sample according to age and case-control status are seen at the bottom of FIG. 2 and indicate good separation between cases and controls particularly for older individuals.

Described in this example is the independent association of six genetic variants with AMD adjusting for all of these variants in addition to demographic and behavioral factors. Discrimination between cases and controls is excellent for the overall risk score in both the original and replication samples. The predictive power of this composite of risk factors for advanced AMD, with C statistic score of 0.86 and a replication C statistic of 0.81, are comparable to or better than the Framingham risk functions for CHD in which the C statistics were 0.79 for white men and 0.83 for white women in the Framingham study cohort and somewhat lower in several replication samples. Genetic factors clearly play a major role in AMD as demonstrated by the large and consistent estimates of the effects of the genetic variants on various groups with advanced AMD, including unilateral and bilateral disease, as well as the subtypes of geographic atrophy (dry) and neovascular (wet) advanced AMD. On the other hand, modifiable factors also have an impact. Cigarette smoking increased risk for all genotypes. Risk of advanced AMD increased, for example, from over 3-fold for non-smokers to almost 10-fold for smokers among individuals with the same homozygous C3 risk genotype compared with non-smokers with the non-risk genotype. Higher BMI also contributed to the risk profile for all genotypes.

This study included the evaluation of predictive power based on a large, well characterized population of Caucasian patients with or without advanced AMD from various geographic regions around the U.S. The standardized collection of risk factor information, direct measurements of height and weight, and classification of maculopathy by standardized ophthalmologic examinations and grading of fundus photographs. Misclassification was unlikely since grades were assigned without knowledge of risk factors or genotype. Controls were performed for known AMD risk factors, including age, education, BMI, smoking, and treatment assignment in assessing the relationship between genetic variants and advanced AMD. Both the environmental and genetic risk factors were independently associated with AMD, when considered simultaneously. Subjects likely represent the typical patient with advanced AMD, and the overall population is similar to others in this age range in terms of smoking and prevalence of obesity, as well as the distribution of the genotypes. This large and well-characterized population provided a unique opportunity to evaluate gene-environment associations and interactions. Furthermore, the biological effects of the genetic variants do not appear to differ in major ways among various Caucasian populations with AMD.

It is unlikely that many individuals without AMD in this elderly age group would progress to advanced disease during the remainder of their lifetime. Thus the potential for misclassification of controls who might ultimately become cases is likely to be small.

These analyses and results indicate the potential for individual risk prediction for AMD. In calculating the risk score, for example, one could estimate "points" from the regression coefficients (Table 2) for smoking (1.3), higher BMI (0.4), and the various genetic variants (ranging from -1.3 to +3.2) to obtain an overall risk for an individual to develop advanced AMD. This could be refined as new genetic and other risk predictors are established. Advantages of knowing such a risk score include the possibility for more targeted education and counseling about known modifiable factors. Screening would identify high risk people who would be encouraged to follow a healthy lifestyle by not smoking, eating vegetables and fish, maintaining a normal weight and getting exercise, and taking AREDS type antioxidant and mineral supplements for those with signs of AMD. All of these factors influence the inflammatory and immune pathways that are involved in the pathogenesis of AMD. Targeting high risk individuals could also lead to heightened awareness and more frequent surveillance and clinical examinations, as well as identification of high risk individuals for inclusion in clinical trials of new therapies.

**Table 2.** Calculation of AMD Risk Score.

The risk score was calculated from the following formula:

$$S = \sum_{i=1}^{18} \beta_i\, X_i \text{ where } \beta_i \text{ and } X_i \text{ are given as follows:}$$

| Variable Name ($X_i$) | Regression Coeff ($\beta$) | Code | Control ($X_0$) | $\beta_i X_i$ | Case ($X_i$) | $\beta_i X_i$ |
|---|---|---|---|---|---|---|
| Age 70+ | 1.0130 | | 0 | 0 | 0 | 0 |
| Gender | -0.1053 | 1=m /0=f | 1 | -0.11 | 1 | -0.11 |
| Education | -0.5845 | 1= some college/0= high school or less | 1 | -0.58 | 1 | -0.58 |
| Antioxidant Use | 0.2404 | 1 = yes/ 0= no | 0 | 0 | 1 | 0.24 |
| BMI 25-29 | 0.0871 | 1= yes/ 0= no | 1 | 0.09 | 1 | 0.09 |
| BMI 30+ | 0.4370 | 1= yes/ 0= no | 0 | 0 | 0 | 0 |
| Current Smoking | 1.3555 | 1= yes/ 0 = no | 0 | 0 | 0 | 0 |
| Past Smoking | 0.6247 | 1= yes/ 0= no | 1 | 0.62 | 1 | 0.62 |
| CFH:rs1061170 (Y402H) CT | 0.6002 | 1= yes/ 0 = no | 0 | 0 | 1 | 0.60 |
| CFH:rs1061170 (Y402H) CC | 1.2582 | 1= yes/ 0= no | 0 | 0 | 0 | 0 |
| LOC387715:rs10490924 (A69S) GT | 1.1238 | 1= yes/ 0= no | 0 | 0 | 0 | 0 |
| LOC387715:rs10490924 (A69S) TT | 3.2343 | 1= yes/ 0= no | 0 | 0 | 1 | 3.23 |
| C3:rs2230199 (R102H) CG | 0.4879 | 1= yes/ 0= no | 0 | 0 | 0 | 0 |
| C3:rs2230199 (R102H) GG | 1.2898 | 1= yes/ 0= no | 0 | 0 | 0 | 0 |
| CFB:rs641153 (R32Q) CT or TT | -1.3453 | 1=yes/ 0= no | 1 | -1.35 | 0 | 0 |
| C2: rs9332739 (E318D) CT or CC | -1.1830 | 1=yes/ 0= no | 0 | 0 | 0 | 0 |
| CFH:rs1410996 CT | 0.4989 | 1=yes/ 0= no | 0 | 0 | 0 | 0 |
| CFH:rs1410996 CC | 1.3004 | 1=yes/ 0= no | 0 | 0 | 1 | 1.30 |
| Risk Score | | | | -1.32 | | 5.4 |

*There was a constant of -1.9401 obtained from fitting the logistic model. This constant was not used in calculating risk score, so as to make most of the risk scores positive and easier to understand.

EP 2 283 163 B1

**Table 3. Genotype-Phenotype Associations Among Controls**

| | CFH:rs1061170(Y402H) | | | | | | | LOC387715:rs10490924(A69S) | | | | | | |
|---|---|---|---|---|---|---|---|---|---|---|---|---|---|---|
| | TT | | CT | | CC | | | GG | | GT | | TT | | |
| Variable | N | % | N | % | N | % | p (trend) | N | % | N | % | N | % | p (trend) |
| **Baseline Age** | | | | | | | | | | | | | | |
| ≤ 70 | 64 | 70.3 | 69 | 67.6 | 23 | 79.3 | | 104 | 68.9 | 48 | 71.6 | 4 | 100 | |
| 70+ | 27 | 29.7 | 33 | 32.4 | 6 | 20.7 | | 47 | 31.1 | 19 | 28.4 | 0 | | |
| p (trend) | | | | | | | 0.58 | | | | | | | 0.34 |
| **Gender** | | | | | | | | | | | | | | |
| Male | 42 | 46.2 | 42 | 41.2 | 17 | 58.6 | | 66 | 43.7 | 35 | 52.2 | 0 | | |
| Female | 49 | 53.8 | 60 | 58.8 | 12 | 41.4 | | 85 | 56.3 | 32 | 47.8 | 4 | 100 | |
| p (trend) | | | | | | | 0.53 | | | | | | | 0.82 |
| **Education** | | | | | | | | | | | | | | |
| High School or Less | 24 | 26.4 | 30 | 29.4 | 10 | 34.5 | | 44 | 29.1 | 19 | 28.4 | 1 | 25.0 | |
| College or More | 67 | 73.6 | 72 | 70.6 | 19 | 65.5 | | 107 | 70.9 | 48 | 71.6 | 3 | 75.0 | |
| p (trend) | | | | | | | 0.40 | | | | | | | 0.86 |
| **Smoking Baseline** | | | | | | | | | | | | | | |
| Never | 43 | 47.3 | 60 | 58.8 | 11 | 37.9 | | 81 | 53.6 | 30 | 44.8 | 3 | 75.0 | |
| Past | 42 | 46.2 | 38 | 37.3 | 17 | 58.6 | | 62 | 41.1 | 34 | 50.7 | 1 | 25.0 | |
| Current | 6 | 6.6 | 4 | 3.9 | 1 | 3.4 | | 8 | 5.3 | 3 | 4.5 | 0 | | |
| p (trend) | | | | | | | 0.77 | | | | | | | 0.69 |
| **BMI Baseline** | | | | | | | | | | | | | | |
| < 25 | 28 | 30.8 | 37 | 36.3 | 9 | 31 | | 51 | 33.8 | 22 | 32.8 | 1 | 33.3 | |
| 25-29 | 34 | 37.4 | 47 | 46.1 | 15 | 51.7 | | 65 | 43.0 | 30 | 44.8 | 1 | 33.3 | |
| ≥ 30 | 29 | 31.9 | 18 | 17.6 | 5 | 17.2 | | 35 | 23.2 | 15 | 22.4 | 1 | 33.3 | |
| p (trend) | | | | | | | 0.14 | | | | | | | 0.67 |
| **Antioxidants** | | | | | | | | | | | | | | |
| Yes | 37 | 40.7 | 50 | 49.0 | 11 | 37.9 | | 68 | 45.0 | 28 | 41.8 | 2 | 50.0 | |
| No | 54 | 59.3 | 52 | 51.0 | 18 | 62.1 | | 83 | 55.0 | 39 | 58.2 | 2 | 50.0 | |
| p (trend) | | | | | | | 0.79 | | | | | | | 0.77 |

15

### Table 3, continued. Genotype-Phenotype Associations Among Controls Genotype

| | | | | | | | C2:rs9332739(E318D) | | | | | CFB:rs641153(R32Q) | | | | | C3:rs2230199(R102H) | | | | | | |
|---|---|---|---|---|---|---|---|---|---|---|---|---|---|---|---|---|---|---|---|---|---|---|---|
| | TT | | CT | | CC | | TT | | CT or CC | | | CC | | CT or TT | | | CC | | CG | | GG | | |
| Variable | N | % | N | % | N | % | N | % | N | % | | N | % | N | % | | N | % | N | % | N | % | |
| **Baseline Age** | | | | | | | | | | | | | | | | | | | | | | | |
| ≤ 70 | 30 | 75.0 | 78 | 66.7 | 48 | 73.8 | 140 | 71.1 | 16 | 64.0 | | 119 | 70.4 | 37 | 69.8 | | 92 | 65.7 | 58 | 78.4 | 6 | 75.0 | |
| 70+ | 10 | 25.0 | 39 | 33.3 | 17 | 26.2 | 57 | 28.9 | 9 | 36.0 | | 50 | 29.6 | 16 | 30.2 | | 48 | 34.3 | 16 | 21.6 | 2 | 25.0 | |
| p (trend) | | | | | | 0.93 | | | | | 0.47 | | | | | 0.93 | | | | | | | 0.08 |
| **Gender** | | | | | | | | | | | | | | | | | | | | | | | |
| Male | 17 | 42.5 | 52 | 44.4 | 32 | 49.2 | 93 | 47.2 | 8 | 32.0 | | 76 | 45.0 | 25 | 47.2 | | 66 | 47.1 | 34 | 45.9 | 1 | 12.5 | |
| Female | 23 | 57.5 | 65 | 55.6 | 33 | 50.8 | 104 | 52.8 | 17 | 68.0 | | 93 | 55.0 | 28 | 52.8 | | 74 | 52.9 | 40 | 54.1 | 7 | 87.5 | |
| p (trend) | | | | | | 0.47 | | | | | 0.15 | | | | | 0.78 | | | | | | | 0.23 |
| **Education** | | | | | | | | | | | | | | | | | | | | | | | |
| High School or Less | 11 | 27.5 | 28 | 23.9 | 25 | 38.5 | 58 | 29.4 | 6 | 24.0 | | 47 | 27.8 | 17 | 32.1 | | 46 | 32.9 | 16 | 21.6 | 2 | 25.0 | |
| College or More | 29 | 72.5 | 89 | 76.1 | 40 | 61.5 | 139 | 70.6 | 19 | 76.0 | | 122 | 72.2 | 36 | 67.9 | | 94 | 67.1 | 58 | 78.4 | 6 | 75.0 | |
| p (trend) | | | | | | 0.14 | | | | | 0.57 | | | | | 0.55 | | | | | | | 0.12 |
| **Smoking Baseline** | | | | | | | | | | | | | | | | | | | | | | | |
| Never | 18 | 45.0 | 66 | 56.4 | 30 | 46.2 | 101 | 51.3 | 13 | 52.0 | | 91 | 53.8 | 23 | 43.4 | | 75 | 53.6 | 34 | 45.9 | 5 | 62.5 | |
| Past | 19 | 47.5 | 46 | 39.3 | 32 | 49.2 | 85 | 43.1 | 12 | 48.0 | | 70 | 41.4 | 27 | 50.9 | | 58 | 41.4 | 37 | 50.0 | 2 | 25.0 | |
| Current | 3 | 7.5 | 5 | 4.3 | 3 | 4.6 | 11 | 5.6 | 0 | | | 8 | 4.7 | 3 | 5.7 | | 7 | 5.0 | 3 | 4.1 | 1 | 12.5 | |
| p (trend) | | | | | | 0.95 | | | | | 0.61 | | | | | 0.22 | | | | | | | 0.58 |
| **BMI Baseline** | | | | | | | | | | | | | | | | | | | | | | | |
| < 25 | 14 | 35.0 | 41 | 35.0 | 19 | 29.2 | 70 | 35.5 | 4 | 16.0 | | 57 | 33.7 | 17 | 32.1 | | 49 | 35.0 | 21 | 28.4 | 4 | 50.0 | |
| 25-29 | 15 | 37.5 | 51 | 43.6 | 30 | 46.2 | 82 | 41.6 | 14 | 56.0 | | 72 | 42.6 | 24 | 45.3 | | 59 | 42.1 | 36 | 48.6 | 1 | 12.5 | |
| ≥ 30 | 11 | 27.5 | 25 | 21.4 | 16 | 24.6 | 45 | 22.8 | 7 | 28.0 | | 40 | 23.7 | 12 | 22.6 | | 32 | 22.9 | 17 | 23.0 | 3 | 37.5 | |
| p (trend) | | | | | | 0.74 | | | | | 0.12 | | | | | 0.96 | | | | | | | 0.64 |

**Antioxidants**

(continued)

**BMI Baseline**

| | | | | | | | | | | | | | | | | | | | | |
|---|---|---|---|---|---|---|---|---|---|---|---|---|---|---|---|---|---|---|---|---|
| Yes | 17 | 42.5 | 56 | 47.9 | 25 | 38.5 | | 84 | 42.6 | 14 | 56.0 | | 78 | 46.2 | 20 | 37.7 | | 59 | 42.1 | 37 | 50.0 | 2 | 25.0 |
| No | 23 | 57.5 | 61 | 52.1 | 40 | 61.5 | | 113 | 57.4 | 11 | 44.0 | | 91 | 53.8 | 33 | 62.3 | | 81 | 57.9 | 37 | 50.0 | 6 | 75.0 |
| p (trend) | | | | | | 0.55 | | | | | 0.21 | | | | | 0.28 | | | | | 0.76 |

**Table 4. Association Between Advanced AMD and Demographic, Behavioral and Genetic Risk Factors.**

| No. Cases/Controls Variable | All Advanced AMD OR (95%CI)* 509/222 | p-values | Unilateral advanced AMD† OR (95% CI) 202/222 | p-values | Bilateral advanced AMD† OR (95% CI) 307/222 | p-value | Geographic atrophy ‡ OR (95% CI) 136/222 | p-value |
|---|---|---|---|---|---|---|---|---|
| Age (yr) | | | | | | | | |
| <70 | 1.0 | | 1.0 | | 1.0 | | 1.0 | |
| ≥ 70 | 2.8 (1.8-4.2) | <0.0001 | 2.3 (1.4-3.8) | 0.001 | 3.7 (2.2-6.2) | <0.0001 | 2.6 (1.5-4.6) | 0.001 |
| Gender | | | | | | | | |
| Female | 1.0 | | 1.0 | | 1.0 | | 1.0 | |
| Male | 0.9 (0.6-1.4) | 0.62 | 1.0 (0.6-1.5) | 0.85 | 0.9 (0.5-1.4) | 0.55 | 1.0 (0.6-1.8) | 0.89 |
| Education | | | | | | | | |
| ≤ High School | 1.0 | | 1.0 | | 1.0 | | 1.0 | |
| > High School | 0.6 (0.4-0.9) | 0.01 | 0.5 (0.3-0.9) | 0.01 | 0.6 (0.4-1.0) | 0.07 | 0.7 (0.4-1.2) | 0.18 |
| Smoking | | | | | | | | |
| Never | 1.0 | | 1.0 | | 1.0 | | 1.0 | |
| Past | 1.9 (1.2-2.9) | 0.004 | 2.2 (1.3-3.6) | 0.002 | 1.6 (0.9-2.6) | 0.09 | 1.8 (1.0-3.1) | 0.06 |
| Current | 3.9 (1.7-8.9) | 0.001 | 3.7 (1.5-9.6) | 0.01 | 4.0 (1.5-10.7) | 0.01 | 2.7 (0.8-8.9) | 0.11 |
| BMI | | | | | | | | |
| <25 | 1.0 | | 1.0 | | 1.0 | | 1.0 | |
| 25-29 | 1.1 (0.7-1.8) | 0.72 | 1.2 (0.7-2.1) | 0.53 | 1.0 (0.6-1.8) | 0.99 | 1.0 (0.5-1.9) | 0.97 |
| 30+ | 1.5 (0.9-2.6) | 0.11 | 1.7 (0.9-3.2) | 0.09 | 1.5 (0.8-2.9) | 0.25 | 2.7 (0.8-8.9) | 0.44 |
| Antioxidant | | | | | | | | |
| No | 1.0 | | 1.0 | | 1.0 | | 1.0 | |
| Yes | 1.3 (0.8-1.9) | 0.25 | 1.3 (0.8-2.1) | 0.29 | 1.2 (0.7-2.0) | 0.42 | 1.1 (0.6-1.9) | 0.77 |

**Table 5. Interaction Effects of BMI, Smoking, and Genotype on Risk of Advanced AMD.**

| | | BMI OR (95% CI)* | | | | |
|---|---|---|---|---|---|---|
| | <25 | 25+ | P (interaction) | P trend | Never | Ever |
| **Variable** | | | | | | |
| CFH:rs1061170(Y402H) | | | | | | |
| TT | 1.0 | 0.6 (0.3-1.4) | | | 1.0 | 1.6(0.8-3.4) |
| CT | 0.9 (0.4-2.0) | 1.6 (0.8-3.3) | 0.035 (CT vs TT) | | 1.3 (0.6-2.7) | 3.6(1.8-7.4) |
| CC | 1.8 (0.6-5.2) | 2.8 (1.1-6.9) | 0.14 (CC vs TT) | | 3.5 (1.3-9.1) | 5.1(2.1-12.3) |
| | | | | 0.090 | | |
| LOC387715:rs10490924(A69S) | | | | | | |
| GG | 1.0 | 1.3 (0.7-2.3) | | | 1.0 | 2.5(1.4-4.3) |
| GT | 3.3 (1.6-6.9) | 3.9 (2.1-7.2) | 0.81 (GT vs GG) | | 4.2 (2.2-7.8) | 6.0(3.4-10.8) |
| TT | 25.9 (3.2-211.1) | 32.1 (8.7-118.3) | 0.96 (TT vs GG) | 0.90 | 17.4 (4.7-63.5) | 120.4 (15.1-957.2) |
| CFH:rs1410996 | | | | | | |
| TT | 1.0 | 2.0 (0.5-8.0) | | | 1.0 | 2.1 (0.6-7.9) |
| CT | 2.4 (0.7-8.4) | 2.8 (0.8-9.6) | 0.46 (CT vs TT) | | 1.4 (0.4-4.5) | 4.0 (1.3-12.7) |
| CC | 5.3 (1.4-20.2) | 6.4 (1.8-22.7) | 0.50 (CC vs TT) | | 4.6 (1.4-15.2) | 6.5 (2.0-21.6) |
| | | | | 0.65 | | |
| C2:rs9332739(E318D) | | | | | | |
| TT | 1.0 | 1.3 (0.8-2.0) | | | 1.0 | 1.9 (1.3-3.0) |
| CT or CC | 0.6 (0.1-3.9) | 0.3 (0.1-0.6) | 0.44 (CT-CC vs TT) | | 0.2 (0.05-0.7) | 0.8 (0.3-2.2) |
| CFB:rs641153(R32Q) | | | | | | |
| CC | 1.0 | 1.3 (0.8-2.0) | | | 1.0 | 2.1 (1.3-3.2) |
| CT or TT | 0.3 (0.1-0.7) | 0.3 (0.1-0.6) | 0.9 (CT-TT vs CC) | | 0.3 (0.1-0.6) | 0.5 (0.2-1.0) |
| C3:rs2230199(R102H) | | | | | | |
| CC | 1.0 | 1.5 (0.9-2.7) | | | 1.0 | 2.2 (1.3-3.8) |
| CG | 2.4 (1.2-5.1) | 2.1 (1.1-3.9) | 0.21 (CG vs CC) | | 1.9 (1.0-3.6) | 3.3 (1.8-5.9) |
| GG | 2.5 (0.5-11.1) | 7.2 (1.9-27.2) | 0.51 (GG vs CC) | | 3.3 (1.0-10.9) | 9.8 (2.0-47.5) |
| | | | | 0.62 | | |

*OR=Odds Ratio, CI=confidence interval

OR's adjusted for age (<70, ≥ 70), gender, education (≤ high school, >high school), smoking (never, past, current), BMI (25, 25-29, 30+), antioxidant treatment (yes,no), and all genetic variants and associated genotypes.

**Table 6. C Statistics for Advanced AMD Based on Models with Different Combinations of Genetic and Environmental Variables.**

| Model | Sample | Genetic Variables | Demographic, Environmental Variables | C Statistic (+/- SE)* |
|---|---|---|---|---|
| 1 | original | CFH Y402H, LOC387715 A69S | Age, gender, education, antioxidant treatment | 0.803 +/- 0.018 |
| 2 | original | CFH Y402H, LOC387715 A69S | Age, gender, education, antioxidant treatment, smoking BMI | 0.822 +/- 0.017 |
| 3 | original | CFH Y402H, LOC387715 A69S, CFH 1410996, C2E318D, CFB R32Q, C3 R102H | Age, gender, education, antioxidant treatment | 0.846 +/- 0.016 |

(continued)

| Model | Sample | Genetic Variables | Demographic, Environmental Variables | C Statistic (+/- SE)* |
|---|---|---|---|---|
| 4 | original | CFH Y402H, LOC387715 A69S, CFH 1410996, C2E318D, CFB R320, C3 R102H | Age, gender, education, antioxidant treatment, smoking BMI | 0.859 +/- 0.015 |
| 4a | replication | CFH Y402H, LOC387715 A69S, CFH 1410996, C2E318D, CFB R320, C3 R102H | Age, gender, education, antioxidant treatment, smoking BMI | 0.810 +/- 0.016 |

*p value (model 1 vs 2, p = 0.027; 1 vs 3 p<0.001; 2 vs 4, p = 0.001, 3 vs 4, p = 0.037)

Example 3. Variation near Complement Factor I is associated with risk of Advanced AMD.

Case-control association studies for AMD in several genomic regions continued, yielding a SNP just 3' of Complement Factor I (CFI) on chromosome 4 with significant association ($p < 10^{-7}$). Sequencing was performed on coding exons in linkage disequilibrium with the detected association. No obvious functional variation was discovered that could be the proximate cause of the association, suggesting a non-coding regulatory mechanism.

The association of age-related macular degeneration (AMD) with variants on chromosome 1 (CFH), chromosome 6 (CFB; C2), chromosome 10 (LOC387715/ARMS2), and chromosome 19 (C3) have identified the primary role of the complement pathway in disease pathogenesis (Fisher, S. et al., Hum. Mol. Genet., 14:2257-2264, 2005; Klein, R. et al., Science, 308:385-389, 2005; Haines, J. et al., Science, 308:419-421, 2005; Edwards et al., Science, 421-424, 2005; Hageman, G. et al., Proc. Natl. Acad. Sci. USA, 102:7227-7232, 2005; Rivera, A. et al., Hum. Mol. Genet., 14:3227-3236, 2005; Gold, B. et al., Nat. Genet., 38:458-462, 2006; Maller, J. et al., Nat. Genet., 38:1055-1059, 2006; Maller, J. et al., Nat. Genet., 39:1200-1201, 2007;). Following up whole-genome linkage regions with fine-mapping has met with limited success in other complex diseases, however the effect sizes of the identified risk variants for AMD are dramatically larger than most late-onset disease associations (Science, 316:1331-1336, 2007; Easton, D. et al., Nature, 447:1087-1093, 2007; Samani, N. et al., N. Engl. J. Med., 357:443-453, 2007). In light of these successes, 1,500 SNPs were selected using two different criteria: targeting genes in regions under suggestive linkage peaks from a recent meta-analysis and genes selected from the complement pathway not in these regions. Genotyping was performed on 2,053 unrelated individuals using the Illumina GoldenGate assay and Sequenom MassARRAY iPLEX assay as previously described.

*Sample:* the study population consisted of 2,053 unrelated Caucasian individuals 60 years of age or older diagnosed based on ocular examination and fundus photography (1,228 cases of both dry and neovascular (wet) advanced AMD and 825 controls; Seddon, J. et al., Ophthalmol., 113:260-266, 2006). Informed consent was obtained in writing from all participants, and procedures were approved by the appropriate institutional review boards. This is largely the same sample set with the same phenotyping criteria described above- importantly, this sample has been previously confirmed to show no inflation of case-control association statistics due to population substructure.

*SNP Selection:* a total of 1,536 SNPs across regions of chromosomes 1, 2, 3, 4, 6 and 16 were genotyped based upon the Fisher *et. al.* bin rank of a meta-analysis of previous whole-genome linkage studies. SNPs were chosen in and around regions of transcription as described by Wiltshire, S. et al. (Eur. J. Hum. Genet., 14:1209-1214, 2006), however, the efficiency of this strategy was augmented by only selecting SNPs that tag seven or more other SNPs (super informative SNPs). This SNP selection routine was conducted by using Tagger and HapMap data from the CEPH population (Phase II). SNPs with a minor allele frequency (>10%) and with a minimum $r^2$ of 0.8 were selected. Within this set of SNPs were nine SNPs in and around the CFI region. Another 20 SNPs were chosen in the region to adequately tag the entire region using the same tagging parameters as above. The 29 total SNPs genotyped had very good information coverage (a mean $r^2 = 0.966$) for the 173Kb long region of interest that covered 114 out of the 116 HapMap SNPs in the region above a minor allele frequency (MAF) of 5%.

A total of eight SNPs were genotyped across C3 and seven SNPs were genotyped across C5. SNPs were picked using Tagger and HapMap data from the CEPH population (Phase II). SNPs were selected with a minor allele frequency (>5%) and a minimum $r^2$ of 0.8 to other selected SNPs. Thus, the SNPs that were selected should have been broadly representative of regional genetic variation because they were either direct proxies of other SNPs in those areas, or combined to form specific multimarker haplotypes that were correlated with other untyped SNPs in the region.

*Genotyping:* the 1,536 SNPs were genotyped at the Center for Inherited Disease Research (CIDR) using an Illumina OPA. The follow-up genotyping and sequencing was performed using the Sequenom MassARRAY system for iPLEX assays.

*Sequencing:* a novel SNP was discovered by sequencing 85 subjects as a subset of our case-control cohort.

This SNP (see SEQ ID NO:10) is an A/G SNP, with A being the major allele, and G having an MAF of 7.65%. The SNP of SEQ ID NO:10 is on chromosome 4 just 5' (113 bp) of CFI's exon 12 according to dbSNP, and is located at the coordinate 110 883 313 base pairs on chromosome 4 according to NCBI build 36.1. The SNPs have an $r^2$ of 0.057, 0.006 and 0.003 respectively with respect to rs13117504, rs10033900, and rs11726949. Since the two most associated SNPs are not in high correlation with this novel SNP, nor does it have a MAF that is very close to the associated SNPs, it is fairly certain that this new CFI-related SNP is not the causal SNP driving the association in this region.

*Analysis:* all linkage disequilibrium calculations (*e.g.,* D' and $r^2$) were performed with Haploview (Barrett, J. et al., Bioinformatics, 21:263-265, 2005). Single-locus and two-marker haplotype association analysis was conducted using logistic regression tests implemented in PLINK (Purcell, S. et al., Am. J. Hum. Genet., 81:559-575, 2007).

To calculate the percent variance accounted for by any risk alleles, a prevalence of late-stage AMD was assumed in this older age group to be 5% and that liability is normally distributed in the population, with a mean of 0 and a variance of 1.

*Subjects:* the methods employed in this study conformed to the tenets of the Declaration of Helsinki and received approval from the appropriate institutional review boards. Informed consent was signed by all participants. For these analyses, unrelated Caucasian individuals with extremely discordant phenotypes were included. Cases were defined as described above.

*Statistical Analysis:* association testing as well as the other statistical analyses were performed using Haploview (found at the world wide web site, broad.mit.edu/mpg/haploview) and PLINK (found at the web site, pngu.mgh.harvard.edu/-purcell/plink/).

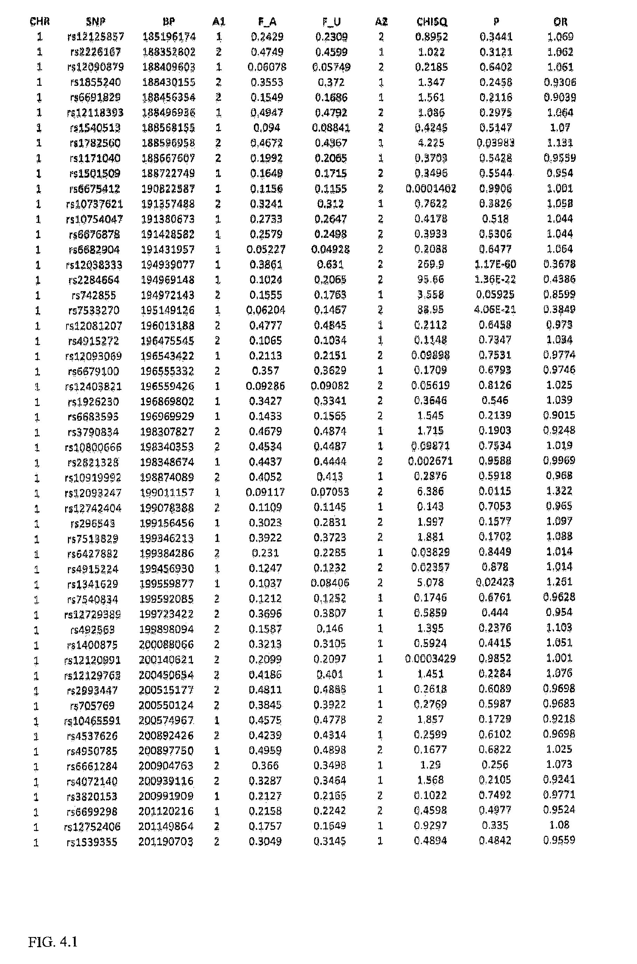

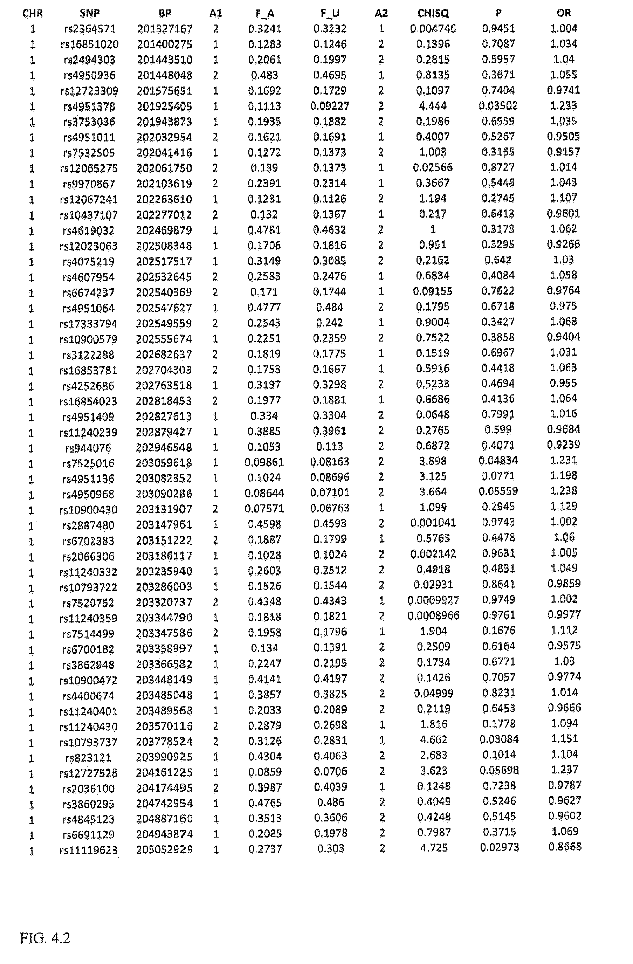

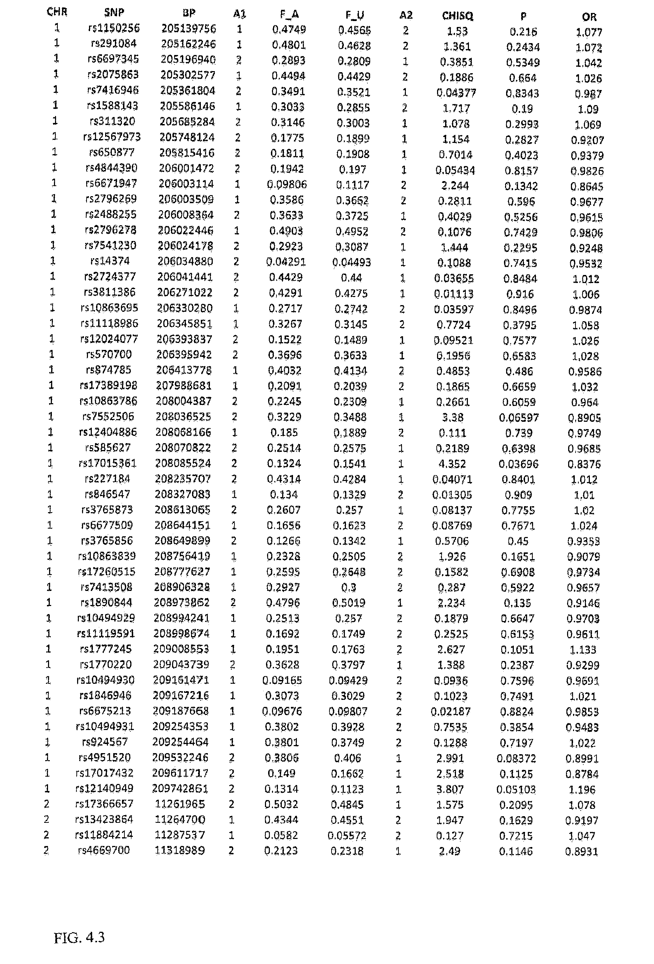

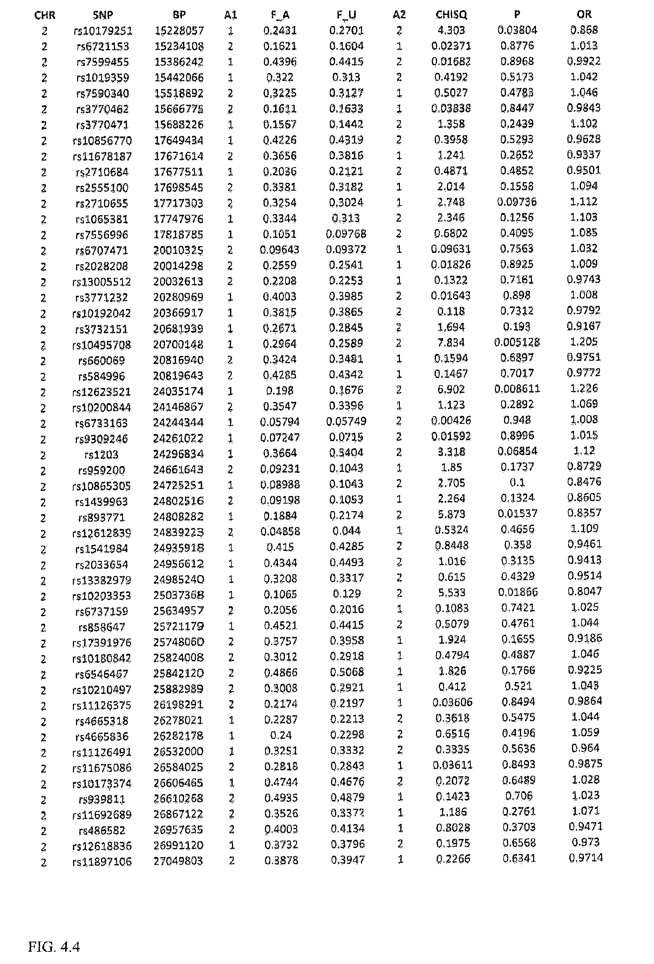

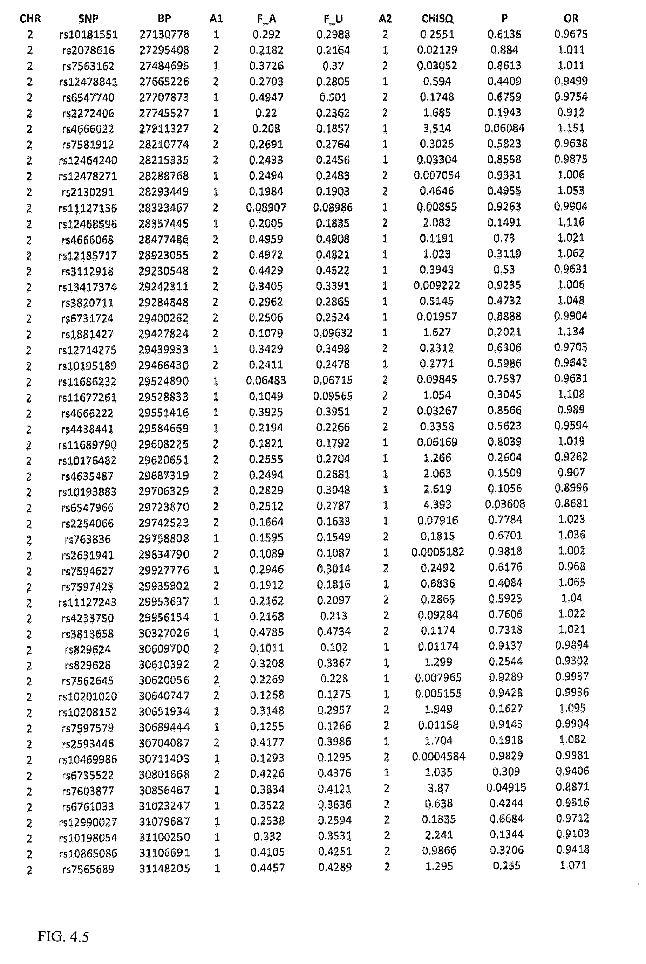

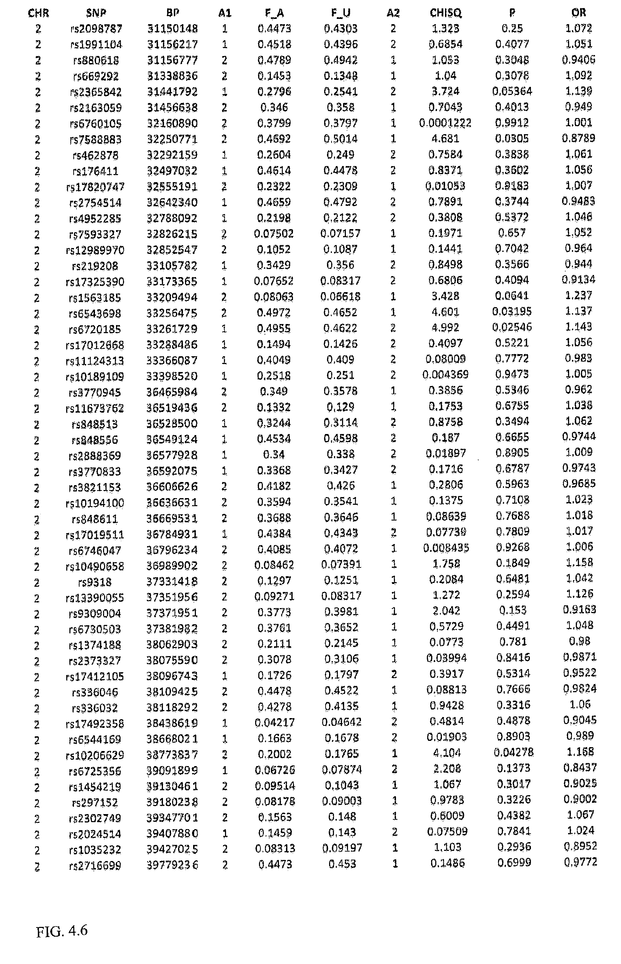

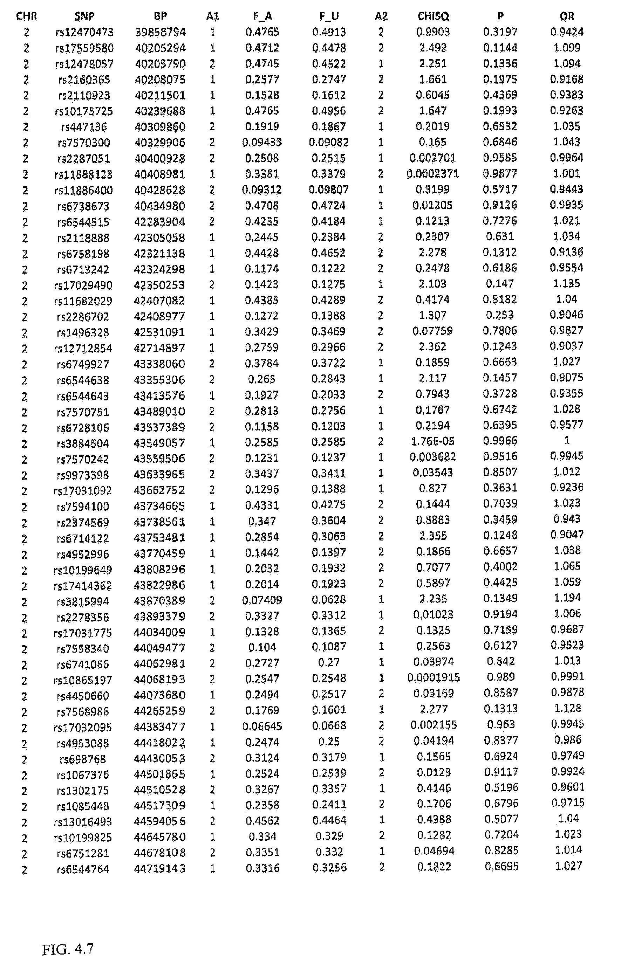

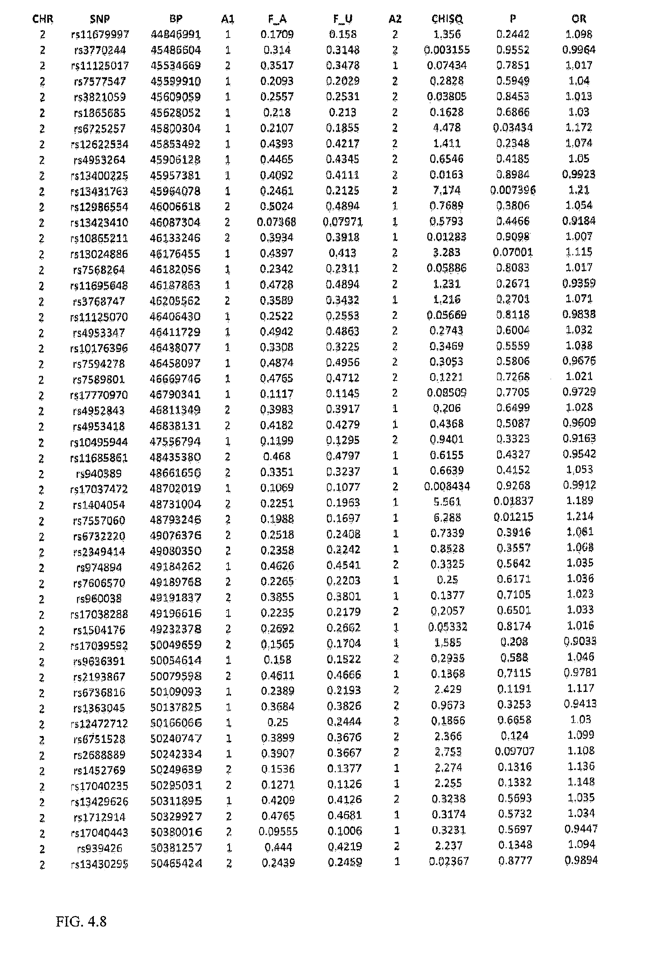

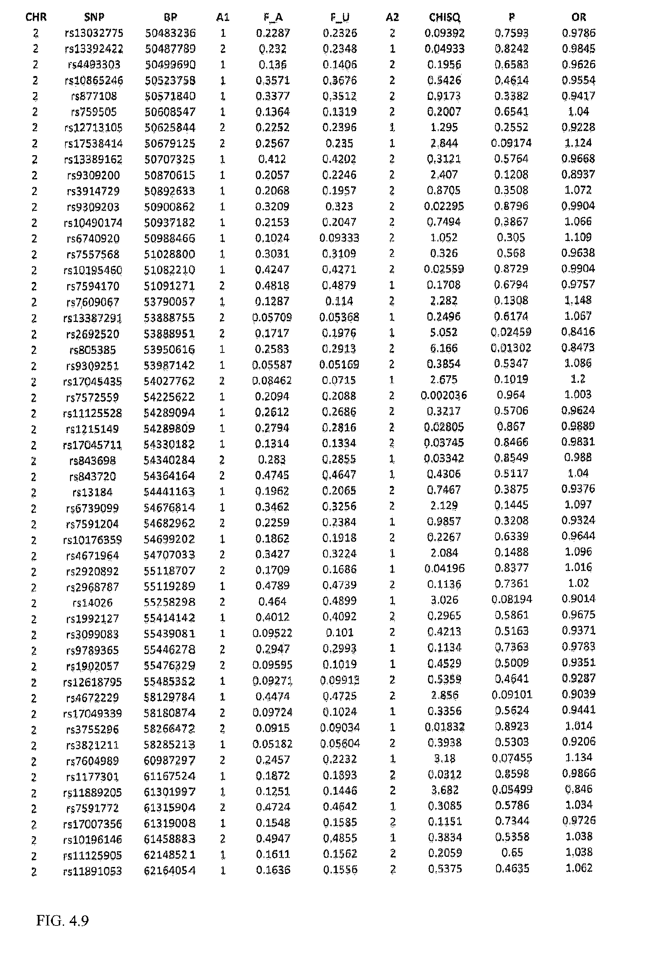

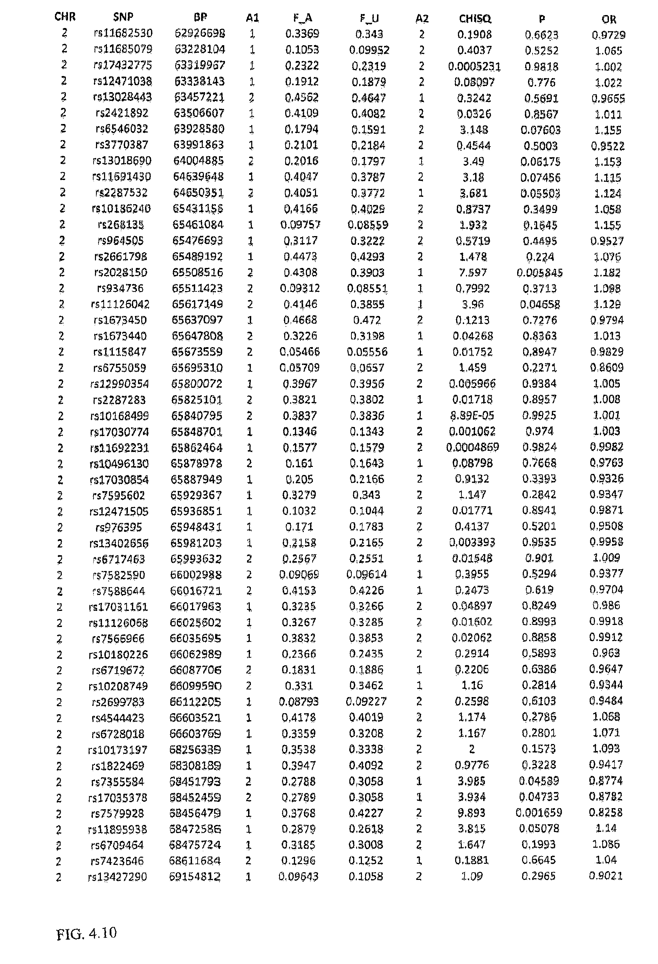

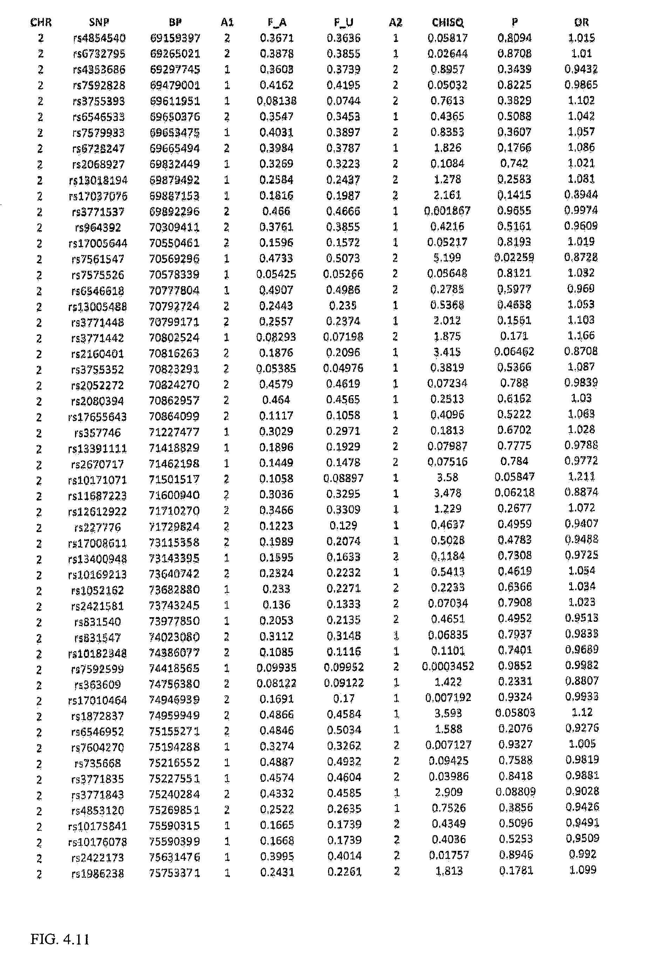

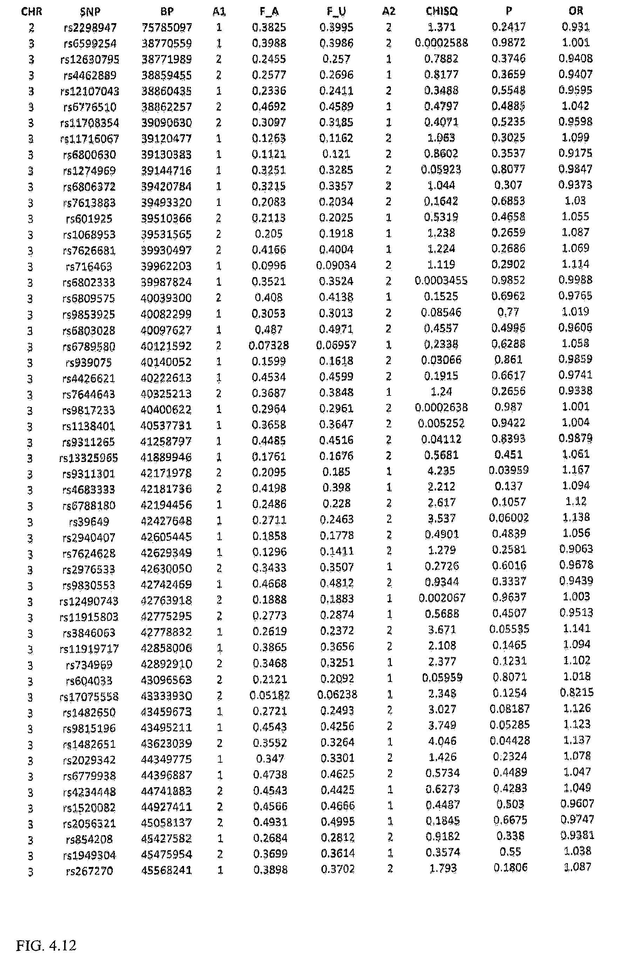

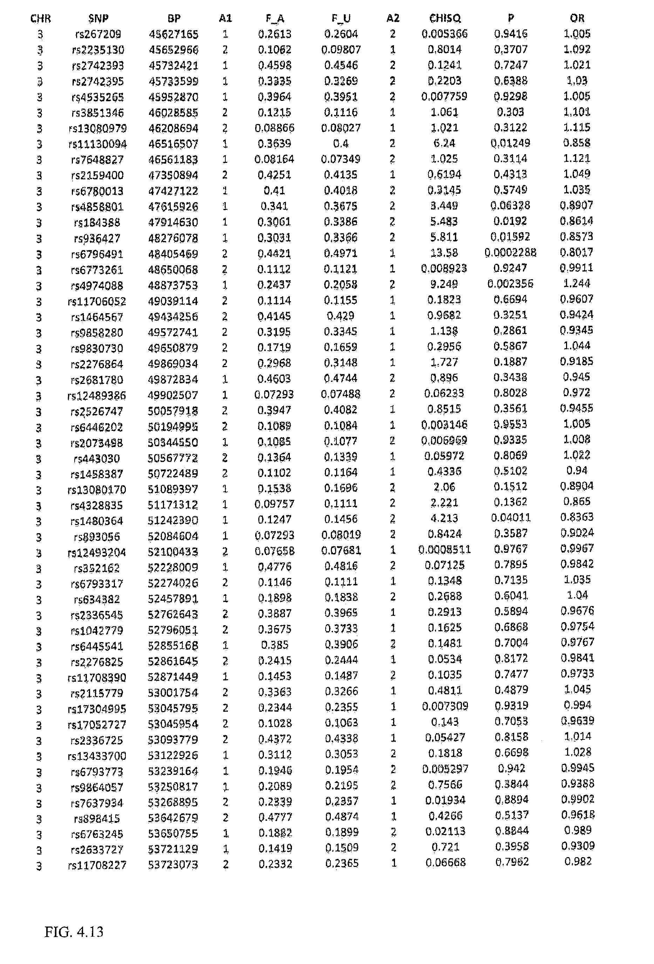

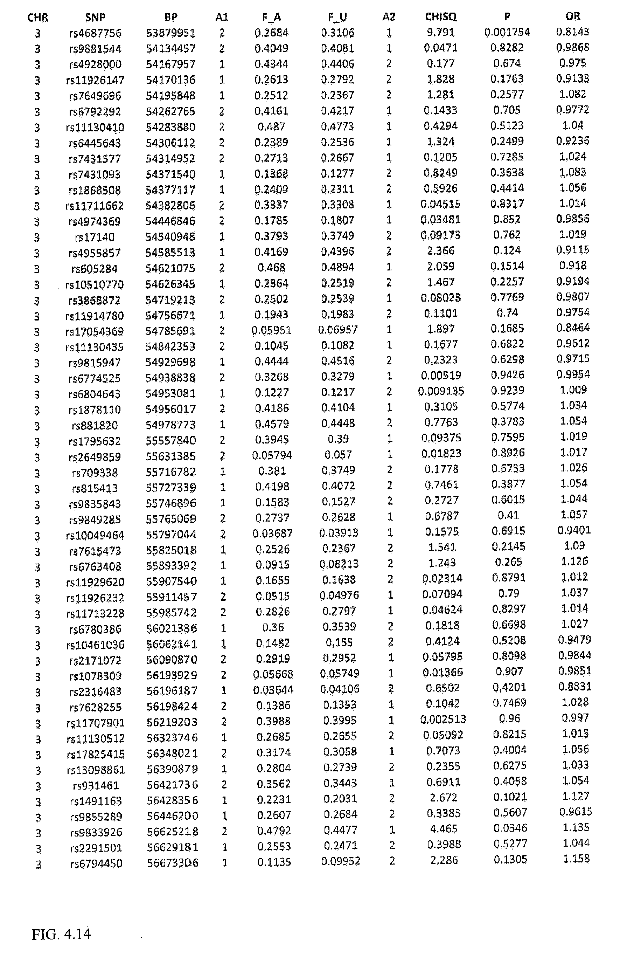

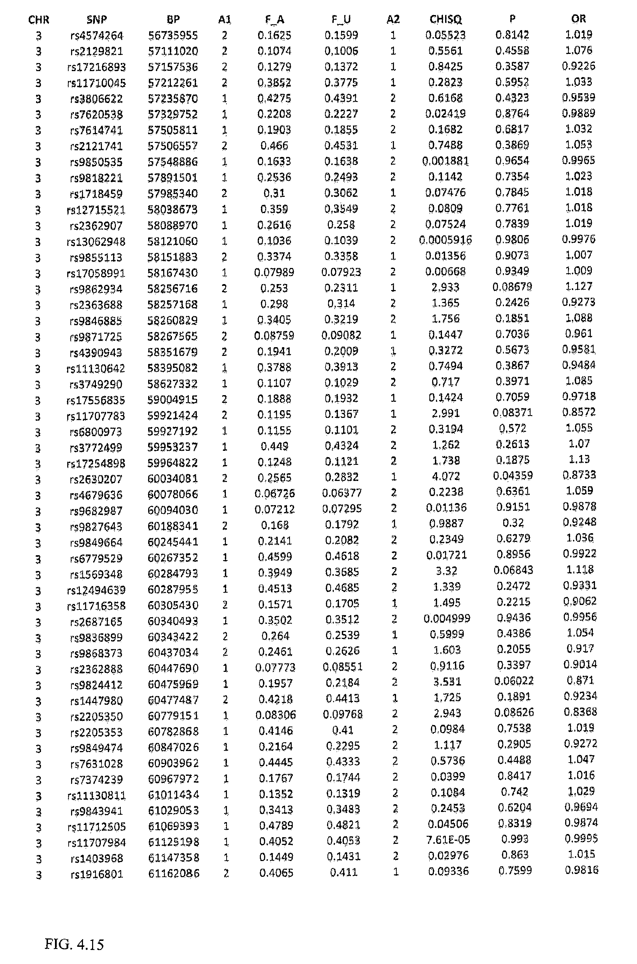

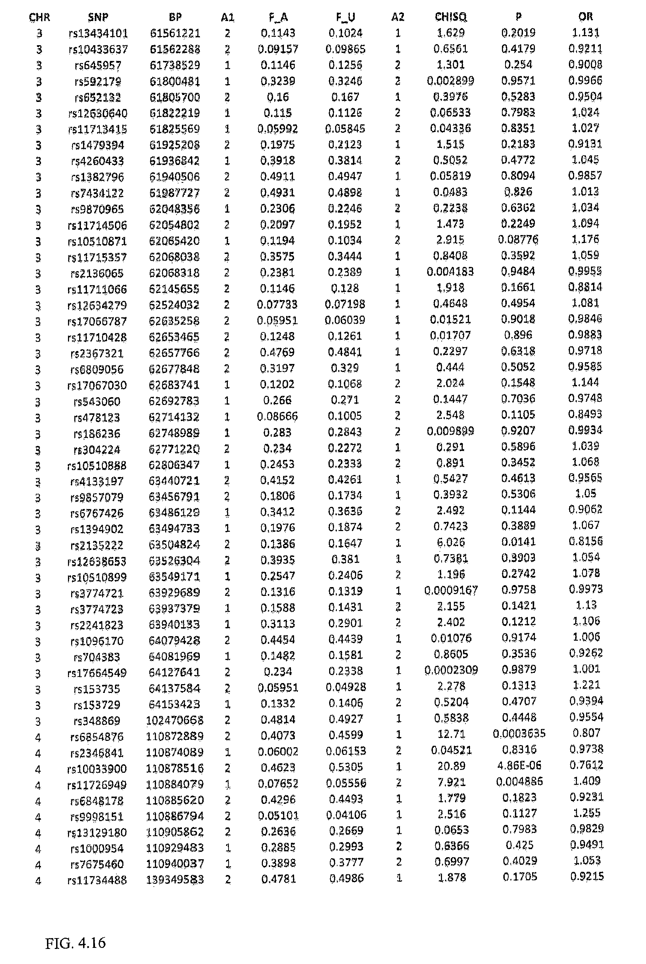

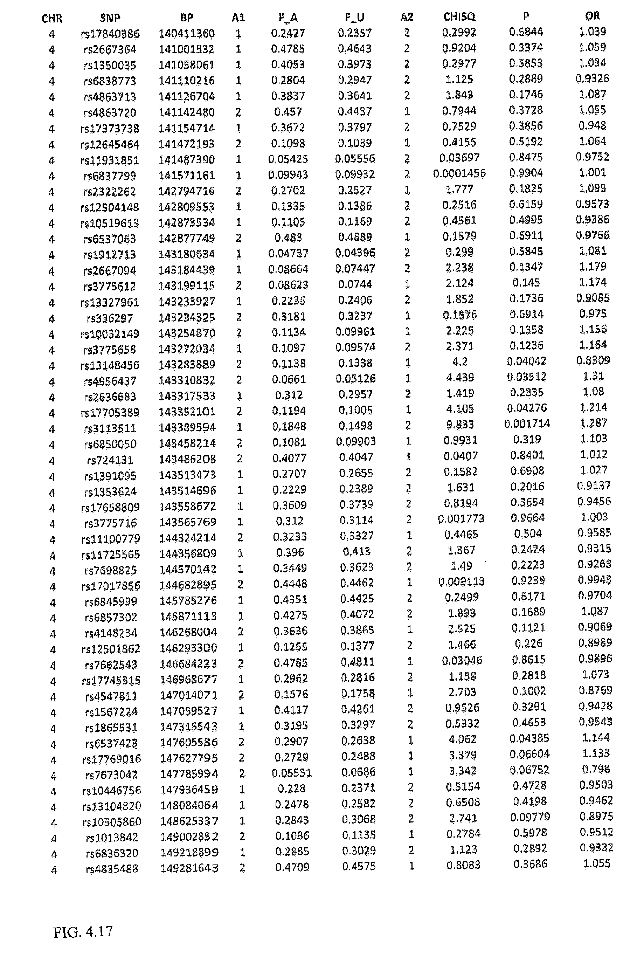

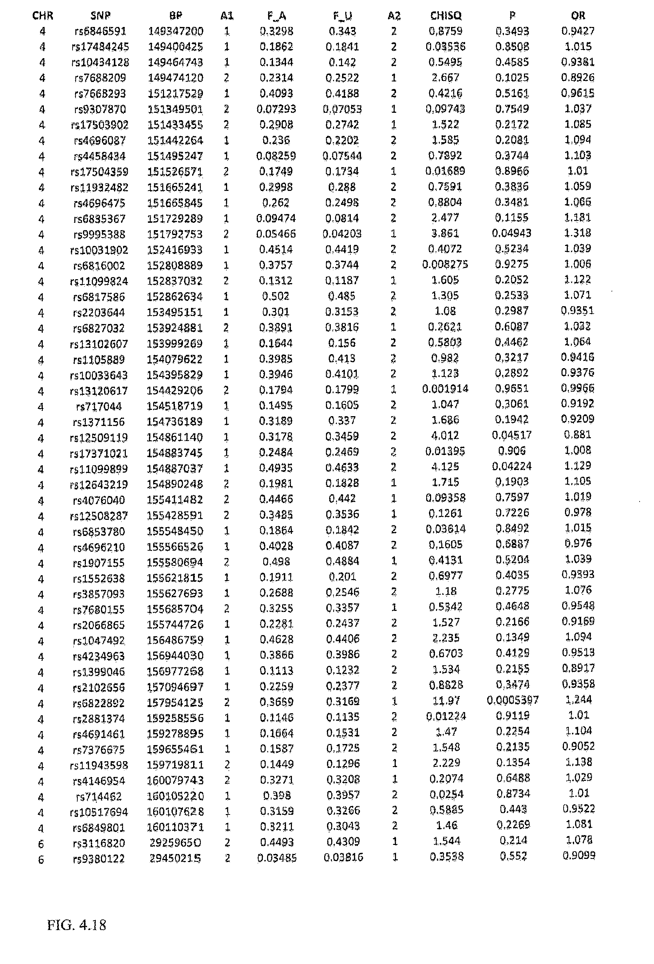

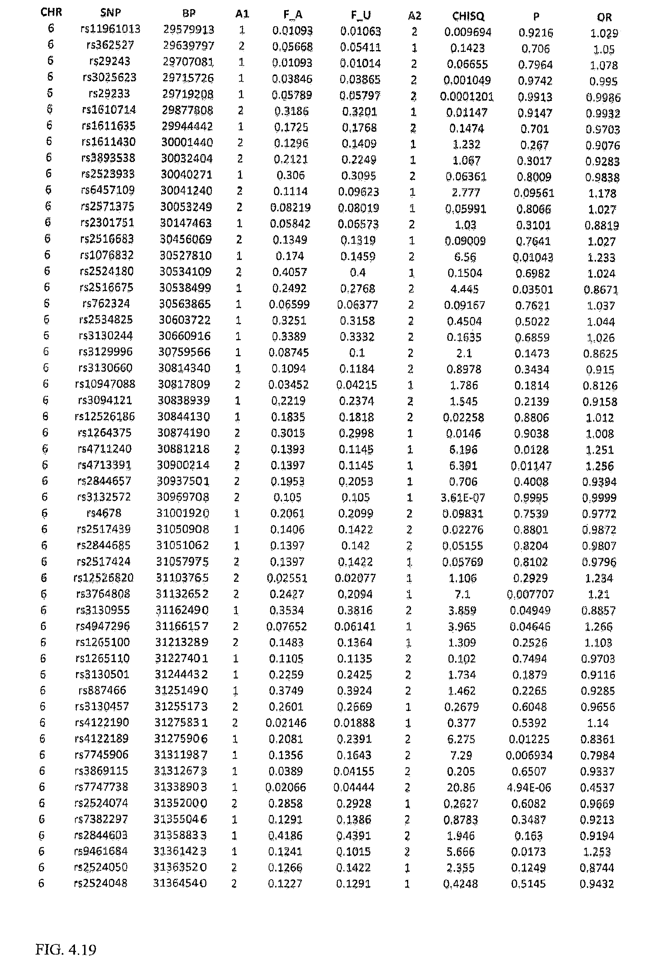

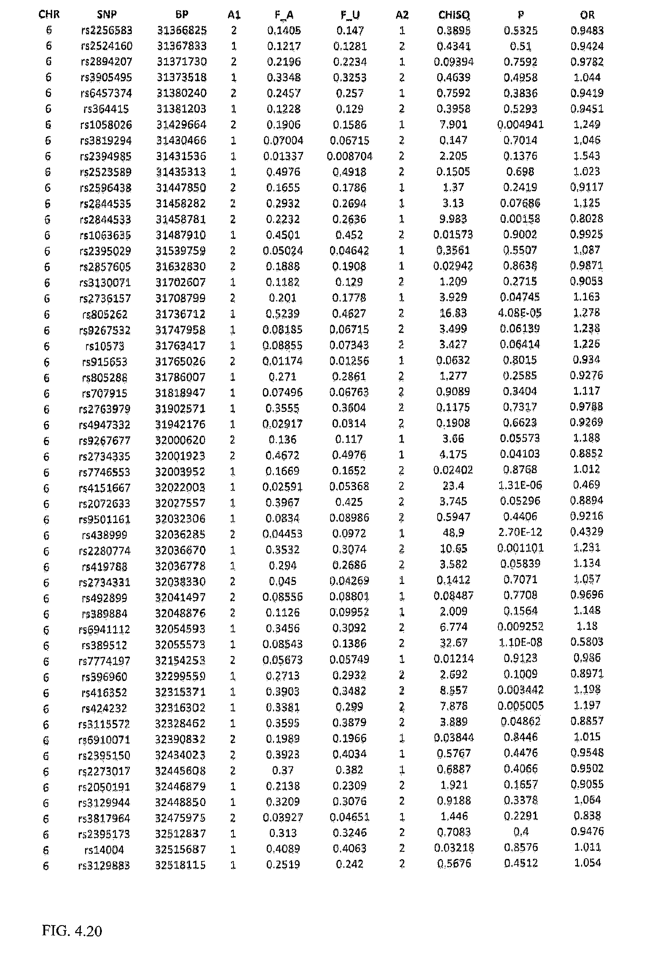

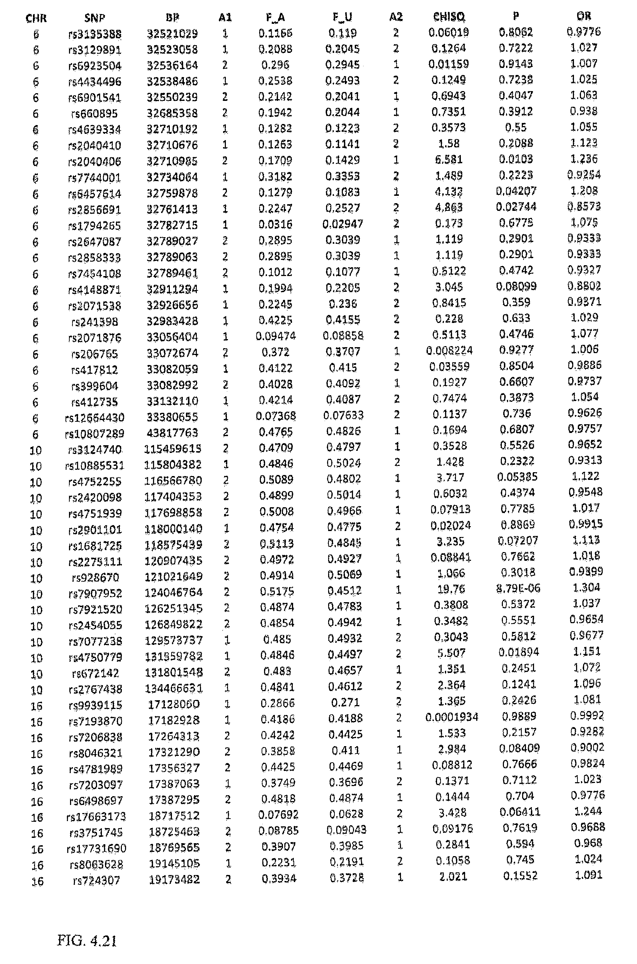

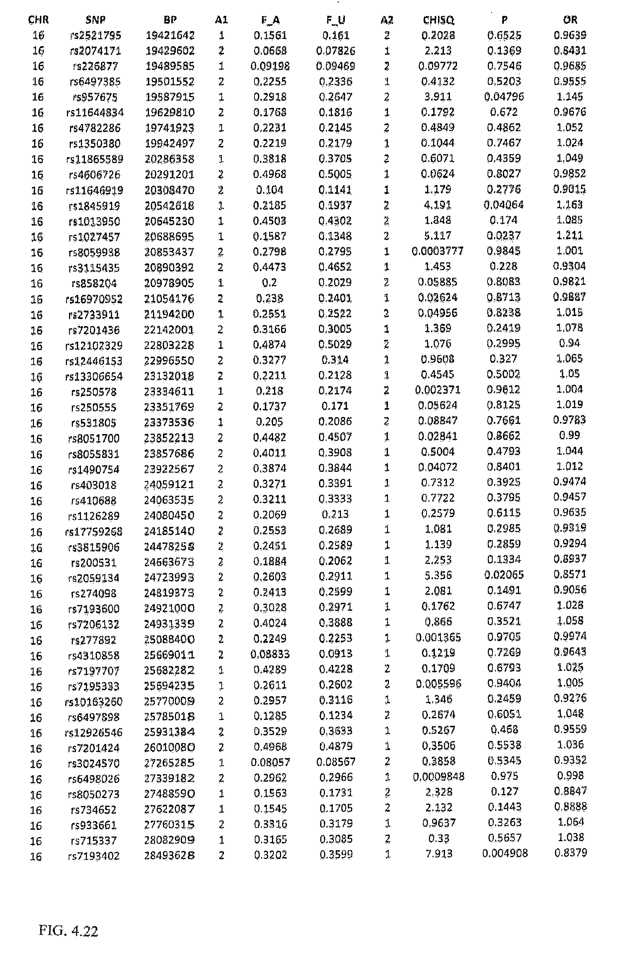

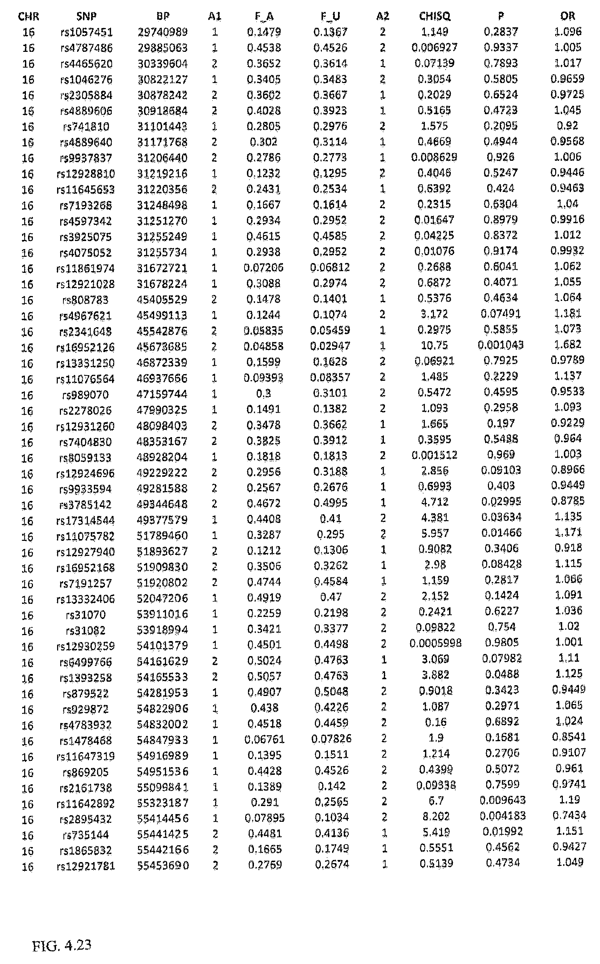

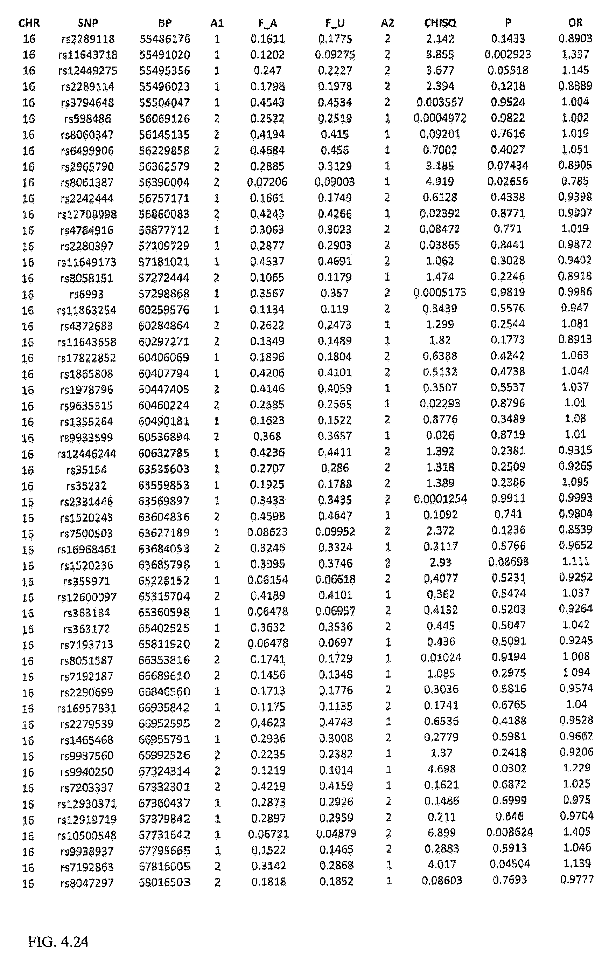

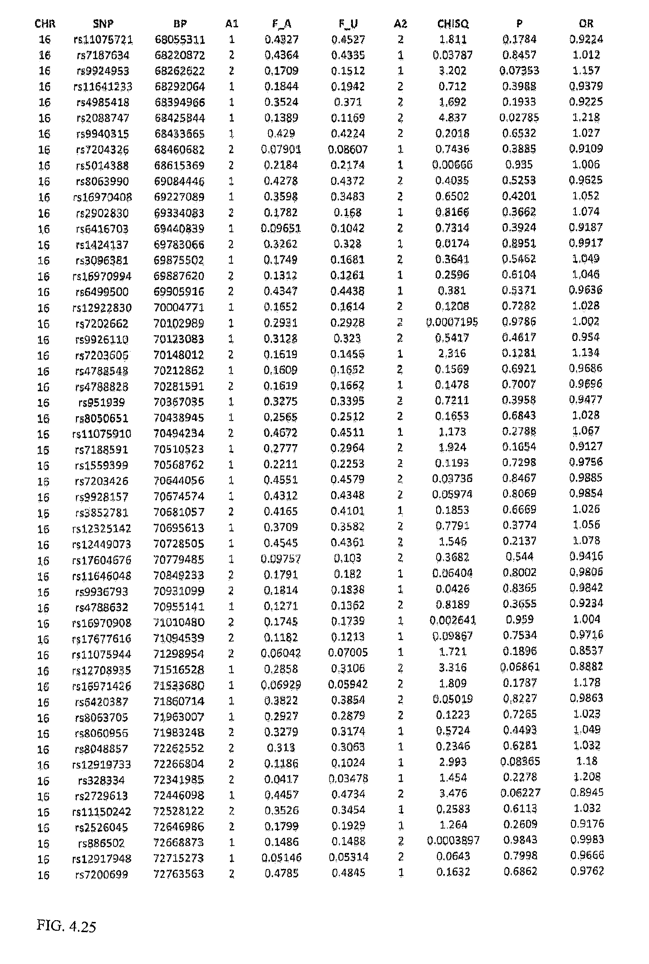

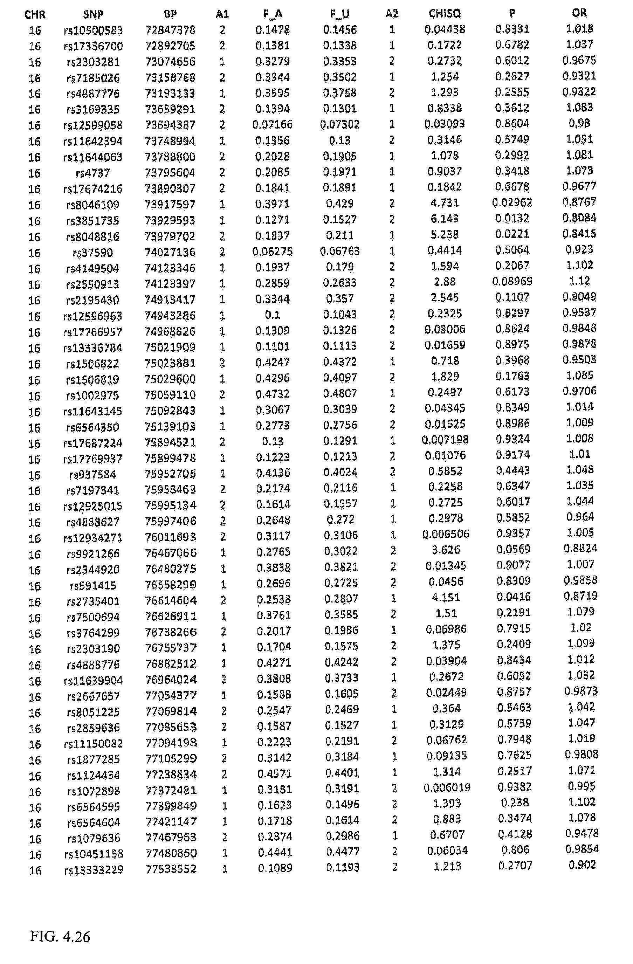

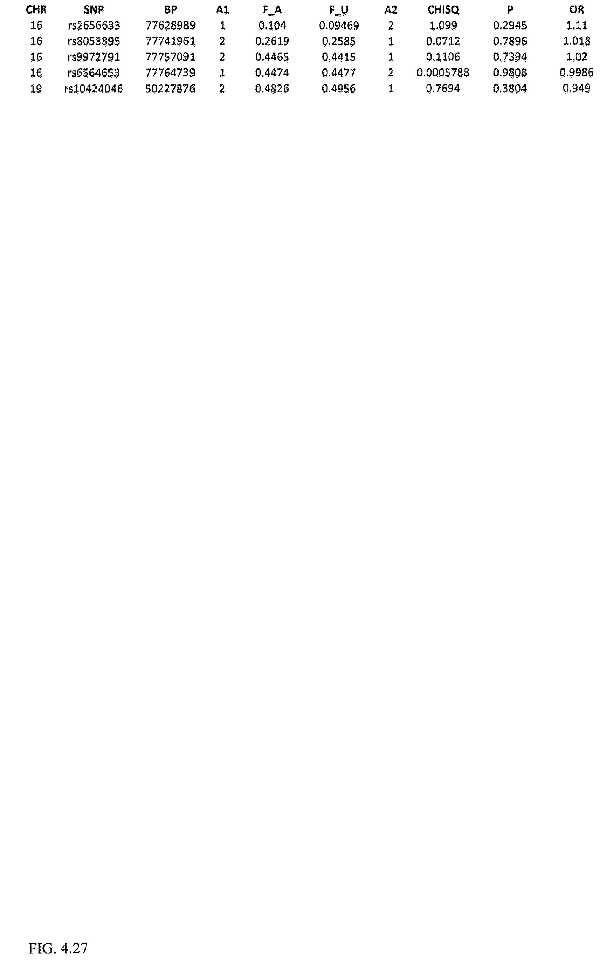

The most significantly associated SNP in the experiment, rs10033900 (p = 4.86 $\times$ 10$^{-6}$), resides in the chromosome 4 linkage peak and was significant even after a Bonferroni correction for 1409 (FIG. 4 shows the full results of the screen). Several nearby SNPs were also associated with p < 0.0005, suggesting this association was not due to a sporadic genotyping artifact. Remarkably, this SNP happened to be adjacent to the 3' UTR of Complement Factor I (CFI).

Given this compelling result, a much higher density of SNPs in this region was genotyped in an expanded panel of samples. The original SNP (rs10033900) remained the most highly associated SNP with a p-value = 6.46 $\times$ 10$^{-8}$ (OR = 0.7056 indicating a protective effect for the C allele) (Table 7).

29 SNPs were tested across this region for association, based on the most associated SNP, rs10033900. No significant independent associations were identified. Modest residual association at two neighboring, highly correlated SNPs (Table 7) was observed, however. This result suggests that rs10033900 may not be the causal variant but may be highly correlated with said variant. Therefore, multimarker haplotype tests were performed in an attempt to refine and isolate the association signal. The two-marker haplotype of the two closest SNPs to rs10033900, both 5' (rs13117504) and 3' (rs11726949) were tested. The two-marker haplotype between rs13117504 and rs10033900 shows a somewhat stronger association to AMD than either SNP alone with a p-value = 1.18 $\times$ 10$^{-8}$ (Table 8). None of these three SNPs appear to be functional, although rs11726949 is in Intron 11 of CFI. When a search was performed for differences in association between the neovascular ("wet") and geographic atrophy ("dry") forms of advanced AMD, only a 0.2% difference in minor allele frequency (46%) between the two groups.

To determine whether an obvious functional variant exists that explains this association, all exons within the span of LD defined by HapMap were sequenced (all markers correlated to the associated SNPs reside ($r^2$ > 0.35) in this haplotype block). This block of LD spans the exonic regions of the 3' end of CFI and all four exons of phospholipase A(2) Group 12A (PLA2G12A). No SNPs were found in either gene transcript that could statistically explain the association observed at rs10033900. A novel SNP just 5' of exon 12 in CFI was found, but this SNP does not appear to be in high $r^2$ with the associated CFI SNP or haplotype and is therefore not the biological source of association.

The role of epistasis between rs10033900 and rs13117504 and the six variants previously established to be associated with AMD was examined. Using logistic regression, no statistically significant interaction terms between any pair of these SNPs was found. While weak interactions cannot be excluded, this result suggests that despite targeting the same pathway, these variants largely confer risk in an independent, log-additive fashion.

Given the independent action of this new variant, we were able to add it to the multi-locus risk model. Since the individual and combined effects of the AMD-associated variants are additive, the overall proportion of population variance in risk (assuming a prevalence of late-stage AMD in this age group to be 5%) explained by this locus is estimated to be roughly 1% (assuming an underlying normal distribution N(0,1) of liability across the population).

The CFI gene spans 63 kb and contains 13 exons, the first 8 of which encode the heavy chain and the last 5 the light chain, which contains the serine protease domain (Vyse, T. et al., Genomics, 24:90-98, 1994). This serine protease domain is responsible for cleaving and inactivating the activities of C4b and C3b (Catterall, C. et al., Biochem. J., 242:849-856, 1987). C3b inactivation by CFI is regulated by Complement Factor H (CFH). CFH acts as a cofactor for CFI-mediated cleavage of C3b and also has decay accelerating activity against the alternative pathway C3 convertase, C3bBb. MCP also acts as a cofactor for CFI-mediated cleavage of C3b by down regulating the complement cascade (Jha, P. et al., Mol. Immunol., 44:3997-4003,2004).

Specific alleles associated with AMD or a susceptibility to AMD are as follows:

rs13117504:

TGAGATGACCTGACTCCAAGCTTCTCCTAGTTTAGAGGTCTGTCTCAGCG
CTCCTAATTCCAGACTACAGAAGCCAATCTAACTGGTTTAATGAAAAAA
TAGATTTATTCAAAGATATTATGCAGTTCACAGAATTTCCAAAAGAGCCA
GAGAATTGGAGTCTACACATCTGGAAATAACGCTCACAGACCACACTGC
AG[C/G]ACTGCTCCATCAGAGACTACCATGGCCACTAGCATGGGTCCCAG
TCTCCACGTACAGCTTGTACTGCAGAGCCTGGGCACTGGACATTGCTGCT
TCTGTGAGCTCGCCTGAAGGTGGCCAGGGACACAACTCATTGGATGTGG
AGCTCTGCCATCGTCTTATATTTAACCCTGGTTTCAGAGCTCTCTGTCTTA
CAAGAG (SEQ ID NO:8)

rs10033900:

AAAAGTACTCCAGTGCTACAAGGTGGGAAACCCAGCATATAGGGCATCC
TCAGCCAATCTAGGAAGGGGGCCCCAAAAGGGCCAAGCCAGTCTGCAC
AGTGACCTAGCATCTGGCAGCTTCACAGAAAGTAGACCAGGGTCAAGC
CTTGAAGGGTGAAAGTCAGCCCTCTGAAGCGACTCTATGTGACAGAGAC
CAGG[A/G]ACAGCAGGAGTGAGATGACCTGACTCCAAGCTTCTCCTAGTT
TAGAGGTCTGTCTCAGCGCTCCTAATTCCAGACTACAGAAGCCAATCTAA
CTGGTTTAATGAAAAAATAGATTTATTCAAAGATATTATGCAGTTCACAG
AATTTCCAAAAGAGCCAGAGAATTGGAGTCTACACATCTGGAAATAACG
CTCACAGAC (SEQ ID NO:9)

CFI SNP:

TACATCTTGACATCTTGGATAAACCACTTGGCACTTACCTGCACATTCCA
TTTCTTTTTCATAGAAACGATTTCCGTAAAACTTAGAGCAGTTGCTTATT
AGTTAACTTCACCCCACTGAAGTGAAAAGACTCTTTCGTTATCTAAACA
AAGTGAGAAAGCAAACATTTAGAAGTCACAAATGAGAAATCTAAATAC
ATACTCTCATAACTTAAACCATTGGGATTATGAAAGGGTGTATAGTTTTC
AATATAT[A/G]TAAATTTTTGAGAACTCTTCCTTAGCGTGTTTAATCATAT
CATATGTCTATTTCTAAAATTGGATGGATAGTCAAGGGGGACTATTGAAC
ATATGGTCTGAAGTCACATTCATTTAATAAAGAGCTGGTACCTTAAGTTG
AATGAGATAAATCTTTCCCTTTTGGTTGCAGAACTCAGTCAGGCAATTGG
ATAGGAATCAGTGATAAGGTCTTTTCACATAAATACCTCCTATCTTCTCA
CAGTCCCTTATTTCC (SEQ ID NO:10)

Table 7. 29 SNPs Tested Across PLA2G12A and CFI

| CHR | SNP | hg18 | Allele 1 | MAF Affected | MAF UnAffected | Allele 2 | CHISQ | P-Value | Odds Ratio | Conditional rs10033900 |
|---|---|---|---|---|---|---|---|---|---|---|
| 4 | rs13101299 | 110787671 | A | 10.0% | 8.9% | G | 1.40 | 0.2371 | 1.138 | 0.1229 |
| 4 | rs9990765 | 110788160 | C | 30.1% | 36.5% | T | 18.15 | 2.040E-05 | 0.75 | 0.1135 |
| 4 | rs4698774 | 110791792 | G | 27.5% | 27.6% | C | 0.00 | 0.9534 | 0.9958 | 0.7822 |
| 4 | rs6830606 | 110793498 | C | 9.7% | 8.5% | T | 1.74 | 0.1874 | 1.158 | 0.8431 |
| 4 | rs7690921 | 110798195 | T | 35.4% | 29.5% | A | 15.59 | 7.870E-05 | 1.312 | 0.2237 |
| 4 | rs17440280 | 110801549 | C | 37.5% | 36.8% | T | 0.21 | 0.6477 | 1.031 | 0.6611 |
| 4 | rs4698779 | 110816366 | G | 12.9% | 13.9% | C | 0.89 | 0.3460 | 0.9159 | 0.2519 |
| 4 | rs1800627 | 110833107 | T | 45.8% | 51.4% | C | 12.53 | 4.013E-04 | 0.7981 | 0.7469 |
| 4 | rs768063 | 110839905 | A | 3.8% | 3.3% | G | 0.89 | 0.3450 | 1.18 | 0.3177 |
| 4 | rs5030535 | 110841576 | G | 34.3% | 40.3% | A | 14.82 | 1.182E-04 | 0.7759 | 0.6100 |
| 4 | rs2285714 | 110858259 | T | 46.2% | 38.8% | C | 21.35 | 3.835E-06 | 1.35 | 0.4456 |
| 4 | rs2107047 | 110867224 | A | 7.4% | 8.2% | G | 0.82 | 0.3639 | 0.8982 | 0.6670 |
| 4 | rs6854876 | 110872889 | C | 40.1% | 47.3% | G | 18.86 | 1.410E-05 | 0.7466 | 0.1183 |
| 4 | rs2346841 | 110874089 | A | 6.0% | 6.4% | G | 0.26 | 0.6068 | 0.9312 | 0.4367 |
| 4 | rs13117504 | 110878305 | G | 37.7% | 45.8% | C | 26.93 | 2.110E-07 | 0.7154 | 0.02806 |
| 4 | rs10033900 | 110878516 | C | 46.0% | 54.7% | T | 29.22 | 6.460E-08 | 0.7056 | NA |
| 4 | rs11726949 | 110884079 | T | 7.9% | 5.0% | C | 11.96 | 5.442E-04 | 1.627 | 0.007167 |
| 4 | rs6848178 | 110885620 | T | 42.8% | 46.0% | A | 3.71 | 0.0542 | 0.8787 | 0.4991 |
| 4 | rs6822976 | 110885741 | A | 48.4% | 49.0% | G | 0.15 | 0.6975 | 0.9755 | 0.2998 |
| 4 | rs9998151 | 110886794 | C | 5.2% | 3.7% | T | 4.32 | 0.0377 | 1.41 | 0.2001 |
| 4 | rs4698784 | 110888710 | A | 32.6% | 31.3% | T | 0.72 | 0.3969 | 1.061 | 0.4749 |
| 4 | rs11098043 | 110891734 | G | 23.8% | 24.9% | A | 0.55 | 0.4569 | 0.9461 | 0.8048 |
| 4 | rs13129180 | 110905862 | T | 26.3% | 27.2% | A | 0.43 | 0.5127 | 0.9519 | 0.8159 |
| 4 | rs1000954 | 110929483 | A | 28.9% | 30.6% | G | 1.36 | 0.2432 | 0.9184 | 0.6391 |
| 4 | rs4698788 | 110938983 | C | 2.8% | 2.7% | T | 0.04 | 0.8468 | 1.039 | 0.6855 |
| 4 | rs7675460 | 110940037 | A | 39.1% | 37.6% | C | 0.84 | 0.3588 | 1.065 | 0.2338 |
| 4 | rs4698792 | 110948753 | T | 29.2% | 30.3% | C | 0.61 | 0.4337 | 0.9468 | 0.9426 |
| 4 | rs1002989 | 110953291 | C | 8.7% | 5.9% | T | 11.19 | 8.245E-04 | 1.527 | 0.02009 |
| 4 | rs4422417 | 110961059 | G | 14.1% | 11.9% | A | 3.95 | 0.0468 | 1.213 | 0.0729 |

Table 8: Two-marker Haplotype Association Results for most significant SNPs in CFI region

| SNPs | Haplotype | Frequency of Affected | Frequency of Unaffected | DF | CHISQ | P-Value | OR |
|---|---|---|---|---|---|---|---|
| rs13117504\|rs10033900 | OMNIBUS | NA | NA | 3 | 34.61 | 1.48E-07 | NA |
| rs13117504\|rs10033900 | CT | 50.07% | 40.92% | 1 | 32.52 | 1.18E-08 | 1.448 |
| rs13117504\|rs10033900 | GC | 33.64% | 41.48% | 1 | 25.51 | 4.39E-07 | 0.715 |
| rs13117504\|rs10033900 | CC | 12.39% | 13.25% | 1 | 0.6447 | 0.4220 | 0.926 |
| rs13117504\|rs10033900 | GT | 3.90% | 4.35% | 1 | 0.5068 | 0.4765 | 0.891 |

**Claims**

1. A method for diagnosing age-related macular degeneration or a susceptibility to age-related macular degeneration comprising detecting the presence or absence of a guanidine allele at a polymorphic site rs13117504 (SEQ ID NO: 8), rs10033900 (SEQ ID NO: 9), or SEQ ID NO: 10 which are in linkage disequilibrium with complement factor I, wherein the allele is indicative of age-related macular degeneration or a susceptibility to age-related macular degeneration.

2. The method of claim 1, wherein the polymorphic site is a single nucleotide polymorphism.

3. The method of claim 1, wherein the presence or absence of a particular allele is detected by a hybridization assay.

4. The method of claim 1, wherein the presence or absence of a particular allele is determined using a microarray.

5. The method of claim 1, wherein the presence or absence of a particular allele is determined using an antibody.

6. The method of claim 1, further comprising detecting one or more genetic markers associated with age-related macular degeneration and a gene other than complement factor I.

7. The method of claim 6, wherein the one or more genetic markers are associated with C3, C5 or CFH.

8. A diagnostic system comprising a diagnostic array, an array reader, an image processor, a database having data records and information records, a processor, and an information output, wherein the system compiles and processes patient data and outputs information relating to the statistical probability of the patient developing AMD, and wherein the array comprises a polynucleotide probe comprising SEQ ID NO: 8, SEQ ID NO: 9, or SEQ ID NO: 10, or a complement thereof.

9. A method of determining the risk of a subject for developing age-related macular degeneration, comprising detecting the presence or absence of one or more alleles at one or more polymorphic sites rs13117504 (SEQ ID NO: 8), rs10033900 (SEQ ID NO: 9), or SEQ ID NO: 10 which are in linkage disequilibrium with complement factor I and age-related macular degeneration, wherein the subject has been determined to be at risk for developing age-related macular degeneration due to one or more environmental risk factors.

10. The method of Claim 9, wherein at least one of the one or more polymorphic sites is a single nucleotide polymorphism.

11. The method of claim 9, wherein the one or more environmental risk factors are selected from the group consisting of: obesity, smoking, vitamin and dietary supplement intake, use of alcohol or drugs, poor diet and a sedentary lifestyle.

12. The method of claim 1 or 9, wherein the method comprises detecting the presence or absence of a haplotype, wherein the haplotype comprises:

    (a) polymorphic site rs13117504 (SEQ ID NO: 8) which is in linkage disequilibrium thereto;
    (b) polymorphic site rs10033900 (SEQ ID NO: 9) which is in linkage disequilibrium thereto; or
    (c) the polymorphic site of SEQ ID NO: 10 which is in linkage disequilibrium thereto.

**Patentansprüche**

1. Verfahren zur Diagnose von altersbedingter Makuladegeneration oder einer Anfälligkeit für altersbedingte Makuladegeneration, das den Nachweis der Gegenwart oder Abwesenheit eines Guanidin-Allels an einer polymorphen Stelle rs13117504 (SEQ.-ID Nr. 8), rs10033900 (SEQ.-ID Nr. 9) oder SEQ.-ID Nr. 10, die im Kopplungsungleichgewicht mit Komplementfaktor I stehen, umfasst, worin das Allel altersbedingte Makuladegeneration oder eine Anfälligkeit für altersbedingte Makuladegeneration anzeigt.

2. Verfahren nach Anspruch 1, worin die polymorphe Stelle ein Einzelnukleotidpolymorphismus ist.

3. Verfahren nach Anspruch 1, worin die Gegenwart oder Abwesenheit eines bestimmten Allels durch einen Hybridisierungsassay nachgewiesen wird.

4. Verfahren nach Anspruch 1, worin die Gegenwart oder Abwesenheit eines bestimmten Allels unter Verwendung eines Mikroarrays bestimmt wird.

5. Verfahren nach Anspruch 1, worin die Gegenwart oder Abwesenheit eines bestimmten Allels unter Verwendung eines Antikörpers bestimmt wird.

6. Verfahren nach Anspruch 1, das weiter den Nachweis von einem oder mehreren genetischen Markern umfasst, die mit altersbedingter Makuladegeneration und einem Gen, das nicht Komplementfaktor I ist, in Verbindung stehen.

7. Verfahren nach Anspruch 6, worin der eine oder die mehreren genetischen Marker mit C3, C5 oder CFH in Verbindung stehen.

8. Diagnosesystem, das Folgendes umfasst: einen Diagnostik-Array, einen Array-Reader, einen Bildprozessor, eine Datenbank mit Datensätzen und Informationssätzen, einen Prozessor und eine Informationsausgabe, worin das System Patientendaten erstellt und verarbeitet und Information, die sich auf die statistische Wahrscheinlichkeit des Patienten zur Entwicklung von AMD bezieht, ausgibt und worin der Array eine Polynukleotidsonde umfasst, die SEQ.-ID Nr. 8, SEQ.-ID Nr. 9 oder SEQ.-ID Nr. 10 oder eine komplementäre Sequenz davon umfasst.

9. Verfahren zur Bestimmung des Risikos eines Subjekts zur Entwicklung von altersbedingter Makuladegeneration, das den Nachweis der Gegenwart oder Abwesenheit von einem oder mehreren Allelen an einer oder mehreren polymorphen Stellen rs13117504 (SEQ.-ID Nr. 8), rs10033900 (SEQ.-ID Nr. 9) oder SEQ.-ID Nr. 10, die im Kopplungsungleichgewicht mit Komplementfaktor I und altersbedingter Makuladegeneration stehen, umfasst, worin bei dem Subjekt bestimmt wurde, dass das Risiko zur Entwicklung von altersbedingter Makuladegeneration aufgrund von einem oder mehreren umweltbedingten Risikofaktoren besteht.

10. Verfahren nach Anspruch 9, worin mindestens eine der einen oder mehreren polymorphen Stellen ein Einzelnukleotidpolymorphismus ist.

11. Verfahren nach Anspruch 9, worin der eine oder die mehreren umweltbedingten Risikofaktoren aus der Gruppe ausgewählt sind, die aus Folgenden besteht: Fettleibigkeit, Rauchen, Einnahme von Vitamin- und Nahrungsergänzungsmitteln, Konsum von Alkohol oder Drogen, schlechte Ernährung und eine bewegungsarme Lebensführung.

12. Verfahren nach Anspruch 1 oder 9, worin das Verfahren den Nachweis der Gegenwart oder Abwesenheit eines Haplotyps umfasst, worin der Haplotyp Folgendes umfasst:

    (a) polymorphe Stelle rs13117504 (SEQ.-ID Nr. 8), die im Kopplungsungleichgewicht dazu steht;
    (b) polymorphe Stelle rs10033900 (SEQ.-ID Nr. 9), die im Kopplungsungleichgewicht dazu steht; oder
    (c) die polymorphe Stelle von SEQ.-ID Nr. 10, die im Kopplungsungleichgewicht dazu steht.

**Revendications**

1. Procédé de diagnostic de dégénération maculaire liée à l'âge ou d'une susceptibilité à la dégénération maculaire liée à l'âge comprenant la détection de la présence ou l'absence d'un allèle de guanidine en un site polymorphe rs13117504 (SEQ ID N° : 8), rs10033900 (SEQ ID N° : 9) ou SEQ ID N° : 10 qui sont en déséquilibre de liaison avec le facteur de complément I, dans lequel l'allèle est indicateur de dégénération maculaire liée à l'âge ou d'une susceptibilité à la dégénération maculaire liée à l'âge.

2. Procédé selon la revendication 1, dans lequel le site polymorphe est un polymorphisme d'un seul nucléotide.

3. Procédé selon la revendication 1, dans lequel la présence ou l'absence d'un allèle particulier est détectée par un dosage d'hybridation.

4. Procédé selon la revendication 1, dans lequel la présence ou l'absence d'un allèle particulier est déterminée en utilisant un microréseau.

5. Procédé selon la revendication 1, dans lequel la présence ou l'absence d'un allèle particulier est déterminée en utilisant un anticorps.

6. Procédé selon la revendication 1, comprenant en outre la détection d'un ou plusieurs marqueurs génétiques associés à la dégénération maculaire liée à l'âge et un gène autre que le facteur de complément I.

7. Procédé selon la revendication 6, dans lequel le ou les marqueurs génétiques sont associés à C3, C5 ou CFH.

8. Système diagnostique comprenant une matrice diagnostique, un lecteur de matrice, un processeur d'images, une base de données ayant des enregistrements de données et des enregistrements d'informations, un processeur et une sortie d'informations, dans lequel le système compile et traite des données de patients et émet des informations concernant la probabilité statistique du patient à développer une AMD, et dans lequel la matrice comprend une sonde polynucléotidique comprenant SEQ ID N° : 8, SEQ ID N° : 9 ou SEQ ID N° : 10, ou un complément de celle-ci.

9. Procédé de détermination du risque d'un sujet de développer une dégénération maculaire liée à l'âge, comprenant la détection de la présence ou l'absence d'un ou plusieurs allèles en un ou plusieurs sites polymorphes rs13117504 (SEQ ID N° : 8), rs10033900 (SEQ ID N° : 9) ou SEQ ID N° : 10 qui sont en déséquilibre de liaison avec le facteur de complément I et la dégénération maculaire liée à l'âge, dans lequel le sujet a été déterminé comme étant à risque de développer une dégénération maculaire liée à l'âge en raison d'un ou plusieurs facteurs de risque environnementaux.

10. Procédé selon la revendication 9, dans lequel au moins un du ou des sites polymorphes est un polymorphisme d'un seul nucléotide.

11. Procédé selon la revendication 9, dans lequel le ou les facteurs de risque environnementaux sont sélectionnés parmi le groupe constitué de : obésité, fumer, prise de vitamines et suppléments diététiques, utilisation d'alcool ou de drogues, mauvais régime et un style de vie sédentaire.

12. Procédé selon la revendication 1 ou 9, dans lequel le procédé comprend la détection de la présence ou l'absence d'un haplotype, dans lequel l'haplotype comprend :

(a) site polymorphe rs13117504 (SEQ ID N° : 8) qui est en déséquilibre de liaison à celui-ci ;
(b) site polymorphe rs10033900 (SEQ ID N° : 9) qui est en déséquilibre de liaison à celui-ci ; ou
(c) le site polymorphe de SEQ ID N° : 10 qui est en déséquilibre de liaison à celui-ci.

FIG. 1

FIG. 2

| CHR | SNP | BP | A1 | F_A | F_U | A2 | CHISQ | P | OR |
|---|---|---|---|---|---|---|---|---|---|
| 1 | rs12125857 | 185196174 | 1 | 0.2429 | 0.2309 | 2 | 0.8952 | 0.3441 | 1.069 |
| 1 | rs2226167 | 188352802 | 2 | 0.4749 | 0.4599 | 1 | 1.022 | 0.3121 | 1.062 |
| 1 | rs12090879 | 188409603 | 1 | 0.06078 | 0.05749 | 2 | 0.2185 | 0.6402 | 1.061 |
| 1 | rs1855240 | 188430155 | 2 | 0.3553 | 0.372 | 1 | 1.347 | 0.2458 | 0.9306 |
| 1 | rs6691829 | 188456354 | 2 | 0.1549 | 0.1686 | 1 | 1.561 | 0.2116 | 0.9039 |
| 1 | rs12118393 | 188496936 | 1 | 0.4947 | 0.4792 | 2 | 1.086 | 0.2975 | 1.064 |
| 1 | rs1540513 | 188568155 | 1 | 0.094 | 0.08841 | 2 | 0.4245 | 0.5147 | 1.07 |
| 1 | rs1782560 | 188596958 | 2 | 0.4672 | 0.4367 | 1 | 4.225 | 0.03983 | 1.131 |
| 1 | rs1171040 | 188667607 | 2 | 0.1992 | 0.2065 | 1 | 0.3703 | 0.5428 | 0.9559 |
| 1 | rs1501509 | 188722749 | 1 | 0.1649 | 0.1715 | 2 | 0.3496 | 0.5544 | 0.954 |
| 1 | rs6675412 | 190822587 | 1 | 0.1156 | 0.1155 | 2 | 0.0001402 | 0.9906 | 1.001 |
| 1 | rs10737621 | 191357488 | 2 | 0.3241 | 0.312 | 1 | 0.7622 | 0.3826 | 1.058 |
| 1 | rs10754047 | 191380673 | 1 | 0.2733 | 0.2647 | 2 | 0.4178 | 0.518 | 1.044 |
| 1 | rs6676878 | 191428582 | 1 | 0.2579 | 0.2498 | 2 | 0.3933 | 0.5306 | 1.044 |
| 1 | rs6682904 | 191431957 | 1 | 0.05227 | 0.04928 | 2 | 0.2088 | 0.6477 | 1.064 |
| 1 | rs12038333 | 194939077 | 1 | 0.3861 | 0.631 | 2 | 269.9 | 1.17E-60 | 0.3678 |
| 1 | rs2284664 | 194969148 | 1 | 0.1024 | 0.2065 | 2 | 95.66 | 1.36E-22 | 0.4386 |
| 1 | rs742855 | 194972143 | 2 | 0.1555 | 0.1763 | 1 | 3.558 | 0.05925 | 0.8599 |
| 1 | rs7533270 | 195149126 | 1 | 0.06204 | 0.1467 | 2 | 88.95 | 4.06E-21 | 0.3849 |
| 1 | rs12081207 | 196013188 | 2 | 0.4777 | 0.4845 | 1 | 0.2112 | 0.6458 | 0.973 |
| 1 | rs4915272 | 196475545 | 2 | 0.1065 | 0.1034 | 1 | 0.1148 | 0.7347 | 1.034 |
| 1 | rs12093069 | 196543422 | 1 | 0.2113 | 0.2151 | 2 | 0.09898 | 0.7531 | 0.9774 |
| 1 | rs6679100 | 196555332 | 2 | 0.357 | 0.3629 | 1 | 0.1709 | 0.6793 | 0.9746 |
| 1 | rs12403821 | 196559426 | 1 | 0.09286 | 0.09082 | 2 | 0.05619 | 0.8126 | 1.025 |
| 1 | rs1926230 | 196869802 | 1 | 0.3427 | 0.3341 | 2 | 0.3646 | 0.546 | 1.039 |
| 1 | rs6683595 | 196969929 | 1 | 0.1433 | 0.1565 | 2 | 1.545 | 0.2139 | 0.9015 |
| 1 | rs3790834 | 198307827 | 2 | 0.4679 | 0.4874 | 1 | 1.715 | 0.1903 | 0.9248 |
| 1 | rs10800666 | 198340353 | 2 | 0.4534 | 0.4487 | 1 | 0.09871 | 0.7534 | 1.019 |
| 1 | rs2821328 | 198348674 | 1 | 0.4437 | 0.4444 | 2 | 0.002671 | 0.9588 | 0.9969 |
| 1 | rs10919992 | 198874089 | 2 | 0.4052 | 0.413 | 1 | 0.2876 | 0.5918 | 0.968 |
| 1 | rs12093247 | 199011157 | 1 | 0.09117 | 0.07053 | 2 | 6.386 | 0.0115 | 1.322 |
| 1 | rs12742404 | 199078388 | 2 | 0.1109 | 0.1145 | 1 | 0.143 | 0.7053 | 0.965 |
| 1 | rs296543 | 199156456 | 1 | 0.3023 | 0.2831 | 2 | 1.997 | 0.1577 | 1.097 |
| 1 | rs7513829 | 199346213 | 1 | 0.3922 | 0.3723 | 2 | 1.881 | 0.1702 | 1.088 |
| 1 | rs6427882 | 199384286 | 2 | 0.231 | 0.2285 | 1 | 0.03829 | 0.8449 | 1.014 |
| 1 | rs4915224 | 199456930 | 1 | 0.1247 | 0.1232 | 2 | 0.02357 | 0.878 | 1.014 |
| 1 | rs1341629 | 199559877 | 1 | 0.1037 | 0.08406 | 2 | 5.078 | 0.02423 | 1.261 |
| 1 | rs7540834 | 199592085 | 2 | 0.1212 | 0.1252 | 1 | 0.1746 | 0.6761 | 0.9628 |
| 1 | rs12729389 | 199723422 | 2 | 0.3696 | 0.3807 | 1 | 0.5859 | 0.444 | 0.954 |
| 1 | rs492563 | 199898094 | 2 | 0.1587 | 0.146 | 1 | 1.395 | 0.2376 | 1.103 |
| 1 | rs1400875 | 200088066 | 2 | 0.3213 | 0.3105 | 1 | 0.5924 | 0.4415 | 1.051 |
| 1 | rs12120991 | 200140621 | 2 | 0.2099 | 0.2097 | 1 | 0.0003429 | 0.9852 | 1.001 |
| 1 | rs12129763 | 200450654 | 2 | 0.4186 | 0.401 | 1 | 1.451 | 0.2284 | 1.076 |
| 1 | rs2993447 | 200515177 | 2 | 0.4811 | 0.4888 | 1 | 0.2618 | 0.6089 | 0.9698 |
| 1 | rs705769 | 200550124 | 2 | 0.3845 | 0.3922 | 1 | 0.2769 | 0.5987 | 0.9683 |
| 1 | rs10465591 | 200574967 | 1 | 0.4575 | 0.4778 | 2 | 1.857 | 0.1729 | 0.9218 |
| 1 | rs4537626 | 200892426 | 2 | 0.4239 | 0.4314 | 1 | 0.2599 | 0.6102 | 0.9698 |
| 1 | rs4950785 | 200897750 | 1 | 0.4959 | 0.4898 | 2 | 0.1677 | 0.6822 | 1.025 |
| 1 | rs6661284 | 200904763 | 2 | 0.366 | 0.3498 | 1 | 1.29 | 0.256 | 1.073 |
| 1 | rs4072140 | 200939116 | 2 | 0.3287 | 0.3464 | 1 | 1.568 | 0.2105 | 0.9241 |
| 1 | rs3820153 | 200991909 | 1 | 0.2127 | 0.2166 | 2 | 0.1022 | 0.7492 | 0.9771 |
| 1 | rs6699298 | 201120216 | 1 | 0.2158 | 0.2242 | 2 | 0.4598 | 0.4977 | 0.9524 |
| 1 | rs12752406 | 201149864 | 2 | 0.1757 | 0.1649 | 1 | 0.9297 | 0.335 | 1.08 |
| 1 | rs1539355 | 201190703 | 2 | 0.3049 | 0.3145 | 1 | 0.4894 | 0.4842 | 0.9559 |

FIG. 4.1

| CHR | SNP | BP | A1 | F_A | F_U | A2 | CHISQ | P | OR |
|---|---|---|---|---|---|---|---|---|---|
| 1 | rs2364571 | 201327167 | 2 | 0.3241 | 0.3232 | 1 | 0.004746 | 0.9451 | 1.004 |
| 1 | rs16851020 | 201400275 | 1 | 0.1283 | 0.1246 | 2 | 0.1396 | 0.7087 | 1.034 |
| 1 | rs2494303 | 201443510 | 1 | 0.2061 | 0.1997 | 2 | 0.2815 | 0.5957 | 1.04 |
| 1 | rs4950936 | 201448048 | 2 | 0.483 | 0.4695 | 1 | 0.8135 | 0.3671 | 1.055 |
| 1 | rs12723309 | 201575651 | 1 | 0.1692 | 0.1729 | 2 | 0.1097 | 0.7404 | 0.9741 |
| 1 | rs4951378 | 201925405 | 1 | 0.1113 | 0.09227 | 2 | 4.444 | 0.03502 | 1.233 |
| 1 | rs3753036 | 201943873 | 1 | 0.1935 | 0.1882 | 2 | 0.1986 | 0.6559 | 1.035 |
| 1 | rs4951011 | 202032954 | 2 | 0.1621 | 0.1691 | 1 | 0.4007 | 0.5267 | 0.9505 |
| 1 | rs7532505 | 202041416 | 1 | 0.1272 | 0.1373 | 2 | 1.003 | 0.3165 | 0.9157 |
| 1 | rs12065275 | 202061750 | 2 | 0.139 | 0.1373 | 1 | 0.02566 | 0.8727 | 1.014 |
| 1 | rs9970867 | 202103619 | 2 | 0.2391 | 0.2314 | 1 | 0.3667 | 0.5448 | 1.043 |
| 1 | rs12067241 | 202263610 | 1 | 0.1231 | 0.1126 | 2 | 1.194 | 0.2745 | 1.107 |
| 1 | rs10437107 | 202277012 | 2 | 0.132 | 0.1367 | 1 | 0.217 | 0.6413 | 0.9601 |
| 1 | rs4619032 | 202469879 | 1 | 0.4781 | 0.4632 | 2 | 1 | 0.3173 | 1.062 |
| 1 | rs12023063 | 202508348 | 1 | 0.1706 | 0.1816 | 2 | 0.951 | 0.3295 | 0.9266 |
| 1 | rs4075219 | 202517517 | 1 | 0.3149 | 0.3085 | 2 | 0.2162 | 0.642 | 1.03 |
| 1 | rs4607954 | 202532645 | 2 | 0.2583 | 0.2476 | 1 | 0.6834 | 0.4084 | 1.058 |
| 1 | rs6674237 | 202540369 | 2 | 0.171 | 0.1744 | 1 | 0.09155 | 0.7622 | 0.9764 |
| 1 | rs4951064 | 202547627 | 1 | 0.4777 | 0.484 | 2 | 0.1795 | 0.6718 | 0.975 |
| 1 | rs17333794 | 202549559 | 2 | 0.2543 | 0.242 | 1 | 0.9004 | 0.3427 | 1.068 |
| 1 | rs10900579 | 202555674 | 1 | 0.2251 | 0.2359 | 2 | 0.7522 | 0.3858 | 0.9404 |
| 1 | rs3122288 | 202682637 | 2 | 0.1819 | 0.1775 | 1 | 0.1519 | 0.6967 | 1.031 |
| 1 | rs16853781 | 202704303 | 2 | 0.1753 | 0.1667 | 1 | 0.5916 | 0.4418 | 1.063 |
| 1 | rs4252686 | 202763518 | 1 | 0.3197 | 0.3298 | 2 | 0.5233 | 0.4694 | 0.955 |
| 1 | rs16854023 | 202818453 | 2 | 0.1977 | 0.1881 | 1 | 0.6686 | 0.4136 | 1.064 |
| 1 | rs4951409 | 202827613 | 1 | 0.334 | 0.3304 | 2 | 0.0648 | 0.7991 | 1.016 |
| 1 | rs11240239 | 202879427 | 1 | 0.3885 | 0.3961 | 2 | 0.2765 | 0.599 | 0.9684 |
| 1 | rs944076 | 202946548 | 1 | 0.1053 | 0.113 | 2 | 0.6872 | 0.4071 | 0.9239 |
| 1 | rs7525016 | 203059618 | 1 | 0.09861 | 0.08163 | 2 | 3.898 | 0.04834 | 1.231 |
| 1 | rs4951136 | 203082352 | 1 | 0.1024 | 0.08696 | 2 | 3.125 | 0.0771 | 1.198 |
| 1 | rs4950968 | 203090286 | 1 | 0.08644 | 0.07101 | 2 | 3.664 | 0.05559 | 1.238 |
| 1 | rs10900430 | 203131907 | 2 | 0.07571 | 0.06763 | 1 | 1.099 | 0.2945 | 1.129 |
| 1 | rs2887480 | 203147961 | 1 | 0.4598 | 0.4593 | 2 | 0.001041 | 0.9743 | 1.002 |
| 1 | rs6702383 | 203151222 | 2 | 0.1887 | 0.1799 | 1 | 0.5763 | 0.4478 | 1.06 |
| 1 | rs2066306 | 203186117 | 1 | 0.1028 | 0.1024 | 2 | 0.002142 | 0.9631 | 1.005 |
| 1 | rs11240332 | 203235940 | 1 | 0.2603 | 0.2512 | 2 | 0.4918 | 0.4831 | 1.049 |
| 1 | rs10793722 | 203286003 | 1 | 0.1526 | 0.1544 | 2 | 0.02931 | 0.8641 | 0.9859 |
| 1 | rs7520752 | 203320737 | 2 | 0.4348 | 0.4343 | 1 | 0.0009927 | 0.9749 | 1.002 |
| 1 | rs11240359 | 203344790 | 1 | 0.1818 | 0.1821 | 2 | 0.0008966 | 0.9761 | 0.9977 |
| 1 | rs7514499 | 203347586 | 2 | 0.1958 | 0.1796 | 1 | 1.904 | 0.1676 | 1.112 |
| 1 | rs6700182 | 203358997 | 1 | 0.134 | 0.1391 | 2 | 0.2509 | 0.6164 | 0.9575 |
| 1 | rs3862948 | 203366582 | 1 | 0.2247 | 0.2195 | 2 | 0.1734 | 0.6771 | 1.03 |
| 1 | rs10900472 | 203448149 | 1 | 0.4141 | 0.4197 | 2 | 0.1426 | 0.7057 | 0.9774 |
| 1 | rs4400674 | 203485048 | 1 | 0.3857 | 0.3825 | 2 | 0.04999 | 0.8231 | 1.014 |
| 1 | rs11240401 | 203489568 | 1 | 0.2033 | 0.2089 | 2 | 0.2119 | 0.6453 | 0.9666 |
| 1 | rs11240430 | 203570116 | 2 | 0.2879 | 0.2698 | 1 | 1.816 | 0.1778 | 1.094 |
| 1 | rs10793737 | 203778524 | 2 | 0.3126 | 0.2831 | 1 | 4.662 | 0.03084 | 1.151 |
| 1 | rs823121 | 203990925 | 1 | 0.4304 | 0.4063 | 2 | 2.683 | 0.1014 | 1.104 |
| 1 | rs12727528 | 204161225 | 1 | 0.0859 | 0.0706 | 2 | 3.623 | 0.05698 | 1.237 |
| 1 | rs2036100 | 204174495 | 2 | 0.3987 | 0.4039 | 1 | 0.1248 | 0.7238 | 0.9787 |
| 1 | rs3860295 | 204742954 | 1 | 0.4765 | 0.486 | 2 | 0.4049 | 0.5246 | 0.9627 |
| 1 | rs4845123 | 204887160 | 1 | 0.3513 | 0.3606 | 2 | 0.4248 | 0.5145 | 0.9602 |
| 1 | rs6691129 | 204943874 | 1 | 0.2085 | 0.1978 | 2 | 0.7987 | 0.3715 | 1.069 |
| 1 | rs11119623 | 205052929 | 1 | 0.2737 | 0.303 | 2 | 4.725 | 0.02973 | 0.8668 |

FIG. 4.2

| CHR | SNP | BP | A1 | F_A | F_U | A2 | CHISQ | P | OR |
|-----|-----|-----|-----|-----|-----|-----|-------|-----|-----|
| 1 | rs1150256 | 205139756 | 1 | 0.4749 | 0.4565 | 2 | 1.53 | 0.216 | 1.077 |
| 1 | rs291084 | 205162246 | 1 | 0.4801 | 0.4628 | 2 | 1.361 | 0.2434 | 1.072 |
| 1 | rs6697345 | 205196940 | 2 | 0.2893 | 0.2809 | 1 | 0.3851 | 0.5349 | 1.042 |
| 1 | rs2075863 | 205302577 | 1 | 0.4494 | 0.4429 | 2 | 0.1886 | 0.664 | 1.026 |
| 1 | rs7416946 | 205361804 | 2 | 0.3491 | 0.3521 | 1 | 0.04377 | 0.8343 | 0.987 |
| 1 | rs1588143 | 205586146 | 1 | 0.3033 | 0.2855 | 2 | 1.717 | 0.19 | 1.09 |
| 1 | rs311320 | 205685284 | 2 | 0.3146 | 0.3003 | 1 | 1.078 | 0.2993 | 1.069 |
| 1 | rs12567973 | 205748124 | 2 | 0.1775 | 0.1899 | 1 | 1.154 | 0.2827 | 0.9207 |
| 1 | rs650877 | 205815416 | 2 | 0.1811 | 0.1908 | 1 | 0.7014 | 0.4023 | 0.9379 |
| 1 | rs4844390 | 206001472 | 2 | 0.1942 | 0.197 | 1 | 0.05434 | 0.8157 | 0.9826 |
| 1 | rs6671947 | 206003114 | 1 | 0.09806 | 0.1117 | 2 | 2.244 | 0.1342 | 0.8645 |
| 1 | rs2796269 | 206003509 | 1 | 0.3586 | 0.3662 | 2 | 0.2811 | 0.596 | 0.9677 |
| 1 | rs2488255 | 206008364 | 2 | 0.3633 | 0.3725 | 1 | 0.4029 | 0.5256 | 0.9615 |
| 1 | rs2796278 | 206022446 | 1 | 0.4903 | 0.4952 | 2 | 0.1076 | 0.7429 | 0.9806 |
| 1 | rs7541230 | 206024178 | 2 | 0.2923 | 0.3087 | 1 | 1.444 | 0.2295 | 0.9248 |
| 1 | rs14374 | 206034880 | 2 | 0.04291 | 0.04493 | 1 | 0.1088 | 0.7415 | 0.9532 |
| 1 | rs2724377 | 206041441 | 2 | 0.4429 | 0.44 | 1 | 0.03655 | 0.8484 | 1.012 |
| 1 | rs3811386 | 206271022 | 2 | 0.4291 | 0.4275 | 1 | 0.01113 | 0.916 | 1.006 |
| 1 | rs10863695 | 206330280 | 1 | 0.2717 | 0.2742 | 2 | 0.03597 | 0.8496 | 0.9874 |
| 1 | rs11118986 | 206345851 | 1 | 0.3267 | 0.3145 | 2 | 0.7724 | 0.3795 | 1.058 |
| 1 | rs12024077 | 206393837 | 2 | 0.1522 | 0.1489 | 1 | 0.09521 | 0.7577 | 1.026 |
| 1 | rs570700 | 206395942 | 2 | 0.3696 | 0.3633 | 1 | 0.1956 | 0.6583 | 1.028 |
| 1 | rs874785 | 206413778 | 1 | 0.4032 | 0.4134 | 2 | 0.4853 | 0.486 | 0.9586 |
| 1 | rs17389198 | 207988681 | 1 | 0.2091 | 0.2039 | 2 | 0.1865 | 0.6659 | 1.032 |
| 1 | rs10863786 | 208004387 | 2 | 0.2245 | 0.2309 | 1 | 0.2661 | 0.6059 | 0.964 |
| 1 | rs7552506 | 208036525 | 2 | 0.3229 | 0.3488 | 1 | 3.38 | 0.06597 | 0.8905 |
| 1 | rs12404886 | 208068166 | 1 | 0.185 | 0.1889 | 2 | 0.111 | 0.739 | 0.9749 |
| 1 | rs585627 | 208070822 | 2 | 0.2514 | 0.2575 | 1 | 0.2189 | 0.6398 | 0.9685 |
| 1 | rs17015361 | 208085524 | 2 | 0.1324 | 0.1541 | 1 | 4.352 | 0.03696 | 0.8376 |
| 1 | rs227184 | 208235707 | 2 | 0.4314 | 0.4284 | 1 | 0.04071 | 0.8401 | 1.012 |
| 1 | rs846547 | 208327083 | 1 | 0.134 | 0.1329 | 2 | 0.01305 | 0.909 | 1.01 |
| 1 | rs3765873 | 208613065 | 2 | 0.2607 | 0.257 | 1 | 0.08137 | 0.7755 | 1.02 |
| 1 | rs6677509 | 208644151 | 1 | 0.1656 | 0.1623 | 2 | 0.08769 | 0.7671 | 1.024 |
| 1 | rs3765856 | 208649899 | 2 | 0.1266 | 0.1342 | 1 | 0.5706 | 0.45 | 0.9353 |
| 1 | rs10863839 | 208756419 | 1 | 0.2328 | 0.2505 | 2 | 1.926 | 0.1651 | 0.9079 |
| 1 | rs17260515 | 208777627 | 1 | 0.2595 | 0.2648 | 2 | 0.1582 | 0.6908 | 0.9734 |
| 1 | rs7413508 | 208906328 | 1 | 0.2927 | 0.3 | 2 | 0.287 | 0.5922 | 0.9657 |
| 1 | rs1890844 | 208973862 | 2 | 0.4796 | 0.5019 | 1 | 2.234 | 0.135 | 0.9146 |
| 1 | rs10494929 | 208994241 | 1 | 0.2513 | 0.257 | 2 | 0.1879 | 0.6647 | 0.9703 |
| 1 | rs11119591 | 208998674 | 1 | 0.1692 | 0.1749 | 2 | 0.2525 | 0.6153 | 0.9611 |
| 1 | rs1777245 | 209008553 | 1 | 0.1951 | 0.1763 | 2 | 2.627 | 0.1051 | 1.133 |
| 1 | rs1770220 | 209043739 | 2 | 0.3628 | 0.3797 | 1 | 1.388 | 0.2387 | 0.9299 |
| 1 | rs10494930 | 209161471 | 1 | 0.09165 | 0.09429 | 2 | 0.0936 | 0.7596 | 0.9691 |
| 1 | rs1846946 | 209167216 | 1 | 0.3073 | 0.3029 | 2 | 0.1023 | 0.7491 | 1.021 |
| 1 | rs6675213 | 209187668 | 1 | 0.09676 | 0.09807 | 2 | 0.02187 | 0.8824 | 0.9853 |
| 1 | rs10494931 | 209254353 | 1 | 0.3802 | 0.3928 | 2 | 0.7535 | 0.3854 | 0.9483 |
| 1 | rs924567 | 209254464 | 1 | 0.3801 | 0.3749 | 2 | 0.1288 | 0.7197 | 1.022 |
| 1 | rs4951520 | 209532246 | 2 | 0.3806 | 0.406 | 1 | 2.991 | 0.08372 | 0.8991 |
| 1 | rs17017432 | 209611717 | 2 | 0.149 | 0.1662 | 1 | 2.518 | 0.1125 | 0.8784 |
| 1 | rs12140949 | 209742861 | 2 | 0.1314 | 0.1123 | 1 | 3.807 | 0.05103 | 1.196 |
| 2 | rs17366657 | 11261965 | 2 | 0.5032 | 0.4845 | 1 | 1.575 | 0.2095 | 1.078 |
| 2 | rs13423864 | 11264700 | 1 | 0.4344 | 0.4551 | 2 | 1.947 | 0.1629 | 0.9197 |
| 2 | rs11884214 | 11287537 | 1 | 0.0582 | 0.05572 | 2 | 0.127 | 0.7215 | 1.047 |
| 2 | rs4669700 | 11318989 | 2 | 0.2123 | 0.2318 | 1 | 2.49 | 0.1146 | 0.8931 |

FIG. 4.3

| CHR | SNP | BP | A1 | F_A | F_U | A2 | CHISQ | P | OR |
|-----|-----|-----|-----|-----|-----|-----|-----|-----|-----|
| 2 | rs10179251 | 15228057 | 1 | 0.2431 | 0.2701 | 2 | 4.303 | 0.03804 | 0.868 |
| 2 | rs6721153 | 15234108 | 2 | 0.1621 | 0.1604 | 1 | 0.02371 | 0.8776 | 1.013 |
| 2 | rs7599455 | 15386242 | 1 | 0.4396 | 0.4415 | 2 | 0.01682 | 0.8968 | 0.9922 |
| 2 | rs1019359 | 15442066 | 1 | 0.322 | 0.313 | 2 | 0.4192 | 0.5173 | 1.042 |
| 2 | rs7590340 | 15518892 | 2 | 0.3225 | 0.3127 | 1 | 0.5027 | 0.4783 | 1.046 |
| 2 | rs3770462 | 15666775 | 2 | 0.1611 | 0.1633 | 1 | 0.03838 | 0.8447 | 0.9843 |
| 2 | rs3770471 | 15688226 | 1 | 0.1567 | 0.1442 | 2 | 1.358 | 0.2439 | 1.102 |
| 2 | rs10856770 | 17649434 | 1 | 0.4226 | 0.4319 | 2 | 0.3958 | 0.5293 | 0.9628 |
| 2 | rs11678187 | 17671614 | 2 | 0.3656 | 0.3816 | 1 | 1.241 | 0.2652 | 0.9337 |
| 2 | rs2710684 | 17677511 | 1 | 0.2036 | 0.2121 | 2 | 0.4871 | 0.4852 | 0.9501 |
| 2 | rs2555100 | 17698545 | 2 | 0.3381 | 0.3182 | 1 | 2.014 | 0.1558 | 1.094 |
| 2 | rs2710655 | 17717303 | 2 | 0.3254 | 0.3024 | 1 | 2.748 | 0.09736 | 1.112 |
| 2 | rs1065381 | 17747976 | 1 | 0.3344 | 0.313 | 2 | 2.346 | 0.1256 | 1.103 |
| 2 | rs7556996 | 17818785 | 1 | 0.1051 | 0.09768 | 2 | 0.6802 | 0.4095 | 1.085 |
| 2 | rs6707471 | 20010325 | 2 | 0.09643 | 0.09372 | 1 | 0.09631 | 0.7563 | 1.032 |
| 2 | rs2028208 | 20014298 | 2 | 0.2559 | 0.2541 | 1 | 0.01826 | 0.8925 | 1.009 |
| 2 | rs13005512 | 20032613 | 2 | 0.2208 | 0.2253 | 1 | 0.1322 | 0.7161 | 0.9743 |
| 2 | rs3771232 | 20280969 | 1 | 0.4003 | 0.3985 | 2 | 0.01643 | 0.898 | 1.008 |
| 2 | rs10192042 | 20366917 | 1 | 0.3815 | 0.3865 | 2 | 0.118 | 0.7312 | 0.9792 |
| 2 | rs3732151 | 20681939 | 1 | 0.2671 | 0.2845 | 2 | 1.694 | 0.193 | 0.9167 |
| 2 | rs10495708 | 20700148 | 1 | 0.2964 | 0.2589 | 2 | 7.834 | 0.005128 | 1.205 |
| 2 | rs660069 | 20816940 | 2 | 0.3424 | 0.3481 | 1 | 0.1594 | 0.6897 | 0.9751 |
| 2 | rs584996 | 20819643 | 2 | 0.4285 | 0.4342 | 1 | 0.1467 | 0.7017 | 0.9772 |
| 2 | rs12623521 | 24035174 | 1 | 0.198 | 0.1676 | 2 | 6.902 | 0.008611 | 1.226 |
| 2 | rs10200844 | 24146867 | 2 | 0.3547 | 0.3396 | 1 | 1.123 | 0.2892 | 1.069 |
| 2 | rs6733163 | 24244344 | 1 | 0.05794 | 0.05749 | 2 | 0.00426 | 0.948 | 1.008 |
| 2 | rs9309246 | 24261022 | 1 | 0.07247 | 0.0715 | 2 | 0.01592 | 0.8996 | 1.015 |
| 2 | rs1203 | 24296834 | 1 | 0.3664 | 0.3404 | 2 | 3.318 | 0.06854 | 1.12 |
| 2 | rs959200 | 24661643 | 2 | 0.09231 | 0.1043 | 1 | 1.85 | 0.1737 | 0.8729 |
| 2 | rs10865305 | 24725251 | 1 | 0.08988 | 0.1043 | 2 | 2.705 | 0.1 | 0.8476 |
| 2 | rs1439963 | 24802516 | 2 | 0.09198 | 0.1053 | 1 | 2.264 | 0.1324 | 0.8605 |
| 2 | rs893771 | 24808282 | 1 | 0.1884 | 0.2174 | 2 | 5.873 | 0.01537 | 0.8357 |
| 2 | rs12612839 | 24839223 | 2 | 0.04858 | 0.044 | 1 | 0.5324 | 0.4656 | 1.109 |
| 2 | rs1541984 | 24935918 | 1 | 0.415 | 0.4285 | 2 | 0.8448 | 0.358 | 0.9461 |
| 2 | rs2033654 | 24956612 | 1 | 0.4344 | 0.4493 | 2 | 1.016 | 0.3135 | 0.9413 |
| 2 | rs13382979 | 24985240 | 1 | 0.3208 | 0.3317 | 2 | 0.615 | 0.4329 | 0.9514 |
| 2 | rs10203353 | 25037368 | 1 | 0.1065 | 0.129 | 2 | 5.533 | 0.01866 | 0.8047 |
| 2 | rs6737159 | 25634957 | 2 | 0.2056 | 0.2016 | 1 | 0.1083 | 0.7421 | 1.025 |
| 2 | rs858647 | 25721179 | 1 | 0.4521 | 0.4415 | 2 | 0.5079 | 0.4761 | 1.044 |
| 2 | rs17391976 | 25748060 | 2 | 0.3757 | 0.3958 | 1 | 1.924 | 0.1655 | 0.9186 |
| 2 | rs10180842 | 25824008 | 2 | 0.3012 | 0.2918 | 1 | 0.4794 | 0.4887 | 1.046 |
| 2 | rs6546467 | 25842120 | 2 | 0.4866 | 0.5068 | 1 | 1.826 | 0.1766 | 0.9225 |
| 2 | rs10210497 | 25882989 | 2 | 0.3008 | 0.2921 | 1 | 0.412 | 0.521 | 1.043 |
| 2 | rs11126375 | 26198291 | 2 | 0.2174 | 0.2197 | 1 | 0.03606 | 0.8494 | 0.9864 |
| 2 | rs4665318 | 26278021 | 1 | 0.2287 | 0.2213 | 2 | 0.3618 | 0.5475 | 1.044 |
| 2 | rs4665836 | 26282178 | 1 | 0.24 | 0.2298 | 2 | 0.6516 | 0.4196 | 1.059 |
| 2 | rs11126491 | 26532000 | 1 | 0.3251 | 0.3332 | 2 | 0.3335 | 0.5636 | 0.964 |
| 2 | rs11675086 | 26584025 | 2 | 0.2818 | 0.2843 | 1 | 0.03611 | 0.8493 | 0.9875 |
| 2 | rs10173374 | 26606465 | 1 | 0.4744 | 0.4676 | 2 | 0.2072 | 0.6489 | 1.028 |
| 2 | rs939811 | 26610268 | 2 | 0.4935 | 0.4879 | 1 | 0.1423 | 0.706 | 1.023 |
| 2 | rs11692689 | 26867122 | 2 | 0.3526 | 0.3372 | 1 | 1.186 | 0.2761 | 1.071 |
| 2 | rs486582 | 26957635 | 2 | 0.4003 | 0.4134 | 1 | 0.8028 | 0.3703 | 0.9471 |
| 2 | rs12618836 | 26991120 | 1 | 0.3732 | 0.3796 | 2 | 0.1975 | 0.6568 | 0.973 |
| 2 | rs11897106 | 27049803 | 2 | 0.3878 | 0.3947 | 1 | 0.2266 | 0.6341 | 0.9714 |

FIG. 4.4

| CHR | SNP | BP | A1 | F_A | F_U | A2 | CHISQ | P | OR |
|---|---|---|---|---|---|---|---|---|---|
| 2 | rs10181551 | 27130778 | 1 | 0.292 | 0.2988 | 2 | 0.2551 | 0.6135 | 0.9675 |
| 2 | rs2078616 | 27295408 | 2 | 0.2182 | 0.2164 | 1 | 0.02129 | 0.884 | 1.011 |
| 2 | rs7563162 | 27484695 | 1 | 0.3726 | 0.37 | 2 | 0.03052 | 0.8613 | 1.011 |
| 2 | rs12478841 | 27665226 | 2 | 0.2703 | 0.2805 | 1 | 0.594 | 0.4409 | 0.9499 |
| 2 | rs6547740 | 27707873 | 1 | 0.4947 | 0.501 | 2 | 0.1748 | 0.6759 | 0.9754 |
| 2 | rs2272406 | 27745527 | 1 | 0.22 | 0.2362 | 2 | 1.685 | 0.1943 | 0.912 |
| 2 | rs4666022 | 27911327 | 2 | 0.208 | 0.1857 | 1 | 3.514 | 0.06084 | 1.151 |
| 2 | rs7581912 | 28210774 | 2 | 0.2691 | 0.2764 | 1 | 0.3025 | 0.5823 | 0.9638 |
| 2 | rs12464240 | 28215335 | 2 | 0.2433 | 0.2456 | 1 | 0.03304 | 0.8558 | 0.9875 |
| 2 | rs12478271 | 28288768 | 1 | 0.2494 | 0.2483 | 2 | 0.007054 | 0.9331 | 1.006 |
| 2 | rs2130291 | 28293449 | 1 | 0.1984 | 0.1903 | 2 | 0.4646 | 0.4955 | 1.053 |
| 2 | rs11127136 | 28323467 | 2 | 0.08907 | 0.08986 | 1 | 0.00855 | 0.9263 | 0.9904 |
| 2 | rs12468596 | 28357445 | 1 | 0.2005 | 0.1835 | 2 | 2.082 | 0.1491 | 1.116 |
| 2 | rs4666068 | 28477486 | 2 | 0.4959 | 0.4908 | 1 | 0.1191 | 0.73 | 1.021 |
| 2 | rs12185717 | 28923055 | 2 | 0.4972 | 0.4821 | 1 | 1.023 | 0.3119 | 1.062 |
| 2 | rs3112918 | 29230548 | 2 | 0.4429 | 0.4522 | 1 | 0.3943 | 0.53 | 0.9631 |
| 2 | rs13417374 | 29242311 | 2 | 0.3405 | 0.3391 | 1 | 0.009222 | 0.9235 | 1.006 |
| 2 | rs3820711 | 29284848 | 2 | 0.2962 | 0.2865 | 1 | 0.5145 | 0.4732 | 1.048 |
| 2 | rs6731724 | 29400262 | 2 | 0.2506 | 0.2524 | 1 | 0.01957 | 0.8888 | 0.9904 |
| 2 | rs1881427 | 29427824 | 2 | 0.1079 | 0.09632 | 1 | 1.627 | 0.2021 | 1.134 |
| 2 | rs12714275 | 29439933 | 1 | 0.3429 | 0.3498 | 2 | 0.2312 | 0.6306 | 0.9703 |
| 2 | rs10195189 | 29466430 | 2 | 0.2411 | 0.2478 | 1 | 0.2771 | 0.5986 | 0.9642 |
| 2 | rs11686232 | 29524890 | 1 | 0.06483 | 0.06715 | 2 | 0.09845 | 0.7537 | 0.9631 |
| 2 | rs11677261 | 29528833 | 1 | 0.1049 | 0.09565 | 2 | 1.054 | 0.3045 | 1.108 |
| 2 | rs4666222 | 29551416 | 1 | 0.3925 | 0.3951 | 2 | 0.03267 | 0.8566 | 0.989 |
| 2 | rs4438441 | 29584669 | 1 | 0.2194 | 0.2266 | 2 | 0.3358 | 0.5623 | 0.9594 |
| 2 | rs11689790 | 29608225 | 2 | 0.1821 | 0.1792 | 1 | 0.06169 | 0.8039 | 1.019 |
| 2 | rs10176482 | 29620651 | 2 | 0.2555 | 0.2704 | 1 | 1.266 | 0.2604 | 0.9262 |
| 2 | rs4635487 | 29687319 | 2 | 0.2494 | 0.2681 | 1 | 2.063 | 0.1509 | 0.907 |
| 2 | rs10193883 | 29706329 | 2 | 0.2829 | 0.3048 | 1 | 2.619 | 0.1056 | 0.8996 |
| 2 | rs6547966 | 29723870 | 2 | 0.2512 | 0.2787 | 1 | 4.393 | 0.03608 | 0.8681 |
| 2 | rs2254066 | 29742523 | 2 | 0.1664 | 0.1633 | 1 | 0.07916 | 0.7784 | 1.023 |
| 2 | rs763836 | 29758808 | 1 | 0.1595 | 0.1549 | 2 | 0.1815 | 0.6701 | 1.036 |
| 2 | rs2631941 | 29834790 | 2 | 0.1089 | 0.1087 | 1 | 0.0005182 | 0.9818 | 1.002 |
| 2 | rs7594627 | 29927776 | 1 | 0.2946 | 0.3014 | 2 | 0.2492 | 0.6176 | 0.968 |
| 2 | rs7597423 | 29935902 | 2 | 0.1912 | 0.1816 | 1 | 0.6836 | 0.4084 | 1.065 |
| 2 | rs11127243 | 29953637 | 1 | 0.2162 | 0.2097 | 2 | 0.2865 | 0.5925 | 1.04 |
| 2 | rs4233750 | 29956154 | 1 | 0.2168 | 0.213 | 2 | 0.09284 | 0.7606 | 1.022 |
| 2 | rs3813658 | 30327026 | 1 | 0.4785 | 0.4734 | 2 | 0.1174 | 0.7318 | 1.021 |
| 2 | rs829624 | 30609700 | 2 | 0.1011 | 0.102 | 1 | 0.01174 | 0.9137 | 0.9894 |
| 2 | rs829628 | 30610392 | 2 | 0.3208 | 0.3367 | 1 | 1.299 | 0.2544 | 0.9302 |
| 2 | rs7562645 | 30620056 | 2 | 0.2269 | 0.228 | 1 | 0.007965 | 0.9289 | 0.9937 |
| 2 | rs10201020 | 30640747 | 2 | 0.1268 | 0.1275 | 1 | 0.005155 | 0.9428 | 0.9936 |
| 2 | rs10208152 | 30651934 | 1 | 0.3148 | 0.2957 | 2 | 1.949 | 0.1627 | 1.095 |
| 2 | rs7597579 | 30689444 | 1 | 0.1255 | 0.1266 | 2 | 0.01158 | 0.9143 | 0.9904 |
| 2 | rs2593446 | 30704087 | 2 | 0.4177 | 0.3986 | 1 | 1.704 | 0.1918 | 1.082 |
| 2 | rs10469986 | 30711403 | 1 | 0.1293 | 0.1295 | 2 | 0.0004584 | 0.9829 | 0.9981 |
| 2 | rs6735522 | 30801668 | 2 | 0.4226 | 0.4376 | 1 | 1.035 | 0.309 | 0.9406 |
| 2 | rs7603877 | 30856467 | 1 | 0.3834 | 0.4121 | 2 | 3.87 | 0.04915 | 0.8871 |
| 2 | rs6761033 | 31023247 | 1 | 0.3522 | 0.3636 | 2 | 0.638 | 0.4244 | 0.9516 |
| 2 | rs12990027 | 31079687 | 1 | 0.2538 | 0.2594 | 2 | 0.1835 | 0.6684 | 0.9712 |
| 2 | rs10198054 | 31100250 | 1 | 0.332 | 0.3531 | 2 | 2.241 | 0.1344 | 0.9103 |
| 2 | rs10865086 | 31106691 | 1 | 0.4105 | 0.4251 | 2 | 0.9866 | 0.3206 | 0.9418 |
| 2 | rs7565689 | 31148205 | 1 | 0.4457 | 0.4289 | 2 | 1.295 | 0.255 | 1.071 |

FIG. 4.5

| CHR | SNP | BP | A1 | F_A | F_U | A2 | CHISQ | P | OR |
|-----|-----|-----|-----|-----|-----|-----|-------|-----|-----|
| 2 | rs2098787 | 31150148 | 1 | 0.4473 | 0.4303 | 2 | 1.323 | 0.25 | 1.072 |
| 2 | rs1991104 | 31156217 | 1 | 0.4518 | 0.4396 | 2 | 0.6854 | 0.4077 | 1.051 |
| 2 | rs880618 | 31156777 | 2 | 0.4789 | 0.4942 | 1 | 1.053 | 0.3048 | 0.9406 |
| 2 | rs669292 | 31338836 | 2 | 0.1453 | 0.1348 | 1 | 1.04 | 0.3078 | 1.092 |
| 2 | rs2365842 | 31441792 | 1 | 0.2796 | 0.2541 | 2 | 3.724 | 0.05364 | 1.139 |
| 2 | rs2163059 | 31456638 | 2 | 0.346 | 0.358 | 1 | 0.7043 | 0.4013 | 0.949 |
| 2 | rs6760105 | 32160890 | 2 | 0.3799 | 0.3797 | 1 | 0.0001222 | 0.9912 | 1.001 |
| 2 | rs7588883 | 32250771 | 2 | 0.4692 | 0.5014 | 1 | 4.681 | 0.0305 | 0.8789 |
| 2 | rs462878 | 32292159 | 1 | 0.2604 | 0.249 | 2 | 0.7584 | 0.3838 | 1.061 |
| 2 | rs176411 | 32497032 | 1 | 0.4614 | 0.4478 | 2 | 0.8371 | 0.3602 | 1.056 |
| 2 | rs17820747 | 32555191 | 2 | 0.2322 | 0.2309 | 1 | 0.01053 | 0.9183 | 1.007 |
| 2 | rs2754514 | 32642340 | 1 | 0.4659 | 0.4792 | 2 | 0.7891 | 0.3744 | 0.9483 |
| 2 | rs4952285 | 32788092 | 1 | 0.2198 | 0.2122 | 2 | 0.3808 | 0.5372 | 1.046 |
| 2 | rs7593327 | 32826215 | 2 | 0.07502 | 0.07157 | 1 | 0.1971 | 0.657 | 1.052 |
| 2 | rs12989970 | 32852547 | 2 | 0.1052 | 0.1087 | 1 | 0.1441 | 0.7042 | 0.964 |
| 2 | rs219208 | 33105782 | 1 | 0.3429 | 0.356 | 2 | 0.8498 | 0.3566 | 0.944 |
| 2 | rs17325390 | 33173365 | 1 | 0.07652 | 0.08317 | 2 | 0.6806 | 0.4094 | 0.9134 |
| 2 | rs1563185 | 33209494 | 2 | 0.08063 | 0.06618 | 1 | 3.428 | 0.0641 | 1.237 |
| 2 | rs6543698 | 33256475 | 2 | 0.4972 | 0.4652 | 1 | 4.601 | 0.03195 | 1.137 |
| 2 | rs6720185 | 33261729 | 1 | 0.4955 | 0.4622 | 2 | 4.992 | 0.02546 | 1.143 |
| 2 | rs17012668 | 33288486 | 1 | 0.1494 | 0.1426 | 2 | 0.4097 | 0.5221 | 1.056 |
| 2 | rs11124313 | 33366087 | 1 | 0.4049 | 0.409 | 2 | 0.08009 | 0.7772 | 0.983 |
| 2 | rs10189109 | 33398520 | 1 | 0.2518 | 0.251 | 2 | 0.004369 | 0.9473 | 1.005 |
| 2 | rs3770945 | 36465984 | 2 | 0.349 | 0.3578 | 1 | 0.3856 | 0.5346 | 0.962 |
| 2 | rs11673762 | 36519436 | 2 | 0.1332 | 0.129 | 1 | 0.1753 | 0.6755 | 1.038 |
| 2 | rs848513 | 36528500 | 1 | 0.3244 | 0.3114 | 2 | 0.8758 | 0.3494 | 1.062 |
| 2 | rs848556 | 36549124 | 1 | 0.4534 | 0.4598 | 2 | 0.187 | 0.6655 | 0.9744 |
| 2 | rs2888369 | 36577928 | 1 | 0.34 | 0.338 | 2 | 0.01897 | 0.8905 | 1.009 |
| 2 | rs3770833 | 36592075 | 1 | 0.3368 | 0.3427 | 2 | 0.1716 | 0.6787 | 0.9743 |
| 2 | rs3821153 | 36606626 | 2 | 0.4182 | 0.426 | 1 | 0.2806 | 0.5963 | 0.9685 |
| 2 | rs10194100 | 36636631 | 2 | 0.3594 | 0.3541 | 1 | 0.1375 | 0.7108 | 1.023 |
| 2 | rs848611 | 36669531 | 2 | 0.3688 | 0.3646 | 1 | 0.08639 | 0.7688 | 1.018 |
| 2 | rs17019511 | 36784931 | 1 | 0.4384 | 0.4343 | 2 | 0.07739 | 0.7809 | 1.017 |
| 2 | rs6746047 | 36796234 | 2 | 0.4085 | 0.4072 | 1 | 0.008435 | 0.9268 | 1.006 |
| 2 | rs10490658 | 36989902 | 2 | 0.08462 | 0.07391 | 1 | 1.758 | 0.1849 | 1.158 |
| 2 | rs9318 | 37331418 | 2 | 0.1297 | 0.1251 | 1 | 0.2084 | 0.6481 | 1.042 |
| 2 | rs13390055 | 37351956 | 2 | 0.09271 | 0.08317 | 1 | 1.272 | 0.2594 | 1.126 |
| 2 | rs9309004 | 37371951 | 2 | 0.3773 | 0.3981 | 1 | 2.042 | 0.153 | 0.9163 |
| 2 | rs6730503 | 37381982 | 2 | 0.3761 | 0.3652 | 1 | 0.5729 | 0.4491 | 1.048 |
| 2 | rs1374188 | 38062903 | 2 | 0.2111 | 0.2145 | 1 | 0.0773 | 0.781 | 0.98 |
| 2 | rs2373327 | 38075590 | 2 | 0.3078 | 0.3106 | 1 | 0.03994 | 0.8416 | 0.9871 |
| 2 | rs17412105 | 38096743 | 1 | 0.1726 | 0.1797 | 2 | 0.3917 | 0.5314 | 0.9522 |
| 2 | rs336046 | 38109425 | 2 | 0.4478 | 0.4522 | 1 | 0.08813 | 0.7666 | 0.9824 |
| 2 | rs336032 | 38118292 | 2 | 0.4278 | 0.4135 | 1 | 0.9428 | 0.3316 | 1.06 |
| 2 | rs17492358 | 38438619 | 1 | 0.04217 | 0.04642 | 2 | 0.4814 | 0.4878 | 0.9045 |
| 2 | rs6544169 | 38668021 | 1 | 0.1663 | 0.1678 | 2 | 0.01903 | 0.8903 | 0.989 |
| 2 | rs10206629 | 38773837 | 2 | 0.2002 | 0.1765 | 1 | 4.104 | 0.04278 | 1.168 |
| 2 | rs6725356 | 39091899 | 1 | 0.06726 | 0.07874 | 2 | 2.208 | 0.1373 | 0.8437 |
| 2 | rs1454219 | 39130461 | 2 | 0.09514 | 0.1043 | 1 | 1.067 | 0.3017 | 0.9025 |
| 2 | rs297152 | 39180238 | 2 | 0.08178 | 0.09003 | 1 | 0.9783 | 0.3226 | 0.9002 |
| 2 | rs2302749 | 39347701 | 2 | 0.1563 | 0.148 | 1 | 0.6009 | 0.4382 | 1.067 |
| 2 | rs2024514 | 39407880 | 1 | 0.1459 | 0.143 | 2 | 0.07509 | 0.7841 | 1.024 |
| 2 | rs1035232 | 39427025 | 2 | 0.08313 | 0.09197 | 1 | 1.103 | 0.2936 | 0.8952 |
| 2 | rs2716699 | 39779236 | 2 | 0.4473 | 0.453 | 1 | 0.1486 | 0.6999 | 0.9772 |

FIG. 4.6

| CHR | SNP | BP | A1 | F_A | F_U | A2 | CHISQ | P | OR |
|---|---|---|---|---|---|---|---|---|---|
| 2 | rs12470473 | 39858794 | 1 | 0.4765 | 0.4913 | 2 | 0.9903 | 0.3197 | 0.9424 |
| 2 | rs17559580 | 40205294 | 1 | 0.4712 | 0.4478 | 2 | 2.492 | 0.1144 | 1.099 |
| 2 | rs12478057 | 40205790 | 2 | 0.4745 | 0.4522 | 1 | 2.251 | 0.1336 | 1.094 |
| 2 | rs2160365 | 40208075 | 1 | 0.2577 | 0.2747 | 2 | 1.661 | 0.1975 | 0.9168 |
| 2 | rs2110923 | 40211501 | 1 | 0.1528 | 0.1612 | 2 | 0.6045 | 0.4369 | 0.9383 |
| 2 | rs10175725 | 40239688 | 1 | 0.4765 | 0.4956 | 2 | 1.647 | 0.1993 | 0.9263 |
| 2 | rs447136 | 40309860 | 2 | 0.1919 | 0.1867 | 1 | 0.2019 | 0.6532 | 1.035 |
| 2 | rs7570300 | 40329906 | 2 | 0.09433 | 0.09082 | 1 | 0.165 | 0.6846 | 1.043 |
| 2 | rs2287051 | 40400928 | 2 | 0.2508 | 0.2515 | 1 | 0.002701 | 0.9585 | 0.9964 |
| 2 | rs11888123 | 40408981 | 1 | 0.3381 | 0.3379 | 2 | 0.0002371 | 0.9877 | 1.001 |
| 2 | rs11886400 | 40428628 | 2 | 0.09312 | 0.09807 | 1 | 0.3199 | 0.5717 | 0.9443 |
| 2 | rs6738673 | 40434980 | 2 | 0.4708 | 0.4724 | 1 | 0.01205 | 0.9126 | 0.9935 |
| 2 | rs6544515 | 42283904 | 2 | 0.4235 | 0.4184 | 1 | 0.1213 | 0.7276 | 1.021 |
| 2 | rs2118888 | 42305058 | 1 | 0.2445 | 0.2384 | 2 | 0.2307 | 0.631 | 1.034 |
| 2 | rs6758198 | 42321138 | 1 | 0.4428 | 0.4652 | 2 | 2.278 | 0.1312 | 0.9136 |
| 2 | rs6713242 | 42324298 | 1 | 0.1174 | 0.1222 | 2 | 0.2478 | 0.6186 | 0.9554 |
| 2 | rs17029490 | 42350253 | 2 | 0.1423 | 0.1275 | 1 | 2.103 | 0.147 | 1.135 |
| 2 | rs11682029 | 42407082 | 1 | 0.4385 | 0.4289 | 2 | 0.4174 | 0.5182 | 1.04 |
| 2 | rs2286702 | 42408977 | 1 | 0.1272 | 0.1388 | 2 | 1.307 | 0.253 | 0.9046 |
| 2 | rs1496328 | 42531091 | 1 | 0.3429 | 0.3469 | 2 | 0.07759 | 0.7806 | 0.9827 |
| 2 | rs12712854 | 42714897 | 1 | 0.2759 | 0.2966 | 2 | 2.362 | 0.1243 | 0.9037 |
| 2 | rs6749927 | 43338060 | 2 | 0.3784 | 0.3722 | 1 | 0.1859 | 0.6663 | 1.027 |
| 2 | rs6544638 | 43355306 | 2 | 0.265 | 0.2843 | 1 | 2.117 | 0.1457 | 0.9075 |
| 2 | rs6544643 | 43413576 | 1 | 0.1927 | 0.2033 | 2 | 0.7943 | 0.3728 | 0.9355 |
| 2 | rs7570751 | 43489010 | 2 | 0.2813 | 0.2756 | 1 | 0.1767 | 0.6742 | 1.028 |
| 2 | rs6728106 | 43537389 | 2 | 0.1158 | 0.1203 | 1 | 0.2194 | 0.6395 | 0.9577 |
| 2 | rs3884504 | 43549057 | 1 | 0.2585 | 0.2585 | 2 | 1.76E-05 | 0.9966 | 1 |
| 2 | rs7570242 | 43559506 | 2 | 0.1231 | 0.1237 | 1 | 0.003682 | 0.9516 | 0.9945 |
| 2 | rs9973398 | 43633965 | 2 | 0.3437 | 0.3411 | 1 | 0.03543 | 0.8507 | 1.012 |
| 2 | rs17031092 | 43662752 | 2 | 0.1296 | 0.1388 | 1 | 0.827 | 0.3631 | 0.9236 |
| 2 | rs7594100 | 43734665 | 1 | 0.4331 | 0.4275 | 2 | 0.1444 | 0.7039 | 1.023 |
| 2 | rs2374569 | 43738561 | 1 | 0.347 | 0.3604 | 2 | 0.8883 | 0.3459 | 0.943 |
| 2 | rs6714122 | 43753481 | 1 | 0.2854 | 0.3063 | 2 | 2.355 | 0.1248 | 0.9047 |
| 2 | rs4952996 | 43770459 | 1 | 0.1442 | 0.1397 | 2 | 0.1866 | 0.6657 | 1.038 |
| 2 | rs10199649 | 43808296 | 1 | 0.2032 | 0.1932 | 2 | 0.7077 | 0.4002 | 1.065 |
| 2 | rs17414362 | 43822986 | 1 | 0.2014 | 0.1923 | 2 | 0.5897 | 0.4425 | 1.059 |
| 2 | rs3815994 | 43870389 | 2 | 0.07409 | 0.0628 | 1 | 2.235 | 0.1349 | 1.194 |
| 2 | rs2278356 | 43893379 | 2 | 0.3327 | 0.3312 | 1 | 0.01023 | 0.9194 | 1.006 |
| 2 | rs17031775 | 44034009 | 1 | 0.1328 | 0.1365 | 2 | 0.1325 | 0.7159 | 0.9687 |
| 2 | rs7558340 | 44049477 | 2 | 0.104 | 0.1087 | 1 | 0.2563 | 0.6127 | 0.9523 |
| 2 | rs6741066 | 44062981 | 2 | 0.2727 | 0.27 | 1 | 0.03974 | 0.842 | 1.013 |
| 2 | rs10865197 | 44068193 | 2 | 0.2547 | 0.2548 | 1 | 0.0001915 | 0.989 | 0.9991 |
| 2 | rs4450660 | 44073680 | 1 | 0.2494 | 0.2517 | 2 | 0.03169 | 0.8587 | 0.9878 |
| 2 | rs7568986 | 44265259 | 2 | 0.1769 | 0.1601 | 1 | 2.277 | 0.1313 | 1.128 |
| 2 | rs17032095 | 44383477 | 1 | 0.06645 | 0.0668 | 2 | 0.002155 | 0.963 | 0.9945 |
| 2 | rs4953088 | 44418022 | 1 | 0.2474 | 0.25 | 2 | 0.04194 | 0.8377 | 0.986 |
| 2 | rs698768 | 44430053 | 2 | 0.3124 | 0.3179 | 1 | 0.1565 | 0.6924 | 0.9749 |
| 2 | rs1067376 | 44501865 | 1 | 0.2524 | 0.2539 | 2 | 0.0123 | 0.9117 | 0.9924 |
| 2 | rs1302175 | 44510528 | 2 | 0.3267 | 0.3357 | 1 | 0.4146 | 0.5196 | 0.9601 |
| 2 | rs1085448 | 44517309 | 1 | 0.2358 | 0.2411 | 2 | 0.1706 | 0.6796 | 0.9715 |
| 2 | rs13016493 | 44594056 | 2 | 0.4562 | 0.4464 | 1 | 0.4388 | 0.5077 | 1.04 |
| 2 | rs10199825 | 44645780 | 1 | 0.334 | 0.329 | 2 | 0.1282 | 0.7204 | 1.023 |
| 2 | rs6751281 | 44678108 | 2 | 0.3351 | 0.332 | 1 | 0.04694 | 0.8285 | 1.014 |
| 2 | rs6544764 | 44719143 | 1 | 0.3316 | 0.3256 | 2 | 0.1822 | 0.6695 | 1.027 |

FIG. 4.7

| CHR | SNP | BP | A1 | F_A | F_U | A2 | CHISQ | P | OR |
|---|---|---|---|---|---|---|---|---|---|
| 2 | rs11679997 | 44846991 | 1 | 0.1709 | 0.158 | 2 | 1.356 | 0.2442 | 1.098 |
| 2 | rs3770244 | 45486604 | 1 | 0.314 | 0.3148 | 2 | 0.003155 | 0.9552 | 0.9964 |
| 2 | rs11125017 | 45534669 | 2 | 0.3517 | 0.3478 | 1 | 0.07434 | 0.7851 | 1.017 |
| 2 | rs7577547 | 45599910 | 1 | 0.2093 | 0.2029 | 2 | 0.2828 | 0.5949 | 1.04 |
| 2 | rs3821059 | 45609059 | 1 | 0.2557 | 0.2531 | 2 | 0.03805 | 0.8453 | 1.013 |
| 2 | rs1865685 | 45628052 | 1 | 0.218 | 0.213 | 2 | 0.1628 | 0.6866 | 1.03 |
| 2 | rs6725257 | 45800304 | 1 | 0.2107 | 0.1855 | 2 | 4.478 | 0.03434 | 1.172 |
| 2 | rs12622534 | 45853492 | 1 | 0.4393 | 0.4217 | 2 | 1.411 | 0.2348 | 1.074 |
| 2 | rs4953264 | 45906128 | 1 | 0.4465 | 0.4345 | 2 | 0.6546 | 0.4185 | 1.05 |
| 2 | rs13400225 | 45957381 | 1 | 0.4092 | 0.4111 | 2 | 0.0163 | 0.8984 | 0.9923 |
| 2 | rs13431763 | 45964078 | 1 | 0.2461 | 0.2125 | 2 | 7.174 | 0.007396 | 1.21 |
| 2 | rs12986554 | 46006618 | 2 | 0.5024 | 0.4894 | 1 | 0.7689 | 0.3806 | 1.054 |
| 2 | rs13423410 | 46087304 | 2 | 0.07368 | 0.07971 | 1 | 0.5793 | 0.4466 | 0.9184 |
| 2 | rs10865211 | 46133246 | 2 | 0.3934 | 0.3918 | 1 | 0.01283 | 0.9098 | 1.007 |
| 2 | rs13024886 | 46176455 | 1 | 0.4397 | 0.413 | 2 | 3.283 | 0.07001 | 1.115 |
| 2 | rs7568264 | 46182056 | 1 | 0.2342 | 0.2311 | 2 | 0.05886 | 0.8083 | 1.017 |
| 2 | rs11695648 | 46187863 | 1 | 0.4728 | 0.4894 | 2 | 1.231 | 0.2671 | 0.9359 |
| 2 | rs3768747 | 46205562 | 2 | 0.3589 | 0.3432 | 1 | 1.216 | 0.2701 | 1.071 |
| 2 | rs11125070 | 46406430 | 1 | 0.2522 | 0.2553 | 2 | 0.05669 | 0.8118 | 0.9838 |
| 2 | rs4953347 | 46411729 | 1 | 0.4942 | 0.4863 | 2 | 0.2743 | 0.6004 | 1.032 |
| 2 | rs10176396 | 46438077 | 1 | 0.3308 | 0.3225 | 2 | 0.3469 | 0.5559 | 1.038 |
| 2 | rs7594278 | 46458097 | 1 | 0.4874 | 0.4956 | 2 | 0.3053 | 0.5806 | 0.9676 |
| 2 | rs7589801 | 46669746 | 1 | 0.4765 | 0.4712 | 2 | 0.1221 | 0.7268 | 1.021 |
| 2 | rs17770970 | 46790341 | 1 | 0.1117 | 0.1145 | 2 | 0.08509 | 0.7705 | 0.9729 |
| 2 | rs4952843 | 46811349 | 2 | 0.3983 | 0.3917 | 1 | 0.206 | 0.6499 | 1.028 |
| 2 | rs4953418 | 46838131 | 2 | 0.4182 | 0.4279 | 1 | 0.4368 | 0.5087 | 0.9609 |
| 2 | rs10495944 | 47556794 | 1 | 0.1199 | 0.1295 | 2 | 0.9401 | 0.3323 | 0.9163 |
| 2 | rs11685861 | 48435380 | 2 | 0.468 | 0.4797 | 1 | 0.6155 | 0.4327 | 0.9542 |
| 2 | rs940389 | 48661656 | 2 | 0.3351 | 0.3237 | 1 | 0.6639 | 0.4152 | 1.053 |
| 2 | rs17037472 | 48702019 | 1 | 0.1069 | 0.1077 | 2 | 0.008434 | 0.9268 | 0.9912 |
| 2 | rs1404054 | 48731004 | 2 | 0.2251 | 0.1963 | 1 | 5.561 | 0.01837 | 1.189 |
| 2 | rs7557060 | 48793246 | 2 | 0.1988 | 0.1697 | 1 | 6.288 | 0.01215 | 1.214 |
| 2 | rs6732220 | 49076376 | 2 | 0.2518 | 0.2408 | 1 | 0.7339 | 0.3916 | 1.061 |
| 2 | rs2349414 | 49080350 | 2 | 0.2358 | 0.2242 | 1 | 0.8528 | 0.3557 | 1.068 |
| 2 | rs974894 | 49184262 | 1 | 0.4626 | 0.4541 | 2 | 0.3325 | 0.5642 | 1.035 |
| 2 | rs7606570 | 49189768 | 2 | 0.2265 | 0.2203 | 1 | 0.25 | 0.6171 | 1.036 |
| 2 | rs960038 | 49191837 | 2 | 0.3855 | 0.3801 | 1 | 0.1377 | 0.7105 | 1.023 |
| 2 | rs17038288 | 49196616 | 1 | 0.2235 | 0.2179 | 2 | 0.2057 | 0.6501 | 1.033 |
| 2 | rs1504176 | 49232378 | 2 | 0.2692 | 0.2662 | 1 | 0.05332 | 0.8174 | 1.016 |
| 2 | rs17039592 | 50049659 | 2 | 0.1565 | 0.1704 | 1 | 1.585 | 0.208 | 0.9033 |
| 2 | rs9636391 | 50054614 | 1 | 0.158 | 0.1522 | 2 | 0.2935 | 0.588 | 1.046 |
| 2 | rs2193867 | 50079598 | 2 | 0.4611 | 0.4666 | 1 | 0.1368 | 0.7115 | 0.9781 |
| 2 | rs6736816 | 50109093 | 1 | 0.2389 | 0.2193 | 2 | 2.429 | 0.1191 | 1.117 |
| 2 | rs1363045 | 50137825 | 1 | 0.3684 | 0.3826 | 2 | 0.9673 | 0.3253 | 0.9413 |
| 2 | rs12472712 | 50166066 | 1 | 0.25 | 0.2444 | 2 | 0.1866 | 0.6658 | 1.03 |
| 2 | rs6751528 | 50240747 | 1 | 0.3899 | 0.3676 | 2 | 2.366 | 0.124 | 1.099 |
| 2 | rs2688889 | 50242334 | 1 | 0.3907 | 0.3667 | 2 | 2.753 | 0.09707 | 1.108 |
| 2 | rs1452769 | 50249639 | 2 | 0.1536 | 0.1377 | 1 | 2.274 | 0.1316 | 1.136 |
| 2 | rs17040235 | 50295031 | 2 | 0.1271 | 0.1126 | 1 | 2.255 | 0.1332 | 1.148 |
| 2 | rs13429626 | 50311895 | 1 | 0.4209 | 0.4126 | 2 | 0.3238 | 0.5693 | 1.035 |
| 2 | rs1712914 | 50329927 | 2 | 0.4765 | 0.4681 | 1 | 0.3174 | 0.5732 | 1.034 |
| 2 | rs17040443 | 50380016 | 2 | 0.09555 | 0.1006 | 1 | 0.3231 | 0.5697 | 0.9447 |
| 2 | rs939426 | 50381257 | 1 | 0.444 | 0.4219 | 2 | 2.237 | 0.1348 | 1.094 |
| 2 | rs13430295 | 50465424 | 2 | 0.2439 | 0.2459 | 1 | 0.02367 | 0.8777 | 0.9894 |

FIG. 4.8

| CHR | SNP | BP | A1 | F_A | F_U | A2 | CHISQ | P | OR |
|---|---|---|---|---|---|---|---|---|---|
| 2 | rs13032775 | 50483236 | 1 | 0.2287 | 0.2326 | 2 | 0.09392 | 0.7593 | 0.9786 |
| 2 | rs13392422 | 50487789 | 2 | 0.232 | 0.2348 | 1 | 0.04933 | 0.8242 | 0.9845 |
| 2 | rs4493303 | 50499690 | 1 | 0.136 | 0.1406 | 2 | 0.1956 | 0.6583 | 0.9626 |
| 2 | rs10865246 | 50523758 | 1 | 0.3571 | 0.3676 | 2 | 0.5426 | 0.4614 | 0.9554 |
| 2 | rs877108 | 50571840 | 1 | 0.3377 | 0.3512 | 2 | 0.9173 | 0.3382 | 0.9417 |
| 2 | rs759505 | 50608547 | 1 | 0.1364 | 0.1319 | 2 | 0.2007 | 0.6541 | 1.04 |
| 2 | rs12713105 | 50625844 | 2 | 0.2252 | 0.2396 | 1 | 1.295 | 0.2552 | 0.9228 |
| 2 | rs17538414 | 50679125 | 2 | 0.2567 | 0.235 | 1 | 2.844 | 0.09174 | 1.124 |
| 2 | rs13389162 | 50707325 | 1 | 0.412 | 0.4202 | 2 | 0.3121 | 0.5764 | 0.9668 |
| 2 | rs9309200 | 50870615 | 1 | 0.2057 | 0.2246 | 2 | 2.407 | 0.1208 | 0.8937 |
| 2 | rs3914729 | 50892633 | 1 | 0.2068 | 0.1957 | 2 | 0.8705 | 0.3508 | 1.072 |
| 2 | rs9309203 | 50900862 | 1 | 0.3209 | 0.323 | 2 | 0.02295 | 0.8796 | 0.9904 |
| 2 | rs10490174 | 50937182 | 1 | 0.2153 | 0.2047 | 2 | 0.7494 | 0.3867 | 1.066 |
| 2 | rs6740920 | 50988466 | 1 | 0.1024 | 0.09333 | 2 | 1.052 | 0.305 | 1.109 |
| 2 | rs7557568 | 51028800 | 1 | 0.3031 | 0.3109 | 2 | 0.326 | 0.568 | 0.9638 |
| 2 | rs10195460 | 51082210 | 1 | 0.4247 | 0.4271 | 2 | 0.02559 | 0.8729 | 0.9904 |
| 2 | rs7594170 | 51091271 | 2 | 0.4818 | 0.4879 | 1 | 0.1708 | 0.6794 | 0.9757 |
| 2 | rs7609067 | 53790057 | 1 | 0.1287 | 0.114 | 2 | 2.282 | 0.1308 | 1.148 |
| 2 | rs13387291 | 53888755 | 2 | 0.05709 | 0.05368 | 1 | 0.2496 | 0.6174 | 1.067 |
| 2 | rs2692520 | 53888951 | 2 | 0.1717 | 0.1976 | 1 | 5.052 | 0.02459 | 0.8416 |
| 2 | rs805385 | 53950616 | 1 | 0.2583 | 0.2913 | 2 | 6.166 | 0.01302 | 0.8473 |
| 2 | rs9309251 | 53987142 | 1 | 0.05587 | 0.05169 | 2 | 0.3854 | 0.5347 | 1.086 |
| 2 | rs17045435 | 54027762 | 2 | 0.08462 | 0.0715 | 1 | 2.675 | 0.1019 | 1.2 |
| 2 | rs7572559 | 54225622 | 1 | 0.2094 | 0.2088 | 2 | 0.002036 | 0.964 | 1.003 |
| 2 | rs11125528 | 54289094 | 1 | 0.2612 | 0.2686 | 2 | 0.3217 | 0.5706 | 0.9624 |
| 2 | rs1215149 | 54289809 | 1 | 0.2794 | 0.2816 | 2 | 0.02805 | 0.867 | 0.9889 |
| 2 | rs17045711 | 54330182 | 1 | 0.1314 | 0.1334 | 2 | 0.03745 | 0.8466 | 0.9831 |
| 2 | rs843698 | 54340284 | 2 | 0.283 | 0.2855 | 1 | 0.03342 | 0.8549 | 0.988 |
| 2 | rs843720 | 54364164 | 2 | 0.4745 | 0.4647 | 1 | 0.4306 | 0.5117 | 1.04 |
| 2 | rs13184 | 54441163 | 1 | 0.1962 | 0.2065 | 2 | 0.7467 | 0.3875 | 0.9376 |
| 2 | rs6739099 | 54676814 | 1 | 0.3462 | 0.3256 | 2 | 2.129 | 0.1445 | 1.097 |
| 2 | rs7591204 | 54682962 | 2 | 0.2259 | 0.2384 | 1 | 0.9857 | 0.3208 | 0.9324 |
| 2 | rs10176359 | 54699202 | 1 | 0.1862 | 0.1918 | 2 | 0.2267 | 0.6339 | 0.9644 |
| 2 | rs4671964 | 54707033 | 2 | 0.3427 | 0.3224 | 1 | 2.084 | 0.1488 | 1.096 |
| 2 | rs2920892 | 55118707 | 2 | 0.1709 | 0.1686 | 1 | 0.04196 | 0.8377 | 1.016 |
| 2 | rs2968787 | 55119289 | 1 | 0.4789 | 0.4739 | 2 | 0.1136 | 0.7361 | 1.02 |
| 2 | rs14026 | 55258298 | 2 | 0.464 | 0.4899 | 1 | 3.026 | 0.08194 | 0.9014 |
| 2 | rs1992127 | 55414142 | 1 | 0.4012 | 0.4092 | 2 | 0.2965 | 0.5861 | 0.9675 |
| 2 | rs3099083 | 55439081 | 1 | 0.09522 | 0.101 | 2 | 0.4213 | 0.5163 | 0.9371 |
| 2 | rs9789365 | 55446278 | 2 | 0.2947 | 0.2993 | 1 | 0.1134 | 0.7363 | 0.9783 |
| 2 | rs1902057 | 55476329 | 2 | 0.09595 | 0.1019 | 1 | 0.4529 | 0.5009 | 0.9351 |
| 2 | rs12618795 | 55485352 | 1 | 0.09271 | 0.09913 | 2 | 0.5359 | 0.4641 | 0.9287 |
| 2 | rs4672229 | 58129784 | 1 | 0.4474 | 0.4725 | 2 | 2.856 | 0.09101 | 0.9039 |
| 2 | rs17049339 | 58180874 | 2 | 0.09724 | 0.1024 | 1 | 0.3356 | 0.5624 | 0.9441 |
| 2 | rs3755296 | 58266472 | 2 | 0.0915 | 0.09034 | 1 | 0.01832 | 0.8923 | 1.014 |
| 2 | rs3821211 | 58285213 | 1 | 0.05182 | 0.05604 | 2 | 0.3938 | 0.5303 | 0.9206 |
| 2 | rs7604989 | 60987297 | 2 | 0.2457 | 0.2232 | 1 | 3.18 | 0.07455 | 1.134 |
| 2 | rs1177301 | 61167524 | 1 | 0.1872 | 0.1893 | 2 | 0.0312 | 0.8598 | 0.9866 |
| 2 | rs11889205 | 61301997 | 1 | 0.1251 | 0.1446 | 2 | 3.682 | 0.05499 | 0.846 |
| 2 | rs7591772 | 61315904 | 2 | 0.4724 | 0.4642 | 1 | 0.3085 | 0.5786 | 1.034 |
| 2 | rs17007356 | 61319008 | 1 | 0.1548 | 0.1585 | 2 | 0.1151 | 0.7344 | 0.9726 |
| 2 | rs10196146 | 61458883 | 2 | 0.4947 | 0.4855 | 1 | 0.3834 | 0.5358 | 1.038 |
| 2 | rs11125905 | 62148521 | 1 | 0.1611 | 0.1562 | 2 | 0.2059 | 0.65 | 1.038 |
| 2 | rs11891053 | 62164054 | 1 | 0.1636 | 0.1556 | 2 | 0.5375 | 0.4635 | 1.062 |

FIG. 4.9

| CHR | SNP | BP | A1 | F_A | F_U | A2 | CHISQ | P | OR |
|---|---|---|---|---|---|---|---|---|---|
| 2 | rs11682530 | 62926698 | 1 | 0.3369 | 0.343 | 2 | 0.1908 | 0.6623 | 0.9729 |
| 2 | rs11685079 | 63228104 | 1 | 0.1053 | 0.09952 | 2 | 0.4037 | 0.5252 | 1.065 |
| 2 | rs17432775 | 63319967 | 1 | 0.2322 | 0.2319 | 2 | 0.0005231 | 0.9818 | 1.002 |
| 2 | rs12471038 | 63338143 | 1 | 0.1912 | 0.1879 | 2 | 0.08097 | 0.776 | 1.022 |
| 2 | rs13028443 | 63457221 | 2 | 0.4562 | 0.4647 | 1 | 0.3242 | 0.5691 | 0.9665 |
| 2 | rs2421892 | 63506607 | 1 | 0.4109 | 0.4082 | 2 | 0.0326 | 0.8567 | 1.011 |
| 2 | rs6546032 | 63928580 | 1 | 0.1794 | 0.1591 | 2 | 3.148 | 0.07603 | 1.155 |
| 2 | rs3770387 | 63991863 | 1 | 0.2101 | 0.2184 | 2 | 0.4544 | 0.5003 | 0.9522 |
| 2 | rs13018690 | 64004885 | 2 | 0.2016 | 0.1797 | 1 | 3.49 | 0.06175 | 1.153 |
| 2 | rs11691430 | 64639648 | 1 | 0.4047 | 0.3787 | 2 | 3.18 | 0.07456 | 1.115 |
| 2 | rs2287532 | 64650351 | 2 | 0.4051 | 0.3772 | 1 | 3.681 | 0.05503 | 1.124 |
| 2 | rs10186240 | 65431156 | 1 | 0.4166 | 0.4029 | 2 | 0.8737 | 0.3499 | 1.058 |
| 2 | rs268135 | 65461084 | 1 | 0.09757 | 0.08559 | 2 | 1.932 | 0.1645 | 1.155 |
| 2 | rs964505 | 65476693 | 1 | 0.3117 | 0.3222 | 2 | 0.5719 | 0.4495 | 0.9527 |
| 2 | rs2661798 | 65489192 | 1 | 0.4473 | 0.4293 | 2 | 1.478 | 0.224 | 1.076 |
| 2 | rs2028150 | 65508516 | 2 | 0.4308 | 0.3903 | 1 | 7.597 | 0.005845 | 1.182 |
| 2 | rs934736 | 65511423 | 2 | 0.09312 | 0.08551 | 1 | 0.7992 | 0.3713 | 1.098 |
| 2 | rs11126042 | 65617149 | 2 | 0.4146 | 0.3855 | 1 | 3.96 | 0.04658 | 1.129 |
| 2 | rs1673450 | 65637097 | 1 | 0.4668 | 0.472 | 2 | 0.1213 | 0.7276 | 0.9794 |
| 2 | rs1673440 | 65647808 | 2 | 0.3226 | 0.3198 | 1 | 0.04268 | 0.8363 | 1.013 |
| 2 | rs1115847 | 65673559 | 2 | 0.05466 | 0.05556 | 1 | 0.01752 | 0.8947 | 0.9829 |
| 2 | rs6755059 | 65695310 | 1 | 0.05709 | 0.0657 | 2 | 1.459 | 0.2271 | 0.8609 |
| 2 | rs12990354 | 65800072 | 1 | 0.3967 | 0.3956 | 2 | 0.005966 | 0.9384 | 1.005 |
| 2 | rs2287283 | 65825101 | 2 | 0.3821 | 0.3802 | 1 | 0.01718 | 0.8957 | 1.008 |
| 2 | rs10168499 | 65840795 | 2 | 0.3837 | 0.3836 | 1 | 8.89E-05 | 0.9925 | 1.001 |
| 2 | rs17030774 | 65848701 | 1 | 0.1346 | 0.1343 | 2 | 0.001062 | 0.974 | 1.003 |
| 2 | rs11692231 | 65862464 | 1 | 0.1577 | 0.1579 | 2 | 0.0004869 | 0.9824 | 0.9982 |
| 2 | rs10496130 | 65878978 | 2 | 0.161 | 0.1643 | 1 | 0.08798 | 0.7668 | 0.9763 |
| 2 | rs17030854 | 65887949 | 1 | 0.205 | 0.2166 | 2 | 0.9132 | 0.3393 | 0.9326 |
| 2 | rs7595602 | 65929367 | 1 | 0.3279 | 0.343 | 2 | 1.147 | 0.2842 | 0.9347 |
| 2 | rs12471505 | 65936851 | 1 | 0.1032 | 0.1044 | 2 | 0.01771 | 0.8941 | 0.9871 |
| 2 | rs976395 | 65948431 | 1 | 0.171 | 0.1783 | 2 | 0.4137 | 0.5201 | 0.9508 |
| 2 | rs13402656 | 65981203 | 1 | 0.2158 | 0.2165 | 2 | 0.003393 | 0.9535 | 0.9958 |
| 2 | rs6717463 | 65993632 | 2 | 0.2567 | 0.2551 | 1 | 0.01548 | 0.901 | 1.009 |
| 2 | rs7582590 | 66002988 | 2 | 0.09069 | 0.09614 | 1 | 0.3955 | 0.5294 | 0.9377 |
| 2 | rs7588644 | 66016721 | 2 | 0.4153 | 0.4226 | 1 | 0.2473 | 0.619 | 0.9704 |
| 2 | rs17031161 | 66017963 | 1 | 0.3235 | 0.3266 | 2 | 0.04897 | 0.8249 | 0.986 |
| 2 | rs11126068 | 66025602 | 1 | 0.3267 | 0.3285 | 2 | 0.01602 | 0.8993 | 0.9918 |
| 2 | rs7566966 | 66035695 | 1 | 0.3832 | 0.3853 | 2 | 0.02062 | 0.8858 | 0.9912 |
| 2 | rs10180226 | 66062989 | 1 | 0.2366 | 0.2435 | 2 | 0.2914 | 0.5893 | 0.963 |
| 2 | rs6719672 | 66087706 | 2 | 0.1831 | 0.1886 | 1 | 0.2206 | 0.6386 | 0.9647 |
| 2 | rs10208749 | 66099590 | 2 | 0.331 | 0.3462 | 1 | 1.16 | 0.2814 | 0.9344 |
| 2 | rs2699783 | 66112205 | 1 | 0.08793 | 0.09227 | 2 | 0.2598 | 0.6103 | 0.9484 |
| 2 | rs4544423 | 66603521 | 1 | 0.4178 | 0.4019 | 2 | 1.174 | 0.2786 | 1.068 |
| 2 | rs6728018 | 66603769 | 1 | 0.3359 | 0.3208 | 2 | 1.167 | 0.2801 | 1.071 |
| 2 | rs10173197 | 68256339 | 1 | 0.3538 | 0.3338 | 2 | 2 | 0.1573 | 1.093 |
| 2 | rs1822469 | 68308189 | 1 | 0.3947 | 0.4092 | 2 | 0.9776 | 0.3228 | 0.9417 |
| 2 | rs7355584 | 68451793 | 2 | 0.2788 | 0.3058 | 1 | 3.985 | 0.04589 | 0.8774 |
| 2 | rs17035378 | 68452459 | 2 | 0.2789 | 0.3058 | 1 | 3.934 | 0.04733 | 0.8782 |
| 2 | rs7579928 | 68456479 | 1 | 0.3768 | 0.4227 | 2 | 9.893 | 0.001659 | 0.8258 |
| 2 | rs11895938 | 68472586 | 1 | 0.2879 | 0.2618 | 2 | 3.815 | 0.05078 | 1.14 |
| 2 | rs6709464 | 68475724 | 1 | 0.3185 | 0.3008 | 2 | 1.647 | 0.1993 | 1.086 |
| 2 | rs7423646 | 68611684 | 2 | 0.1296 | 0.1252 | 1 | 0.1881 | 0.6645 | 1.04 |
| 2 | rs13427290 | 69154812 | 1 | 0.09643 | 0.1058 | 2 | 1.09 | 0.2965 | 0.9021 |

FIG. 4.10

| CHR | SNP | BP | A1 | F_A | F_U | A2 | CHISQ | P | OR |
|---|---|---|---|---|---|---|---|---|---|
| 2 | rs4854540 | 69159397 | 2 | 0.3671 | 0.3636 | 1 | 0.05817 | 0.8094 | 1.015 |
| 2 | rs6732795 | 69265021 | 2 | 0.3878 | 0.3855 | 1 | 0.02644 | 0.8708 | 1.01 |
| 2 | rs4353686 | 69297745 | 1 | 0.3603 | 0.3739 | 2 | 0.8957 | 0.3439 | 0.9432 |
| 2 | rs7592828 | 69479001 | 1 | 0.4162 | 0.4195 | 2 | 0.05032 | 0.8225 | 0.9865 |
| 2 | rs3755393 | 69611951 | 1 | 0.08138 | 0.0744 | 2 | 0.7613 | 0.3829 | 1.102 |
| 2 | rs6546533 | 69650376 | 2 | 0.3547 | 0.3453 | 1 | 0.4365 | 0.5088 | 1.042 |
| 2 | rs7579933 | 69653475 | 1 | 0.4031 | 0.3897 | 2 | 0.8353 | 0.3607 | 1.057 |
| 2 | rs6728247 | 69665494 | 2 | 0.3984 | 0.3787 | 1 | 1.826 | 0.1766 | 1.086 |
| 2 | rs2068927 | 69832449 | 1 | 0.3269 | 0.3223 | 2 | 0.1084 | 0.742 | 1.021 |
| 2 | rs13018194 | 69879492 | 1 | 0.2584 | 0.2437 | 2 | 1.278 | 0.2583 | 1.081 |
| 2 | rs17037076 | 69887153 | 1 | 0.1816 | 0.1987 | 2 | 2.161 | 0.1415 | 0.8944 |
| 2 | rs3771537 | 69892296 | 2 | 0.466 | 0.4666 | 1 | 0.001867 | 0.9655 | 0.9974 |
| 2 | rs964392 | 70309411 | 2 | 0.3761 | 0.3855 | 1 | 0.4216 | 0.5161 | 0.9609 |
| 2 | rs17005644 | 70550461 | 2 | 0.1596 | 0.1572 | 1 | 0.05217 | 0.8193 | 1.019 |
| 2 | rs7561547 | 70569296 | 1 | 0.4733 | 0.5073 | 2 | 5.199 | 0.02259 | 0.8728 |
| 2 | rs7575526 | 70578339 | 1 | 0.05425 | 0.05266 | 2 | 0.05648 | 0.8121 | 1.032 |
| 2 | rs6546618 | 70777804 | 1 | 0.4907 | 0.4986 | 2 | 0.2785 | 0.5977 | 0.969 |
| 2 | rs13005488 | 70792724 | 2 | 0.2443 | 0.235 | 1 | 0.5368 | 0.4638 | 1.053 |
| 2 | rs3771448 | 70799171 | 2 | 0.2557 | 0.2374 | 1 | 2.012 | 0.1561 | 1.103 |
| 2 | rs3771442 | 70802524 | 1 | 0.08293 | 0.07198 | 2 | 1.875 | 0.171 | 1.166 |
| 2 | rs2160401 | 70816263 | 2 | 0.1876 | 0.2096 | 1 | 3.415 | 0.06462 | 0.8708 |
| 2 | rs3755352 | 70823291 | 2 | 0.05385 | 0.04976 | 1 | 0.3819 | 0.5366 | 1.087 |
| 2 | rs2052272 | 70824270 | 2 | 0.4579 | 0.4619 | 1 | 0.07234 | 0.788 | 0.9839 |
| 2 | rs2080394 | 70862957 | 2 | 0.464 | 0.4565 | 1 | 0.2513 | 0.6162 | 1.03 |
| 2 | rs17655643 | 70864099 | 2 | 0.1117 | 0.1058 | 1 | 0.4096 | 0.5222 | 1.063 |
| 2 | rs357746 | 71227477 | 1 | 0.3029 | 0.2971 | 2 | 0.1813 | 0.6702 | 1.028 |
| 2 | rs13391111 | 71418829 | 1 | 0.1896 | 0.1929 | 2 | 0.07987 | 0.7775 | 0.9788 |
| 2 | rs2670717 | 71462198 | 1 | 0.1449 | 0.1478 | 2 | 0.07516 | 0.784 | 0.9772 |
| 2 | rs10171071 | 71501517 | 2 | 0.1058 | 0.08897 | 1 | 3.58 | 0.05847 | 1.211 |
| 2 | rs11687223 | 71600940 | 2 | 0.3036 | 0.3295 | 1 | 3.478 | 0.06218 | 0.8874 |
| 2 | rs12612922 | 71710270 | 2 | 0.3466 | 0.3309 | 1 | 1.229 | 0.2677 | 1.072 |
| 2 | rs227776 | 71729824 | 2 | 0.1223 | 0.129 | 1 | 0.4637 | 0.4959 | 0.9407 |
| 2 | rs17008611 | 73115358 | 2 | 0.1989 | 0.2074 | 1 | 0.5028 | 0.4783 | 0.9488 |
| 2 | rs13400948 | 73143395 | 1 | 0.1595 | 0.1633 | 2 | 0.1184 | 0.7308 | 0.9725 |
| 2 | rs10169213 | 73640742 | 2 | 0.2324 | 0.2232 | 1 | 0.5413 | 0.4619 | 1.054 |
| 2 | rs1052162 | 73682880 | 1 | 0.233 | 0.2271 | 2 | 0.2233 | 0.6366 | 1.034 |
| 2 | rs2421581 | 73743245 | 1 | 0.136 | 0.1333 | 2 | 0.07034 | 0.7908 | 1.023 |
| 2 | rs831540 | 73977850 | 1 | 0.2053 | 0.2135 | 2 | 0.4651 | 0.4952 | 0.9513 |
| 2 | rs831547 | 74023080 | 2 | 0.3112 | 0.3148 | 1 | 0.06835 | 0.7937 | 0.9833 |
| 2 | rs10182348 | 74386077 | 2 | 0.1085 | 0.1116 | 1 | 0.1101 | 0.7401 | 0.9689 |
| 2 | rs7592599 | 74418565 | 1 | 0.09935 | 0.09952 | 2 | 0.0003452 | 0.9852 | 0.9982 |
| 2 | rs363609 | 74756380 | 2 | 0.08122 | 0.09122 | 1 | 1.422 | 0.2331 | 0.8807 |
| 2 | rs17010464 | 74946939 | 2 | 0.1691 | 0.17 | 1 | 0.007192 | 0.9324 | 0.9933 |
| 2 | rs1872837 | 74959949 | 2 | 0.4866 | 0.4584 | 1 | 3.593 | 0.05803 | 1.12 |
| 2 | rs6546952 | 75155271 | 2 | 0.4846 | 0.5034 | 1 | 1.588 | 0.2076 | 0.9276 |
| 2 | rs7604270 | 75194288 | 1 | 0.3274 | 0.3262 | 2 | 0.007127 | 0.9327 | 1.005 |
| 2 | rs735668 | 75216552 | 1 | 0.4887 | 0.4932 | 2 | 0.09425 | 0.7588 | 0.9819 |
| 2 | rs3771835 | 75227551 | 1 | 0.4574 | 0.4604 | 2 | 0.03986 | 0.8418 | 0.9881 |
| 2 | rs3771843 | 75240284 | 2 | 0.4332 | 0.4585 | 1 | 2.909 | 0.08809 | 0.9028 |
| 2 | rs4853120 | 75269851 | 2 | 0.2522 | 0.2635 | 1 | 0.7526 | 0.3856 | 0.9426 |
| 2 | rs10175841 | 75590315 | 1 | 0.1665 | 0.1739 | 2 | 0.4349 | 0.5096 | 0.9491 |
| 2 | rs10176078 | 75590399 | 1 | 0.1668 | 0.1739 | 2 | 0.4036 | 0.5253 | 0.9509 |
| 2 | rs2422173 | 75631476 | 1 | 0.3995 | 0.4014 | 2 | 0.01757 | 0.8946 | 0.992 |
| 2 | rs1986238 | 75753371 | 1 | 0.2431 | 0.2261 | 2 | 1.813 | 0.1781 | 1.099 |

FIG. 4.11

| CHR | SNP | BP | A1 | F_A | F_U | A2 | CHISQ | P | OR |
|-----|-----|-----|-----|-----|-----|-----|-----|-----|-----|
| 2 | rs2298947 | 75785097 | 1 | 0.3825 | 0.3995 | 2 | 1.371 | 0.2417 | 0.931 |
| 3 | rs6599254 | 38770559 | 1 | 0.3988 | 0.3986 | 2 | 0.0002588 | 0.9872 | 1.001 |
| 3 | rs12630795 | 38771989 | 2 | 0.2455 | 0.257 | 1 | 0.7882 | 0.3746 | 0.9408 |
| 3 | rs4462889 | 38859455 | 2 | 0.2577 | 0.2696 | 1 | 0.8177 | 0.3659 | 0.9407 |
| 3 | rs12107043 | 38860435 | 1 | 0.2336 | 0.2411 | 2 | 0.3488 | 0.5548 | 0.9595 |
| 3 | rs6776510 | 38862257 | 2 | 0.4692 | 0.4589 | 1 | 0.4797 | 0.4885 | 1.042 |
| 3 | rs11708354 | 39090630 | 2 | 0.3097 | 0.3185 | 1 | 0.4071 | 0.5235 | 0.9598 |
| 3 | rs11716067 | 39120477 | 1 | 0.1263 | 0.1162 | 2 | 1.063 | 0.3025 | 1.099 |
| 3 | rs6800630 | 39130383 | 1 | 0.1121 | 0.121 | 2 | 0.8602 | 0.3537 | 0.9175 |
| 3 | rs1274969 | 39144716 | 1 | 0.3251 | 0.3285 | 2 | 0.05923 | 0.8077 | 0.9847 |
| 3 | rs6806372 | 39420784 | 1 | 0.3215 | 0.3357 | 2 | 1.044 | 0.307 | 0.9373 |
| 3 | rs7613883 | 39493320 | 1 | 0.2083 | 0.2034 | 2 | 0.1642 | 0.6853 | 1.03 |
| 3 | rs601925 | 39510366 | 2 | 0.2113 | 0.2025 | 1 | 0.5319 | 0.4658 | 1.055 |
| 3 | rs1068953 | 39531565 | 2 | 0.205 | 0.1918 | 1 | 1.238 | 0.2659 | 1.087 |
| 3 | rs7626681 | 39930497 | 2 | 0.4166 | 0.4004 | 1 | 1.224 | 0.2686 | 1.069 |
| 3 | rs716463 | 39962203 | 1 | 0.0996 | 0.09034 | 2 | 1.119 | 0.2902 | 1.114 |
| 3 | rs6802333 | 39987824 | 1 | 0.3521 | 0.3524 | 2 | 0.0003455 | 0.9852 | 0.9988 |
| 3 | rs6809575 | 40039300 | 2 | 0.408 | 0.4138 | 1 | 0.1525 | 0.6962 | 0.9765 |
| 3 | rs9853925 | 40082299 | 1 | 0.3053 | 0.3013 | 2 | 0.08546 | 0.77 | 1.019 |
| 3 | rs6803028 | 40097627 | 1 | 0.487 | 0.4971 | 2 | 0.4557 | 0.4996 | 0.9606 |
| 3 | rs6789580 | 40121592 | 2 | 0.07328 | 0.06957 | 1 | 0.2338 | 0.6288 | 1.058 |
| 3 | rs939075 | 40140052 | 1 | 0.1599 | 0.1618 | 2 | 0.03066 | 0.861 | 0.9859 |
| 3 | rs4426621 | 40222613 | 1 | 0.4534 | 0.4599 | 2 | 0.1915 | 0.6617 | 0.9741 |
| 3 | rs7644643 | 40325213 | 2 | 0.3687 | 0.3848 | 1 | 1.24 | 0.2656 | 0.9338 |
| 3 | rs9817233 | 40400622 | 1 | 0.2964 | 0.2961 | 2 | 0.0002638 | 0.987 | 1.001 |
| 3 | rs1138401 | 40537731 | 1 | 0.3658 | 0.3647 | 2 | 0.005252 | 0.9422 | 1.004 |
| 3 | rs9311265 | 41258797 | 1 | 0.4485 | 0.4516 | 2 | 0.04112 | 0.8393 | 0.9879 |
| 3 | rs13325965 | 41889946 | 1 | 0.1761 | 0.1676 | 2 | 0.5681 | 0.451 | 1.061 |
| 3 | rs9311301 | 42171978 | 2 | 0.2095 | 0.185 | 1 | 4.235 | 0.03959 | 1.167 |
| 3 | rs4683333 | 42181736 | 2 | 0.4198 | 0.398 | 1 | 2.212 | 0.137 | 1.094 |
| 3 | rs6788180 | 42194456 | 1 | 0.2486 | 0.228 | 2 | 2.617 | 0.1057 | 1.12 |
| 3 | rs39649 | 42427648 | 1 | 0.2711 | 0.2463 | 2 | 3.537 | 0.06002 | 1.138 |
| 3 | rs2940407 | 42605445 | 1 | 0.1858 | 0.1778 | 2 | 0.4901 | 0.4839 | 1.056 |
| 3 | rs7624628 | 42629349 | 1 | 0.1296 | 0.1411 | 2 | 1.279 | 0.2581 | 0.9063 |
| 3 | rs2976533 | 42630050 | 2 | 0.3433 | 0.3507 | 1 | 0.2726 | 0.6016 | 0.9678 |
| 3 | rs9830553 | 42742469 | 1 | 0.4668 | 0.4812 | 2 | 0.9344 | 0.3337 | 0.9439 |
| 3 | rs12490743 | 42763918 | 2 | 0.1888 | 0.1883 | 1 | 0.002067 | 0.9637 | 1.003 |
| 3 | rs11915803 | 42775295 | 2 | 0.2773 | 0.2874 | 1 | 0.5688 | 0.4507 | 0.9513 |
| 3 | rs3846063 | 42778832 | 1 | 0.2619 | 0.2372 | 2 | 3.671 | 0.05535 | 1.141 |
| 3 | rs11919717 | 42858006 | 1 | 0.3865 | 0.3656 | 2 | 2.108 | 0.1465 | 1.094 |
| 3 | rs734969 | 42892910 | 2 | 0.3468 | 0.3251 | 1 | 2.377 | 0.1231 | 1.102 |
| 3 | rs604033 | 43096563 | 2 | 0.2121 | 0.2092 | 1 | 0.05959 | 0.8071 | 1.018 |
| 3 | rs17075558 | 43333930 | 2 | 0.05182 | 0.06238 | 1 | 2.348 | 0.1254 | 0.8215 |
| 3 | rs1482650 | 43459673 | 1 | 0.2721 | 0.2493 | 2 | 3.027 | 0.08187 | 1.126 |
| 3 | rs9815196 | 43495211 | 1 | 0.4543 | 0.4256 | 2 | 3.749 | 0.05285 | 1.123 |
| 3 | rs1482651 | 43623039 | 2 | 0.3552 | 0.3264 | 1 | 4.046 | 0.04428 | 1.137 |
| 3 | rs2029342 | 44349775 | 1 | 0.347 | 0.3301 | 2 | 1.426 | 0.2324 | 1.078 |
| 3 | rs6779938 | 44396887 | 1 | 0.4738 | 0.4625 | 2 | 0.5734 | 0.4489 | 1.047 |
| 3 | rs4234448 | 44741883 | 2 | 0.4543 | 0.4425 | 1 | 0.6273 | 0.4283 | 1.049 |
| 3 | rs1520082 | 44927411 | 2 | 0.4566 | 0.4666 | 1 | 0.4487 | 0.503 | 0.9607 |
| 3 | rs2056321 | 45058137 | 2 | 0.4931 | 0.4995 | 1 | 0.1845 | 0.6675 | 0.9747 |
| 3 | rs854208 | 45427582 | 1 | 0.2684 | 0.2812 | 2 | 0.9182 | 0.338 | 0.9381 |
| 3 | rs1949304 | 45475954 | 2 | 0.3699 | 0.3614 | 1 | 0.3574 | 0.55 | 1.038 |
| 3 | rs267270 | 45568241 | 1 | 0.3898 | 0.3702 | 2 | 1.793 | 0.1806 | 1.087 |

FIG. 4.12

| CHR | SNP | BP | A1 | F_A | F_U | A2 | CHISQ | P | OR |
|-----|-----|-----|-----|-----|-----|-----|-----|-----|-----|
| 3 | rs267209 | 45627165 | 1 | 0.2613 | 0.2604 | 2 | 0.005366 | 0.9416 | 1.005 |
| 3 | rs2235130 | 45652966 | 2 | 0.1062 | 0.09807 | 1 | 0.8014 | 0.3707 | 1.092 |
| 3 | rs2742393 | 45732421 | 1 | 0.4598 | 0.4546 | 2 | 0.1241 | 0.7247 | 1.021 |
| 3 | rs2742395 | 45733599 | 1 | 0.3335 | 0.3269 | 2 | 0.2203 | 0.6388 | 1.03 |
| 3 | rs4535265 | 45952870 | 1 | 0.3964 | 0.3951 | 2 | 0.007759 | 0.9298 | 1.005 |
| 3 | rs3851346 | 46028585 | 2 | 0.1215 | 0.1116 | 1 | 1.061 | 0.303 | 1.101 |
| 3 | rs13080979 | 46208694 | 2 | 0.08866 | 0.08027 | 1 | 1.021 | 0.3122 | 1.115 |
| 3 | rs11130094 | 46516507 | 1 | 0.3639 | 0.4 | 2 | 6.24 | 0.01249 | 0.858 |
| 3 | rs7648827 | 46561183 | 1 | 0.08164 | 0.07349 | 2 | 1.025 | 0.3114 | 1.121 |
| 3 | rs2159400 | 47350894 | 2 | 0.4251 | 0.4135 | 1 | 0.6194 | 0.4313 | 1.049 |
| 3 | rs6780013 | 47427122 | 1 | 0.41 | 0.4018 | 2 | 0.3145 | 0.5749 | 1.035 |
| 3 | rs4858801 | 47615926 | 1 | 0.341 | 0.3675 | 2 | 3.449 | 0.06328 | 0.8907 |
| 3 | rs184388 | 47914630 | 1 | 0.3061 | 0.3386 | 2 | 5.483 | 0.0192 | 0.8614 |
| 3 | rs936427 | 48276078 | 1 | 0.3031 | 0.3366 | 2 | 5.811 | 0.01592 | 0.8573 |
| 3 | rs6796491 | 48405469 | 2 | 0.4421 | 0.4971 | 1 | 13.58 | 0.0002288 | 0.8017 |
| 3 | rs6773261 | 48650068 | 2 | 0.1112 | 0.1121 | 1 | 0.008923 | 0.9247 | 0.9911 |
| 3 | rs4974088 | 48873753 | 1 | 0.2437 | 0.2058 | 2 | 9.249 | 0.002356 | 1.244 |
| 3 | rs11706052 | 49039114 | 2 | 0.1114 | 0.1155 | 1 | 0.1823 | 0.6694 | 0.9607 |
| 3 | rs1464567 | 49434256 | 2 | 0.4145 | 0.429 | 1 | 0.9682 | 0.3251 | 0.9424 |
| 3 | rs9858280 | 49572741 | 2 | 0.3195 | 0.3345 | 1 | 1.138 | 0.2861 | 0.9345 |
| 3 | rs9830730 | 49650879 | 2 | 0.1719 | 0.1659 | 1 | 0.2956 | 0.5867 | 1.044 |
| 3 | rs2276864 | 49869034 | 2 | 0.2968 | 0.3148 | 1 | 1.727 | 0.1887 | 0.9185 |
| 3 | rs2681780 | 49872834 | 1 | 0.4603 | 0.4744 | 2 | 0.896 | 0.3438 | 0.945 |
| 3 | rs12489386 | 49902507 | 1 | 0.07293 | 0.07488 | 2 | 0.06233 | 0.8028 | 0.972 |
| 3 | rs2526747 | 50057918 | 2 | 0.3947 | 0.4082 | 1 | 0.8515 | 0.3561 | 0.9455 |
| 3 | rs6446202 | 50194995 | 2 | 0.1089 | 0.1084 | 1 | 0.003146 | 0.9553 | 1.005 |
| 3 | rs2073498 | 50344550 | 1 | 0.1085 | 0.1077 | 2 | 0.006969 | 0.9335 | 1.008 |
| 3 | rs443030 | 50567772 | 2 | 0.1364 | 0.1339 | 1 | 0.05972 | 0.8069 | 1.022 |
| 3 | rs1458387 | 50722489 | 2 | 0.1102 | 0.1164 | 1 | 0.4336 | 0.5102 | 0.94 |
| 3 | rs13080170 | 51089397 | 1 | 0.1538 | 0.1696 | 2 | 2.06 | 0.1512 | 0.8904 |
| 3 | rs4328835 | 51171312 | 1 | 0.09757 | 0.1111 | 2 | 2.221 | 0.1362 | 0.865 |
| 3 | rs1480364 | 51242390 | 1 | 0.1247 | 0.1456 | 2 | 4.213 | 0.04011 | 0.8363 |
| 3 | rs893056 | 52084604 | 1 | 0.07293 | 0.08019 | 2 | 0.8424 | 0.3587 | 0.9024 |
| 3 | rs12493204 | 52100433 | 2 | 0.07658 | 0.07681 | 1 | 0.0008511 | 0.9767 | 0.9967 |
| 3 | rs352162 | 52228009 | 1 | 0.4776 | 0.4816 | 2 | 0.07125 | 0.7895 | 0.9842 |
| 3 | rs6793317 | 52274026 | 2 | 0.1146 | 0.1111 | 1 | 0.1348 | 0.7135 | 1.035 |
| 3 | rs634382 | 52457891 | 1 | 0.1898 | 0.1838 | 2 | 0.2688 | 0.6041 | 1.04 |
| 3 | rs2336545 | 52762643 | 2 | 0.3887 | 0.3965 | 1 | 0.2913 | 0.5894 | 0.9676 |
| 3 | rs1042779 | 52796051 | 2 | 0.3675 | 0.3733 | 1 | 0.1625 | 0.6868 | 0.9754 |
| 3 | rs6445541 | 52855168 | 1 | 0.385 | 0.3906 | 2 | 0.1481 | 0.7004 | 0.9767 |
| 3 | rs2276825 | 52861645 | 2 | 0.2415 | 0.2444 | 1 | 0.0534 | 0.8172 | 0.9841 |
| 3 | rs11708390 | 52871449 | 1 | 0.1453 | 0.1487 | 2 | 0.1035 | 0.7477 | 0.9733 |
| 3 | rs2115779 | 53001754 | 2 | 0.3363 | 0.3266 | 1 | 0.4811 | 0.4879 | 1.045 |
| 3 | rs17304995 | 53045795 | 2 | 0.2344 | 0.2355 | 1 | 0.007309 | 0.9319 | 0.994 |
| 3 | rs17052727 | 53045954 | 2 | 0.1028 | 0.1063 | 1 | 0.143 | 0.7053 | 0.9639 |
| 3 | rs2336725 | 53093779 | 2 | 0.4372 | 0.4338 | 1 | 0.05427 | 0.8158 | 1.014 |
| 3 | rs13433700 | 53122926 | 1 | 0.3112 | 0.3053 | 2 | 0.1818 | 0.6698 | 1.028 |
| 3 | rs6793773 | 53239164 | 1 | 0.1946 | 0.1954 | 2 | 0.005297 | 0.942 | 0.9945 |
| 3 | rs9864057 | 53250817 | 1 | 0.2089 | 0.2195 | 2 | 0.7566 | 0.3844 | 0.9388 |
| 3 | rs7637934 | 53268895 | 2 | 0.2339 | 0.2357 | 1 | 0.01934 | 0.8894 | 0.9902 |
| 3 | rs898415 | 53642679 | 2 | 0.4777 | 0.4874 | 1 | 0.4266 | 0.5137 | 0.9618 |
| 3 | rs6763245 | 53650755 | 1 | 0.1882 | 0.1899 | 2 | 0.02113 | 0.8844 | 0.989 |
| 3 | rs2633727 | 53721129 | 1 | 0.1419 | 0.1509 | 2 | 0.721 | 0.3958 | 0.9309 |
| 3 | rs11708227 | 53723073 | 2 | 0.2332 | 0.2365 | 1 | 0.06668 | 0.7962 | 0.982 |

FIG. 4.13

| CHR | SNP | BP | A1 | F_A | F_U | A2 | CHISQ | P | OR |
|-----|-----|----|----|-----|-----|----|-------|---|----|
| 3 | rs4687756 | 53879951 | 2 | 0.2684 | 0.3106 | 1 | 9.791 | 0.001754 | 0.8143 |
| 3 | rs9881544 | 54134457 | 2 | 0.4049 | 0.4081 | 1 | 0.0471 | 0.8282 | 0.9868 |
| 3 | rs4928000 | 54167957 | 1 | 0.4344 | 0.4406 | 2 | 0.177 | 0.674 | 0.975 |
| 3 | rs11926147 | 54170136 | 1 | 0.2613 | 0.2792 | 2 | 1.828 | 0.1763 | 0.9133 |
| 3 | rs7649696 | 54195848 | 1 | 0.2512 | 0.2367 | 2 | 1.281 | 0.2577 | 1.082 |
| 3 | rs6792292 | 54262765 | 2 | 0.4161 | 0.4217 | 1 | 0.1433 | 0.705 | 0.9772 |
| 3 | rs11130410 | 54283880 | 2 | 0.487 | 0.4773 | 1 | 0.4294 | 0.5123 | 1.04 |
| 3 | rs6445643 | 54306112 | 2 | 0.2389 | 0.2536 | 1 | 1.324 | 0.2499 | 0.9236 |
| 3 | rs7431577 | 54314952 | 2 | 0.2713 | 0.2667 | 1 | 0.1205 | 0.7285 | 1.024 |
| 3 | rs7431093 | 54371540 | 1 | 0.1368 | 0.1277 | 2 | 0.8249 | 0.3638 | 1.083 |
| 3 | rs1868508 | 54377117 | 1 | 0.2409 | 0.2311 | 2 | 0.5926 | 0.4414 | 1.056 |
| 3 | rs11711662 | 54382806 | 2 | 0.3337 | 0.3308 | 1 | 0.04515 | 0.8317 | 1.014 |
| 3 | rs4974369 | 54446846 | 2 | 0.1785 | 0.1807 | 1 | 0.03481 | 0.852 | 0.9856 |
| 3 | rs17140 | 54540948 | 1 | 0.3793 | 0.3749 | 2 | 0.09173 | 0.762 | 1.019 |
| 3 | rs4955857 | 54585513 | 1 | 0.4169 | 0.4396 | 2 | 2.366 | 0.124 | 0.9115 |
| 3 | rs605284 | 54621075 | 2 | 0.468 | 0.4894 | 1 | 2.059 | 0.1514 | 0.918 |
| 3 | rs10510770 | 54626345 | 1 | 0.2364 | 0.2519 | 2 | 1.467 | 0.2257 | 0.9194 |
| 3 | rs3868872 | 54719213 | 2 | 0.2502 | 0.2539 | 1 | 0.08028 | 0.7769 | 0.9807 |
| 3 | rs11914780 | 54756671 | 1 | 0.1943 | 0.1983 | 2 | 0.1101 | 0.74 | 0.9754 |
| 3 | rs17054369 | 54785691 | 2 | 0.05951 | 0.06957 | 1 | 1.897 | 0.1685 | 0.8464 |
| 3 | rs11130435 | 54842353 | 2 | 0.1045 | 0.1082 | 1 | 0.1677 | 0.6822 | 0.9612 |
| 3 | rs9815947 | 54929698 | 1 | 0.4444 | 0.4516 | 2 | 0.2323 | 0.6298 | 0.9715 |
| 3 | rs6774525 | 54938838 | 2 | 0.3268 | 0.3279 | 1 | 0.00519 | 0.9426 | 0.9954 |
| 3 | rs6804643 | 54953081 | 1 | 0.1227 | 0.1217 | 2 | 0.009135 | 0.9239 | 1.009 |
| 3 | rs1878110 | 54956017 | 2 | 0.4186 | 0.4104 | 1 | 0.3105 | 0.5774 | 1.034 |
| 3 | rs881820 | 54978773 | 1 | 0.4579 | 0.4448 | 2 | 0.7763 | 0.3783 | 1.054 |
| 3 | rs1795632 | 55557840 | 2 | 0.3945 | 0.39 | 1 | 0.09375 | 0.7595 | 1.019 |
| 3 | rs2649859 | 55631385 | 2 | 0.05794 | 0.057 | 1 | 0.01823 | 0.8926 | 1.017 |
| 3 | rs709338 | 55716782 | 1 | 0.381 | 0.3749 | 2 | 0.1778 | 0.6733 | 1.026 |
| 3 | rs815413 | 55727339 | 1 | 0.4198 | 0.4072 | 2 | 0.7461 | 0.3877 | 1.054 |
| 3 | rs9835843 | 55746896 | 1 | 0.1583 | 0.1527 | 2 | 0.2727 | 0.6015 | 1.044 |
| 3 | rs9849285 | 55765069 | 2 | 0.2737 | 0.2628 | 1 | 0.6787 | 0.41 | 1.057 |
| 3 | rs10049464 | 55797044 | 2 | 0.03687 | 0.03913 | 1 | 0.1575 | 0.6915 | 0.9401 |
| 3 | rs7615473 | 55825018 | 1 | 0.2526 | 0.2367 | 2 | 1.541 | 0.2145 | 1.09 |
| 3 | rs6763408 | 55893392 | 1 | 0.0915 | 0.08213 | 2 | 1.243 | 0.265 | 1.126 |
| 3 | rs11929620 | 55907540 | 1 | 0.1655 | 0.1638 | 2 | 0.02314 | 0.8791 | 1.012 |
| 3 | rs11926232 | 55911457 | 2 | 0.0515 | 0.04976 | 1 | 0.07094 | 0.79 | 1.037 |
| 3 | rs11713228 | 55985742 | 2 | 0.2826 | 0.2797 | 1 | 0.04624 | 0.8297 | 1.014 |
| 3 | rs6780386 | 56021386 | 1 | 0.36 | 0.3539 | 2 | 0.1818 | 0.6698 | 1.027 |
| 3 | rs10461036 | 56062141 | 1 | 0.1482 | 0.155 | 2 | 0.4124 | 0.5208 | 0.9479 |
| 3 | rs2171072 | 56090870 | 2 | 0.2919 | 0.2952 | 1 | 0.05795 | 0.8098 | 0.9844 |
| 3 | rs1078309 | 56193929 | 2 | 0.05668 | 0.05749 | 1 | 0.01366 | 0.907 | 0.9851 |
| 3 | rs2316483 | 56196187 | 1 | 0.03644 | 0.04106 | 2 | 0.6502 | 0.4201 | 0.8831 |
| 3 | rs7628255 | 56198424 | 2 | 0.1386 | 0.1353 | 1 | 0.1042 | 0.7469 | 1.028 |
| 3 | rs11707901 | 56219203 | 2 | 0.3988 | 0.3995 | 1 | 0.002513 | 0.96 | 0.997 |
| 3 | rs11130512 | 56323746 | 1 | 0.2685 | 0.2655 | 2 | 0.05092 | 0.8215 | 1.015 |
| 3 | rs17825415 | 56348021 | 2 | 0.3174 | 0.3058 | 1 | 0.7073 | 0.4004 | 1.056 |
| 3 | rs13098861 | 56390879 | 1 | 0.2804 | 0.2739 | 2 | 0.2355 | 0.6275 | 1.033 |
| 3 | rs931461 | 56421736 | 2 | 0.3562 | 0.3443 | 1 | 0.6911 | 0.4058 | 1.054 |
| 3 | rs1491163 | 56428356 | 1 | 0.2231 | 0.2031 | 2 | 2.672 | 0.1021 | 1.127 |
| 3 | rs9855289 | 56446200 | 1 | 0.2607 | 0.2684 | 2 | 0.3385 | 0.5607 | 0.9615 |
| 3 | rs9833926 | 56625218 | 2 | 0.4792 | 0.4477 | 1 | 4.465 | 0.0346 | 1.135 |
| 3 | rs2291501 | 56629181 | 1 | 0.2553 | 0.2471 | 2 | 0.3988 | 0.5277 | 1.044 |
| 3 | rs6794450 | 56673306 | 1 | 0.1135 | 0.09952 | 2 | 2.286 | 0.1305 | 1.158 |

FIG. 4.14

| CHR | SNP | BP | A1 | F_A | F_U | A2 | CHISQ | P | OR |
|---|---|---|---|---|---|---|---|---|---|
| 3 | rs4574264 | 56735955 | 2 | 0.1625 | 0.1599 | 1 | 0.05523 | 0.8142 | 1.019 |
| 3 | rs2129821 | 57111020 | 2 | 0.1074 | 0.1006 | 1 | 0.5561 | 0.4558 | 1.076 |
| 3 | rs17216893 | 57157536 | 2 | 0.1279 | 0.1372 | 1 | 0.8425 | 0.3587 | 0.9226 |
| 3 | rs11710045 | 57212261 | 2 | 0.3852 | 0.3775 | 1 | 0.2823 | 0.5952 | 1.033 |
| 3 | rs3806622 | 57235870 | 1 | 0.4275 | 0.4391 | 2 | 0.6168 | 0.4323 | 0.9539 |
| 3 | rs7620538 | 57329752 | 1 | 0.2208 | 0.2227 | 2 | 0.02419 | 0.8764 | 0.9889 |
| 3 | rs7614741 | 57505811 | 1 | 0.1903 | 0.1855 | 2 | 0.1682 | 0.6817 | 1.032 |
| 3 | rs2121741 | 57506557 | 2 | 0.466 | 0.4531 | 1 | 0.7488 | 0.3869 | 1.053 |
| 3 | rs9850535 | 57548886 | 1 | 0.1633 | 0.1638 | 2 | 0.001881 | 0.9654 | 0.9965 |
| 3 | rs9818221 | 57891501 | 1 | 0.2536 | 0.2493 | 2 | 0.1142 | 0.7354 | 1.023 |
| 3 | rs1718459 | 57985340 | 2 | 0.31 | 0.3062 | 1 | 0.07476 | 0.7845 | 1.018 |
| 3 | rs12715521 | 58038673 | 1 | 0.359 | 0.3549 | 2 | 0.0809 | 0.7761 | 1.018 |
| 3 | rs2362907 | 58088970 | 1 | 0.2616 | 0.258 | 2 | 0.07524 | 0.7839 | 1.019 |
| 3 | rs13062948 | 58121060 | 1 | 0.1036 | 0.1039 | 2 | 0.0005916 | 0.9806 | 0.9976 |
| 3 | rs9855113 | 58151883 | 2 | 0.3374 | 0.3358 | 1 | 0.01356 | 0.9073 | 1.007 |
| 3 | rs17058991 | 58167430 | 1 | 0.07989 | 0.07923 | 2 | 0.00668 | 0.9349 | 1.009 |
| 3 | rs9862934 | 58256716 | 2 | 0.253 | 0.2311 | 1 | 2.933 | 0.08679 | 1.127 |
| 3 | rs2363688 | 58257168 | 1 | 0.298 | 0.314 | 2 | 1.365 | 0.2426 | 0.9273 |
| 3 | rs9846885 | 58260829 | 1 | 0.3405 | 0.3219 | 2 | 1.756 | 0.1851 | 1.088 |
| 3 | rs9871725 | 58267565 | 2 | 0.08759 | 0.09082 | 1 | 0.1447 | 0.7036 | 0.961 |
| 3 | rs4390943 | 58351679 | 2 | 0.1941 | 0.2009 | 1 | 0.3272 | 0.5673 | 0.9581 |
| 3 | rs11130642 | 58395082 | 1 | 0.3788 | 0.3913 | 2 | 0.7494 | 0.3867 | 0.9484 |
| 3 | rs3749290 | 58627332 | 1 | 0.1107 | 0.1029 | 2 | 0.717 | 0.3971 | 1.085 |
| 3 | rs17556835 | 59004915 | 2 | 0.1888 | 0.1932 | 1 | 0.1424 | 0.7059 | 0.9718 |
| 3 | rs11707783 | 59921424 | 2 | 0.1195 | 0.1367 | 1 | 2.991 | 0.08371 | 0.8572 |
| 3 | rs6800973 | 59927192 | 1 | 0.1155 | 0.1101 | 2 | 0.3194 | 0.572 | 1.055 |
| 3 | rs3772499 | 59953237 | 1 | 0.449 | 0.4324 | 2 | 1.262 | 0.2613 | 1.07 |
| 3 | rs17254898 | 59964822 | 1 | 0.1248 | 0.1121 | 2 | 1.738 | 0.1875 | 1.13 |
| 3 | rs2630207 | 60034081 | 2 | 0.2565 | 0.2832 | 1 | 4.072 | 0.04359 | 0.8733 |
| 3 | rs4679636 | 60078066 | 1 | 0.06726 | 0.06377 | 2 | 0.2238 | 0.6361 | 1.059 |
| 3 | rs9682987 | 60094030 | 1 | 0.07212 | 0.07295 | 2 | 0.01136 | 0.9151 | 0.9878 |
| 3 | rs9827643 | 60188341 | 2 | 0.168 | 0.1792 | 1 | 0.9887 | 0.32 | 0.9248 |
| 3 | rs9849664 | 60245441 | 1 | 0.2141 | 0.2082 | 2 | 0.2349 | 0.6279 | 1.036 |
| 3 | rs6779529 | 60267352 | 1 | 0.4599 | 0.4618 | 2 | 0.01721 | 0.8956 | 0.9922 |
| 3 | rs1569348 | 60284793 | 1 | 0.3949 | 0.3685 | 2 | 3.32 | 0.06843 | 1.118 |
| 3 | rs12494639 | 60287955 | 1 | 0.4513 | 0.4685 | 2 | 1.339 | 0.2472 | 0.9331 |
| 3 | rs11716358 | 60305430 | 2 | 0.1571 | 0.1705 | 1 | 1.495 | 0.2215 | 0.9062 |
| 3 | rs2687165 | 60340493 | 1 | 0.3502 | 0.3512 | 2 | 0.004999 | 0.9436 | 0.9956 |
| 3 | rs9836899 | 60343422 | 2 | 0.264 | 0.2539 | 1 | 0.5999 | 0.4386 | 1.054 |
| 3 | rs9868373 | 60437034 | 2 | 0.2461 | 0.2626 | 1 | 1.603 | 0.2055 | 0.917 |
| 3 | rs2362888 | 60447690 | 1 | 0.07773 | 0.08551 | 2 | 0.9116 | 0.3397 | 0.9014 |
| 3 | rs9824412 | 60475969 | 1 | 0.1957 | 0.2184 | 2 | 3.531 | 0.06022 | 0.871 |
| 3 | rs1447980 | 60477487 | 2 | 0.4218 | 0.4413 | 1 | 1.725 | 0.1891 | 0.9234 |
| 3 | rs2205350 | 60779151 | 1 | 0.08306 | 0.09768 | 2 | 2.943 | 0.08626 | 0.8368 |
| 3 | rs2205353 | 60782868 | 1 | 0.4146 | 0.41 | 2 | 0.0984 | 0.7538 | 1.019 |
| 3 | rs9849474 | 60847026 | 1 | 0.2164 | 0.2295 | 2 | 1.117 | 0.2905 | 0.9272 |
| 3 | rs7631028 | 60903962 | 1 | 0.4445 | 0.4333 | 2 | 0.5736 | 0.4488 | 1.047 |
| 3 | rs7374239 | 60967972 | 1 | 0.1767 | 0.1744 | 2 | 0.0399 | 0.8417 | 1.016 |
| 3 | rs11130811 | 61011434 | 1 | 0.1352 | 0.1319 | 2 | 0.1084 | 0.742 | 1.029 |
| 3 | rs9843941 | 61029053 | 1 | 0.3413 | 0.3483 | 2 | 0.2453 | 0.6204 | 0.9694 |
| 3 | rs11712505 | 61069393 | 1 | 0.4789 | 0.4821 | 2 | 0.04506 | 0.8319 | 0.9874 |
| 3 | rs11707984 | 61125198 | 1 | 0.4052 | 0.4053 | 2 | 7.61E-05 | 0.993 | 0.9995 |
| 3 | rs1403968 | 61147358 | 1 | 0.1449 | 0.1431 | 2 | 0.02976 | 0.863 | 1.015 |
| 3 | rs1916801 | 61162086 | 2 | 0.4065 | 0.411 | 1 | 0.09336 | 0.7599 | 0.9816 |

FIG. 4.15

| CHR | SNP | BP | A1 | F_A | F_U | A2 | CHISQ | P | OR |
|---|---|---|---|---|---|---|---|---|---|
| 3 | rs13434101 | 61561221 | 2 | 0.1143 | 0.1024 | 1 | 1.629 | 0.2019 | 1.131 |
| 3 | rs10433637 | 61562288 | 2 | 0.09157 | 0.09865 | 1 | 0.6561 | 0.4179 | 0.9211 |
| 3 | rs645957 | 61738529 | 1 | 0.1146 | 0.1256 | 2 | 1.301 | 0.254 | 0.9008 |
| 3 | rs592179 | 61800481 | 1 | 0.3239 | 0.3246 | 2 | 0.002899 | 0.9571 | 0.9966 |
| 3 | rs652132 | 61805700 | 2 | 0.16 | 0.167 | 1 | 0.3976 | 0.5283 | 0.9504 |
| 3 | rs12630640 | 61822219 | 1 | 0.115 | 0.1126 | 2 | 0.06533 | 0.7983 | 1.024 |
| 3 | rs11713415 | 61825569 | 1 | 0.05992 | 0.05845 | 2 | 0.04336 | 0.8351 | 1.027 |
| 3 | rs1479394 | 61925208 | 2 | 0.1975 | 0.2123 | 1 | 1.515 | 0.2183 | 0.9131 |
| 3 | rs4260433 | 61936842 | 1 | 0.3918 | 0.3814 | 2 | 0.5052 | 0.4772 | 1.045 |
| 3 | rs1382796 | 61940506 | 2 | 0.4911 | 0.4947 | 1 | 0.05819 | 0.8094 | 0.9857 |
| 3 | rs7434122 | 61987727 | 2 | 0.4931 | 0.4898 | 1 | 0.0483 | 0.826 | 1.013 |
| 3 | rs9870965 | 62048356 | 1 | 0.2306 | 0.2246 | 2 | 0.2238 | 0.6362 | 1.034 |
| 3 | rs11714506 | 62054802 | 2 | 0.2097 | 0.1952 | 1 | 1.473 | 0.2249 | 1.094 |
| 3 | rs10510871 | 62065420 | 1 | 0.1194 | 0.1034 | 2 | 2.915 | 0.08776 | 1.176 |
| 3 | rs11715357 | 62068038 | 2 | 0.3575 | 0.3444 | 1 | 0.8408 | 0.3592 | 1.059 |
| 3 | rs2136065 | 62068318 | 2 | 0.2381 | 0.2389 | 1 | 0.004183 | 0.9484 | 0.9955 |
| 3 | rs11711066 | 62145655 | 2 | 0.1146 | 0.128 | 1 | 1.918 | 0.1661 | 0.8814 |
| 3 | rs12634279 | 62524032 | 2 | 0.07733 | 0.07198 | 1 | 0.4648 | 0.4954 | 1.081 |
| 3 | rs17066787 | 62635258 | 2 | 0.05951 | 0.06039 | 1 | 0.01521 | 0.9018 | 0.9846 |
| 3 | rs11710428 | 62653465 | 2 | 0.1248 | 0.1261 | 1 | 0.01707 | 0.896 | 0.9883 |
| 3 | rs2367321 | 62657766 | 2 | 0.4769 | 0.4841 | 1 | 0.2297 | 0.6318 | 0.9718 |
| 3 | rs6809056 | 62677848 | 2 | 0.3197 | 0.329 | 1 | 0.444 | 0.5052 | 0.9585 |
| 3 | rs17067030 | 62683741 | 1 | 0.1202 | 0.1068 | 2 | 2.024 | 0.1548 | 1.144 |
| 3 | rs543060 | 62692783 | 1 | 0.266 | 0.271 | 2 | 0.1447 | 0.7036 | 0.9748 |
| 3 | rs478123 | 62714132 | 1 | 0.08666 | 0.1005 | 2 | 2.548 | 0.1105 | 0.8493 |
| 3 | rs186236 | 62748989 | 1 | 0.283 | 0.2843 | 2 | 0.009899 | 0.9207 | 0.9934 |
| 3 | rs304224 | 62771220 | 2 | 0.234 | 0.2272 | 1 | 0.291 | 0.5896 | 1.039 |
| 3 | rs10510888 | 62806347 | 1 | 0.2453 | 0.2333 | 2 | 0.891 | 0.3452 | 1.068 |
| 3 | rs4133197 | 63440721 | 2 | 0.4152 | 0.4261 | 1 | 0.5427 | 0.4613 | 0.9565 |
| 3 | rs9857079 | 63456791 | 2 | 0.1806 | 0.1734 | 1 | 0.3932 | 0.5306 | 1.05 |
| 3 | rs6767426 | 63486129 | 1 | 0.3412 | 0.3636 | 2 | 2.492 | 0.1144 | 0.9062 |
| 3 | rs1394902 | 63494733 | 1 | 0.1976 | 0.1874 | 2 | 0.7423 | 0.3889 | 1.067 |
| 3 | rs2135222 | 63504824 | 2 | 0.1386 | 0.1647 | 1 | 6.026 | 0.0141 | 0.8156 |
| 3 | rs12638653 | 63526304 | 2 | 0.3935 | 0.381 | 1 | 0.7381 | 0.3903 | 1.054 |
| 3 | rs10510899 | 63549171 | 1 | 0.2547 | 0.2406 | 2 | 1.196 | 0.2742 | 1.078 |
| 3 | rs3774721 | 63929689 | 2 | 0.1316 | 0.1319 | 1 | 0.0009167 | 0.9758 | 0.9973 |
| 3 | rs3774723 | 63937379 | 1 | 0.1588 | 0.1431 | 2 | 2.155 | 0.1421 | 1.13 |
| 3 | rs2241823 | 63940133 | 1 | 0.3113 | 0.2901 | 2 | 2.402 | 0.1212 | 1.106 |
| 3 | rs1096170 | 64079428 | 2 | 0.4454 | 0.4439 | 1 | 0.01076 | 0.9174 | 1.006 |
| 3 | rs704383 | 64081969 | 1 | 0.1482 | 0.1581 | 2 | 0.8605 | 0.3536 | 0.9262 |
| 3 | rs17664549 | 64127641 | 2 | 0.234 | 0.2338 | 1 | 0.0002309 | 0.9879 | 1.001 |
| 3 | rs153735 | 64137584 | 2 | 0.05951 | 0.04928 | 1 | 2.278 | 0.1313 | 1.221 |
| 3 | rs153729 | 64153423 | 1 | 0.1332 | 0.1406 | 2 | 0.5204 | 0.4707 | 0.9394 |
| 3 | rs348869 | 102470668 | 2 | 0.4814 | 0.4927 | 1 | 0.5838 | 0.4448 | 0.9554 |
| 4 | rs6854876 | 110872889 | 2 | 0.4073 | 0.4599 | 1 | 12.71 | 0.0003635 | 0.807 |
| 4 | rs2346841 | 110874089 | 1 | 0.06002 | 0.06153 | 2 | 0.04521 | 0.8316 | 0.9738 |
| 4 | rs10033900 | 110878516 | 2 | 0.4623 | 0.5305 | 1 | 20.89 | 4.86E-06 | 0.7612 |
| 4 | rs11726949 | 110884079 | 1 | 0.07652 | 0.05556 | 2 | 7.921 | 0.004886 | 1.409 |
| 4 | rs6848178 | 110885620 | 2 | 0.4296 | 0.4493 | 1 | 1.779 | 0.1823 | 0.9231 |
| 4 | rs9998151 | 110886794 | 2 | 0.05101 | 0.04106 | 1 | 2.516 | 0.1127 | 1.255 |
| 4 | rs13129180 | 110905862 | 2 | 0.2636 | 0.2669 | 1 | 0.0653 | 0.7983 | 0.9829 |
| 4 | rs1000954 | 110929483 | 1 | 0.2885 | 0.2993 | 2 | 0.6366 | 0.425 | 0.9491 |
| 4 | rs7675460 | 110940037 | 1 | 0.3898 | 0.3777 | 2 | 0.6997 | 0.4029 | 1.053 |
| 4 | rs11734488 | 139349583 | 2 | 0.4781 | 0.4986 | 1 | 1.878 | 0.1705 | 0.9215 |

FIG. 4.16

| CHR | SNP | BP | A1 | F_A | F_U | A2 | CHISQ | P | OR |
|---|---|---|---|---|---|---|---|---|---|
| 4 | rs17840386 | 140411360 | 1 | 0.2427 | 0.2357 | 2 | 0.2992 | 0.5844 | 1.039 |
| 4 | rs2667364 | 141001532 | 1 | 0.4785 | 0.4643 | 2 | 0.9204 | 0.3374 | 1.059 |
| 4 | rs1350035 | 141058061 | 1 | 0.4053 | 0.3973 | 2 | 0.2977 | 0.5853 | 1.034 |
| 4 | rs6838773 | 141110216 | 1 | 0.2804 | 0.2947 | 2 | 1.125 | 0.2889 | 0.9326 |
| 4 | rs4863713 | 141126704 | 1 | 0.3837 | 0.3641 | 2 | 1.843 | 0.1746 | 1.087 |
| 4 | rs4863720 | 141142480 | 2 | 0.457 | 0.4437 | 1 | 0.7944 | 0.3728 | 1.055 |
| 4 | rs17373738 | 141154714 | 1 | 0.3672 | 0.3797 | 2 | 0.7529 | 0.3856 | 0.948 |
| 4 | rs12645464 | 141472193 | 2 | 0.1098 | 0.1039 | 1 | 0.4155 | 0.5192 | 1.064 |
| 4 | rs11931851 | 141487390 | 1 | 0.05425 | 0.05556 | 2 | 0.03697 | 0.8475 | 0.9752 |
| 4 | rs6837799 | 141571161 | 1 | 0.09943 | 0.09932 | 2 | 0.0001456 | 0.9904 | 1.001 |
| 4 | rs2322262 | 142794716 | 2 | 0.2702 | 0.2527 | 1 | 1.777 | 0.1825 | 1.095 |
| 4 | rs12504148 | 142809553 | 1 | 0.1335 | 0.1386 | 2 | 0.2516 | 0.6159 | 0.9573 |
| 4 | rs10519613 | 142873534 | 1 | 0.1105 | 0.1169 | 2 | 0.4561 | 0.4995 | 0.9386 |
| 4 | rs6537063 | 142877749 | 2 | 0.483 | 0.4889 | 1 | 0.1579 | 0.6911 | 0.9766 |
| 4 | rs1912713 | 143180634 | 1 | 0.04737 | 0.04396 | 2 | 0.299 | 0.5845 | 1.081 |
| 4 | rs2667094 | 143184439 | 1 | 0.08664 | 0.07447 | 2 | 2.238 | 0.1347 | 1.179 |
| 4 | rs3775612 | 143199115 | 2 | 0.08623 | 0.0744 | 1 | 2.124 | 0.145 | 1.174 |
| 4 | rs13327961 | 143233927 | 1 | 0.2235 | 0.2406 | 2 | 1.852 | 0.1736 | 0.9085 |
| 4 | rs336297 | 143234325 | 2 | 0.3181 | 0.3237 | 1 | 0.1576 | 0.6914 | 0.975 |
| 4 | rs10032149 | 143254870 | 2 | 0.1134 | 0.09961 | 1 | 2.225 | 0.1358 | 1.156 |
| 4 | rs3775658 | 143272034 | 1 | 0.1097 | 0.09574 | 2 | 2.371 | 0.1236 | 1.164 |
| 4 | rs13148456 | 143283889 | 2 | 0.1138 | 0.1338 | 1 | 4.2 | 0.04042 | 0.8309 |
| 4 | rs4956437 | 143310832 | 2 | 0.0661 | 0.05126 | 1 | 4.439 | 0.03512 | 1.31 |
| 4 | rs2636683 | 143317533 | 1 | 0.312 | 0.2957 | 2 | 1.419 | 0.2335 | 1.08 |
| 4 | rs17705389 | 143352101 | 2 | 0.1194 | 0.1005 | 1 | 4.105 | 0.04276 | 1.214 |
| 4 | rs3113511 | 143389594 | 1 | 0.1848 | 0.1498 | 2 | 9.833 | 0.001714 | 1.287 |
| 4 | rs6850050 | 143458214 | 2 | 0.1081 | 0.09903 | 1 | 0.9931 | 0.319 | 1.103 |
| 4 | rs724131 | 143486208 | 2 | 0.4077 | 0.4047 | 1 | 0.0407 | 0.8401 | 1.012 |
| 4 | rs1391095 | 143513473 | 1 | 0.2707 | 0.2655 | 2 | 0.1582 | 0.6908 | 1.027 |
| 4 | rs1353624 | 143514696 | 1 | 0.2229 | 0.2389 | 2 | 1.631 | 0.2016 | 0.9137 |
| 4 | rs17658809 | 143558672 | 1 | 0.3609 | 0.3739 | 2 | 0.8194 | 0.3654 | 0.9456 |
| 4 | rs3775716 | 143565769 | 1 | 0.312 | 0.3114 | 2 | 0.001773 | 0.9664 | 1.003 |
| 4 | rs11100779 | 144324214 | 2 | 0.3233 | 0.3327 | 1 | 0.4465 | 0.504 | 0.9585 |
| 4 | rs11725565 | 144356809 | 1 | 0.396 | 0.413 | 2 | 1.367 | 0.2424 | 0.9315 |
| 4 | rs7698825 | 144570142 | 1 | 0.3449 | 0.3623 | 2 | 1.49 | 0.2223 | 0.9268 |
| 4 | rs17017856 | 144682895 | 2 | 0.4448 | 0.4462 | 1 | 0.009113 | 0.9239 | 0.9943 |
| 4 | rs6845999 | 145785276 | 1 | 0.4351 | 0.4425 | 2 | 0.2499 | 0.6171 | 0.9704 |
| 4 | rs6857302 | 145871113 | 1 | 0.4275 | 0.4072 | 2 | 1.893 | 0.1689 | 1.087 |
| 4 | rs4148234 | 146268004 | 2 | 0.3636 | 0.3865 | 1 | 2.525 | 0.1121 | 0.9069 |
| 4 | rs12501862 | 146293300 | 1 | 0.1255 | 0.1377 | 2 | 1.466 | 0.226 | 0.8989 |
| 4 | rs7662543 | 146684223 | 2 | 0.4785 | 0.4811 | 1 | 0.03046 | 0.8615 | 0.9896 |
| 4 | rs17745315 | 146968677 | 1 | 0.2962 | 0.2816 | 2 | 1.158 | 0.2818 | 1.073 |
| 4 | rs4547811 | 147014071 | 2 | 0.1576 | 0.1758 | 1 | 2.703 | 0.1002 | 0.8769 |
| 4 | rs1567224 | 147059527 | 1 | 0.4117 | 0.4261 | 2 | 0.9526 | 0.3291 | 0.9428 |
| 4 | rs1865531 | 147315543 | 1 | 0.3195 | 0.3297 | 2 | 0.5332 | 0.4653 | 0.9543 |
| 4 | rs6537423 | 147605586 | 2 | 0.2907 | 0.2638 | 1 | 4.062 | 0.04385 | 1.144 |
| 4 | rs17769016 | 147627795 | 2 | 0.2729 | 0.2488 | 1 | 3.379 | 0.06604 | 1.133 |
| 4 | rs7673042 | 147785994 | 2 | 0.05551 | 0.0686 | 1 | 3.342 | 0.06752 | 0.798 |
| 4 | rs10446756 | 147936459 | 1 | 0.228 | 0.2371 | 2 | 0.5154 | 0.4728 | 0.9503 |
| 4 | rs13104820 | 148084064 | 1 | 0.2478 | 0.2582 | 2 | 0.6508 | 0.4198 | 0.9462 |
| 4 | rs10505860 | 148625337 | 1 | 0.2843 | 0.3068 | 2 | 2.741 | 0.09779 | 0.8975 |
| 4 | rs1013842 | 149002852 | 2 | 0.1086 | 0.1135 | 1 | 0.2784 | 0.5978 | 0.9512 |
| 4 | rs6836320 | 149218899 | 1 | 0.2885 | 0.3029 | 2 | 1.123 | 0.2892 | 0.9332 |
| 4 | rs4835488 | 149281643 | 2 | 0.4709 | 0.4575 | 1 | 0.8083 | 0.3686 | 1.055 |

FIG. 4.17

| CHR | SNP | BP | A1 | F_A | F_U | A2 | CHISQ | P | OR |
|---|---|---|---|---|---|---|---|---|---|
| 4 | rs6846591 | 149347200 | 1 | 0.3298 | 0.343 | 2 | 0.8759 | 0.3493 | 0.9427 |
| 4 | rs17484245 | 149400425 | 1 | 0.1862 | 0.1841 | 2 | 0.03536 | 0.8508 | 1.015 |
| 4 | rs10434128 | 149464743 | 1 | 0.1344 | 0.142 | 2 | 0.5495 | 0.4585 | 0.9381 |
| 4 | rs7688209 | 149474120 | 2 | 0.2314 | 0.2522 | 1 | 2.667 | 0.1025 | 0.8926 |
| 4 | rs7668293 | 151217529 | 1 | 0.4093 | 0.4188 | 2 | 0.4216 | 0.5161 | 0.9615 |
| 4 | rs9307870 | 151349501 | 2 | 0.07293 | 0.07053 | 1 | 0.09743 | 0.7549 | 1.037 |
| 4 | rs17503902 | 151433455 | 2 | 0.2908 | 0.2742 | 1 | 1.522 | 0.2172 | 1.085 |
| 4 | rs4696087 | 151442264 | 1 | 0.236 | 0.2202 | 2 | 1.585 | 0.2081 | 1.094 |
| 4 | rs4458434 | 151495247 | 1 | 0.08259 | 0.07544 | 2 | 0.7892 | 0.3744 | 1.103 |
| 4 | rs17504359 | 151526571 | 2 | 0.1749 | 0.1734 | 1 | 0.01689 | 0.8966 | 1.01 |
| 4 | rs11932482 | 151665241 | 1 | 0.2998 | 0.288 | 2 | 0.7591 | 0.3836 | 1.059 |
| 4 | rs4696475 | 151665845 | 1 | 0.262 | 0.2498 | 2 | 0.8804 | 0.3481 | 1.066 |
| 4 | rs6835367 | 151729289 | 1 | 0.09474 | 0.0814 | 2 | 2.477 | 0.1155 | 1.181 |
| 4 | rs9995388 | 151792753 | 2 | 0.05466 | 0.04203 | 1 | 3.861 | 0.04943 | 1.318 |
| 4 | rs10031902 | 152416933 | 1 | 0.4514 | 0.4419 | 2 | 0.4072 | 0.5234 | 1.039 |
| 4 | rs6816002 | 152808889 | 1 | 0.3757 | 0.3744 | 2 | 0.008275 | 0.9275 | 1.006 |
| 4 | rs11099824 | 152837032 | 2 | 0.1312 | 0.1187 | 1 | 1.605 | 0.2052 | 1.122 |
| 4 | rs6817586 | 152862634 | 1 | 0.502 | 0.485 | 2 | 1.305 | 0.2533 | 1.071 |
| 4 | rs2203644 | 153495151 | 1 | 0.301 | 0.3153 | 2 | 1.08 | 0.2987 | 0.9351 |
| 4 | rs6827032 | 153924881 | 2 | 0.3891 | 0.3816 | 1 | 0.2621 | 0.6087 | 1.032 |
| 4 | rs13102607 | 153999269 | 1 | 0.1644 | 0.156 | 2 | 0.5803 | 0.4462 | 1.064 |
| 4 | rs1105889 | 154079622 | 1 | 0.3985 | 0.413 | 2 | 0.982 | 0.3217 | 0.9416 |
| 4 | rs10033643 | 154395829 | 1 | 0.3946 | 0.4101 | 2 | 1.123 | 0.2892 | 0.9376 |
| 4 | rs13120617 | 154429206 | 2 | 0.1794 | 0.1799 | 1 | 0.001914 | 0.9651 | 0.9966 |
| 4 | rs717044 | 154518719 | 1 | 0.1495 | 0.1605 | 2 | 1.047 | 0.3061 | 0.9192 |
| 4 | rs1371156 | 154736189 | 1 | 0.3189 | 0.337 | 2 | 1.686 | 0.1942 | 0.9209 |
| 4 | rs12509119 | 154861140 | 1 | 0.3178 | 0.3459 | 2 | 4.012 | 0.04517 | 0.881 |
| 4 | rs17371021 | 154883745 | 1 | 0.2484 | 0.2469 | 2 | 0.01395 | 0.906 | 1.008 |
| 4 | rs11099899 | 154887037 | 1 | 0.4935 | 0.4633 | 2 | 4.125 | 0.04224 | 1.129 |
| 4 | rs12643219 | 154890248 | 2 | 0.1981 | 0.1828 | 1 | 1.715 | 0.1903 | 1.105 |
| 4 | rs4076040 | 155411482 | 2 | 0.4466 | 0.442 | 1 | 0.09358 | 0.7597 | 1.019 |
| 4 | rs12508287 | 155428591 | 2 | 0.3485 | 0.3536 | 1 | 0.1261 | 0.7226 | 0.978 |
| 4 | rs6853780 | 155548450 | 1 | 0.1864 | 0.1842 | 2 | 0.03614 | 0.8492 | 1.015 |
| 4 | rs4696210 | 155566526 | 1 | 0.4028 | 0.4087 | 2 | 0.1605 | 0.6887 | 0.976 |
| 4 | rs1907155 | 155580694 | 2 | 0.498 | 0.4884 | 1 | 0.4131 | 0.5204 | 1.039 |
| 4 | rs1552638 | 155621815 | 1 | 0.1911 | 0.201 | 2 | 0.6977 | 0.4035 | 0.9393 |
| 4 | rs3857093 | 155627693 | 1 | 0.2688 | 0.2546 | 2 | 1.18 | 0.2775 | 1.076 |
| 4 | rs7680155 | 155685704 | 2 | 0.3255 | 0.3357 | 1 | 0.5342 | 0.4648 | 0.9548 |
| 4 | rs2066865 | 155744726 | 1 | 0.2281 | 0.2437 | 2 | 1.527 | 0.2166 | 0.9169 |
| 4 | rs1047492 | 156486759 | 1 | 0.4628 | 0.4406 | 2 | 2.235 | 0.1349 | 1.094 |
| 4 | rs4234963 | 156944030 | 1 | 0.3866 | 0.3986 | 2 | 0.6703 | 0.4129 | 0.9513 |
| 4 | rs1399046 | 156977268 | 1 | 0.1113 | 0.1232 | 2 | 1.534 | 0.2155 | 0.8917 |
| 4 | rs2102656 | 157094697 | 1 | 0.2259 | 0.2377 | 2 | 0.8828 | 0.3474 | 0.9358 |
| 4 | rs6822892 | 157954123 | 2 | 0.3669 | 0.3169 | 1 | 11.97 | 0.0005397 | 1.244 |
| 4 | rs2881374 | 159258556 | 1 | 0.1146 | 0.1135 | 2 | 0.01224 | 0.9119 | 1.01 |
| 4 | rs4691461 | 159278895 | 1 | 0.1664 | 0.1531 | 2 | 1.47 | 0.2254 | 1.104 |
| 4 | rs7376675 | 159655461 | 1 | 0.1587 | 0.1725 | 2 | 1.548 | 0.2135 | 0.9052 |
| 4 | rs11943598 | 159719811 | 2 | 0.1449 | 0.1296 | 1 | 2.229 | 0.1354 | 1.138 |
| 4 | rs4146954 | 160079743 | 2 | 0.3271 | 0.3208 | 1 | 0.2074 | 0.6488 | 1.029 |
| 4 | rs714462 | 160105220 | 1 | 0.398 | 0.3957 | 2 | 0.0254 | 0.8734 | 1.01 |
| 4 | rs10517694 | 160107628 | 1 | 0.3159 | 0.3266 | 2 | 0.5885 | 0.443 | 0.9522 |
| 4 | rs6849801 | 160110371 | 1 | 0.3211 | 0.3043 | 2 | 1.46 | 0.2269 | 1.081 |
| 6 | rs3116820 | 29259650 | 2 | 0.4493 | 0.4309 | 1 | 1.544 | 0.214 | 1.078 |
| 6 | rs9380122 | 29450215 | 2 | 0.03485 | 0.03816 | 1 | 0.3538 | 0.552 | 0.9099 |

FIG. 4.18

| CHR | SNP | BP | A1 | F_A | F_U | A2 | CHISQ | P | OR |
|---|---|---|---|---|---|---|---|---|---|
| 6 | rs11961013 | 29579913 | 1 | 0.01093 | 0.01063 | 2 | 0.009694 | 0.9216 | 1.029 |
| 6 | rs362527 | 29639797 | 2 | 0.05668 | 0.05411 | 1 | 0.1423 | 0.706 | 1.05 |
| 6 | rs29243 | 29707081 | 1 | 0.01093 | 0.01014 | 2 | 0.06655 | 0.7964 | 1.078 |
| 6 | rs3025623 | 29715726 | 1 | 0.03846 | 0.03865 | 2 | 0.001049 | 0.9742 | 0.995 |
| 6 | rs29233 | 29719208 | 1 | 0.05789 | 0.05797 | 2 | 0.0001201 | 0.9913 | 0.9986 |
| 6 | rs1610714 | 29877808 | 2 | 0.3186 | 0.3201 | 1 | 0.01147 | 0.9147 | 0.9932 |
| 6 | rs1611635 | 29944442 | 1 | 0.1725 | 0.1768 | 2 | 0.1474 | 0.701 | 0.9703 |
| 6 | rs1611430 | 30001440 | 2 | 0.1296 | 0.1409 | 1 | 1.232 | 0.267 | 0.9076 |
| 6 | rs3893538 | 30032404 | 2 | 0.2121 | 0.2249 | 1 | 1.067 | 0.3017 | 0.9283 |
| 6 | rs2523933 | 30040271 | 1 | 0.306 | 0.3095 | 2 | 0.06361 | 0.8009 | 0.9838 |
| 6 | rs6457109 | 30041240 | 2 | 0.1114 | 0.09623 | 1 | 2.777 | 0.09561 | 1.178 |
| 6 | rs2571375 | 30053249 | 2 | 0.08219 | 0.08019 | 1 | 0.05991 | 0.8066 | 1.027 |
| 6 | rs2301751 | 30147463 | 1 | 0.05842 | 0.06573 | 2 | 1.03 | 0.3101 | 0.8819 |
| 6 | rs2516683 | 30456069 | 2 | 0.1349 | 0.1319 | 1 | 0.09009 | 0.7641 | 1.027 |
| 6 | rs1076832 | 30527810 | 1 | 0.174 | 0.1459 | 2 | 6.56 | 0.01043 | 1.233 |
| 6 | rs2524180 | 30534109 | 2 | 0.4057 | 0.4 | 1 | 0.1504 | 0.6982 | 1.024 |
| 6 | rs2516675 | 30538499 | 1 | 0.2492 | 0.2768 | 2 | 4.445 | 0.03501 | 0.8671 |
| 6 | rs762324 | 30563865 | 1 | 0.06599 | 0.06377 | 2 | 0.09167 | 0.7621 | 1.037 |
| 6 | rs2534825 | 30603722 | 1 | 0.3251 | 0.3158 | 2 | 0.4504 | 0.5022 | 1.044 |
| 6 | rs3130244 | 30660916 | 1 | 0.3389 | 0.3332 | 2 | 0.1635 | 0.6859 | 1.026 |
| 6 | rs3129996 | 30759566 | 1 | 0.08745 | 0.1 | 2 | 2.1 | 0.1473 | 0.8625 |
| 6 | rs3130660 | 30814340 | 1 | 0.1094 | 0.1184 | 2 | 0.8978 | 0.3434 | 0.915 |
| 6 | rs10947088 | 30817809 | 2 | 0.03452 | 0.04215 | 1 | 1.786 | 0.1814 | 0.8126 |
| 6 | rs3094121 | 30838939 | 1 | 0.2219 | 0.2374 | 2 | 1.545 | 0.2139 | 0.9158 |
| 6 | rs12526186 | 30844130 | 1 | 0.1835 | 0.1818 | 2 | 0.02258 | 0.8806 | 1.012 |
| 6 | rs1264375 | 30874190 | 2 | 0.3015 | 0.2998 | 1 | 0.0146 | 0.9038 | 1.008 |
| 6 | rs4711240 | 30881218 | 2 | 0.1393 | 0.1145 | 1 | 6.196 | 0.0128 | 1.251 |
| 6 | rs4713391 | 30900214 | 2 | 0.1397 | 0.1145 | 1 | 6.391 | 0.01147 | 1.256 |
| 6 | rs2844657 | 30937501 | 2 | 0.1953 | 0.2053 | 1 | 0.706 | 0.4008 | 0.9394 |
| 6 | rs3132572 | 30969708 | 2 | 0.105 | 0.105 | 1 | 3.61E-07 | 0.9995 | 0.9999 |
| 6 | rs4678 | 31001920 | 1 | 0.2061 | 0.2099 | 2 | 0.09831 | 0.7539 | 0.9772 |
| 6 | rs2517439 | 31050908 | 1 | 0.1406 | 0.1422 | 2 | 0.02276 | 0.8801 | 0.9872 |
| 6 | rs2844685 | 31051062 | 1 | 0.1397 | 0.142 | 2 | 0.05155 | 0.8204 | 0.9807 |
| 6 | rs2517424 | 31057975 | 2 | 0.1397 | 0.1422 | 1 | 0.05769 | 0.8102 | 0.9796 |
| 6 | rs12526820 | 31103765 | 2 | 0.02551 | 0.02077 | 1 | 1.106 | 0.2929 | 1.234 |
| 6 | rs3764808 | 31132652 | 2 | 0.2427 | 0.2094 | 1 | 7.1 | 0.007707 | 1.21 |
| 6 | rs3130955 | 31162490 | 1 | 0.3534 | 0.3816 | 2 | 3.859 | 0.04949 | 0.8857 |
| 6 | rs4947296 | 31166157 | 2 | 0.07652 | 0.06141 | 1 | 3.965 | 0.04646 | 1.266 |
| 6 | rs1265100 | 31213289 | 2 | 0.1483 | 0.1364 | 1 | 1.309 | 0.2526 | 1.103 |
| 6 | rs1265110 | 31227401 | 1 | 0.1105 | 0.1135 | 2 | 0.102 | 0.7494 | 0.9703 |
| 6 | rs3130501 | 31244432 | 1 | 0.2259 | 0.2425 | 2 | 1.734 | 0.1879 | 0.9116 |
| 6 | rs887466 | 31251490 | 1 | 0.3749 | 0.3924 | 2 | 1.462 | 0.2265 | 0.9285 |
| 6 | rs3130457 | 31255173 | 2 | 0.2601 | 0.2669 | 1 | 0.2679 | 0.6048 | 0.9656 |
| 6 | rs4122190 | 31275831 | 2 | 0.02146 | 0.01888 | 1 | 0.377 | 0.5392 | 1.14 |
| 6 | rs4122189 | 31275906 | 1 | 0.2081 | 0.2391 | 2 | 6.275 | 0.01225 | 0.8361 |
| 6 | rs7745906 | 31311987 | 1 | 0.1356 | 0.1643 | 2 | 7.29 | 0.006934 | 0.7984 |
| 6 | rs3869115 | 31312673 | 1 | 0.0389 | 0.04155 | 2 | 0.205 | 0.6507 | 0.9337 |
| 6 | rs7747738 | 31338903 | 1 | 0.02066 | 0.04444 | 2 | 20.86 | 4.94E-06 | 0.4537 |
| 6 | rs2524074 | 31352000 | 2 | 0.2858 | 0.2928 | 1 | 0.2627 | 0.6082 | 0.9669 |
| 6 | rs7382297 | 31355046 | 1 | 0.1291 | 0.1386 | 2 | 0.8783 | 0.3487 | 0.9213 |
| 6 | rs2844603 | 31358833 | 1 | 0.4186 | 0.4391 | 2 | 1.946 | 0.163 | 0.9194 |
| 6 | rs9461684 | 31361423 | 1 | 0.1241 | 0.1015 | 2 | 5.666 | 0.0173 | 1.253 |
| 6 | rs2524050 | 31363520 | 2 | 0.1266 | 0.1422 | 1 | 2.355 | 0.1249 | 0.8744 |
| 6 | rs2524048 | 31364540 | 2 | 0.1227 | 0.1291 | 1 | 0.4248 | 0.5145 | 0.9432 |

FIG. 4.19

| CHR | SNP | BP | A1 | F_A | F_U | A2 | CHISQ | P | OR |
|---|---|---|---|---|---|---|---|---|---|
| 6 | rs2256583 | 31366825 | 2 | 0.1405 | 0.147 | 1 | 0.3895 | 0.5325 | 0.9483 |
| 6 | rs2524160 | 31367833 | 1 | 0.1217 | 0.1281 | 2 | 0.4341 | 0.51 | 0.9424 |
| 6 | rs2894207 | 31371730 | 2 | 0.2196 | 0.2234 | 1 | 0.09394 | 0.7592 | 0.9782 |
| 6 | rs3905495 | 31373518 | 1 | 0.3348 | 0.3253 | 2 | 0.4639 | 0.4958 | 1.044 |
| 6 | rs6457374 | 31380240 | 2 | 0.2457 | 0.257 | 1 | 0.7592 | 0.3836 | 0.9419 |
| 6 | rs364415 | 31381203 | 1 | 0.1228 | 0.129 | 2 | 0.3958 | 0.5293 | 0.9451 |
| 6 | rs1058026 | 31429664 | 2 | 0.1906 | 0.1586 | 1 | 7.901 | 0.004941 | 1.249 |
| 6 | rs3819294 | 31430466 | 1 | 0.07004 | 0.06715 | 2 | 0.147 | 0.7014 | 1.046 |
| 6 | rs2394985 | 31431536 | 1 | 0.01337 | 0.008704 | 2 | 2.205 | 0.1376 | 1.543 |
| 6 | rs2523589 | 31435313 | 1 | 0.4976 | 0.4918 | 2 | 0.1505 | 0.698 | 1.023 |
| 6 | rs2596438 | 31447850 | 2 | 0.1655 | 0.1786 | 1 | 1.37 | 0.2419 | 0.9117 |
| 6 | rs2844535 | 31458282 | 2 | 0.2932 | 0.2694 | 1 | 3.13 | 0.07686 | 1.125 |
| 6 | rs2844533 | 31458781 | 2 | 0.2232 | 0.2636 | 1 | 9.983 | 0.00158 | 0.8028 |
| 6 | rs1063635 | 31487910 | 1 | 0.4501 | 0.452 | 2 | 0.01573 | 0.9002 | 0.9925 |
| 6 | rs2395029 | 31539759 | 2 | 0.05024 | 0.04642 | 1 | 0.3561 | 0.5507 | 1.087 |
| 6 | rs2857605 | 31632830 | 2 | 0.1888 | 0.1908 | 1 | 0.02942 | 0.8638 | 0.9871 |
| 6 | rs3130071 | 31702607 | 1 | 0.1182 | 0.129 | 2 | 1.209 | 0.2715 | 0.9053 |
| 6 | rs2736157 | 31708799 | 2 | 0.201 | 0.1778 | 1 | 3.929 | 0.04745 | 1.163 |
| 6 | rs805262 | 31736712 | 1 | 0.5239 | 0.4627 | 2 | 16.83 | 4.08E-05 | 1.278 |
| 6 | rs9267532 | 31747958 | 1 | 0.08185 | 0.06715 | 2 | 3.499 | 0.06139 | 1.238 |
| 6 | rs10573 | 31763417 | 1 | 0.08855 | 0.07343 | 2 | 3.427 | 0.06414 | 1.226 |
| 6 | rs915653 | 31765026 | 2 | 0.01174 | 0.01256 | 1 | 0.0632 | 0.8015 | 0.934 |
| 6 | rs805288 | 31786007 | 1 | 0.271 | 0.2861 | 2 | 1.277 | 0.2585 | 0.9276 |
| 6 | rs707915 | 31818947 | 1 | 0.07496 | 0.06763 | 2 | 0.9089 | 0.3404 | 1.117 |
| 6 | rs2763979 | 31902571 | 1 | 0.3555 | 0.3604 | 2 | 0.1175 | 0.7317 | 0.9788 |
| 6 | rs4947332 | 31942176 | 1 | 0.02917 | 0.0314 | 2 | 0.1908 | 0.6623 | 0.9269 |
| 6 | rs9267677 | 32000620 | 2 | 0.136 | 0.117 | 1 | 3.66 | 0.05573 | 1.188 |
| 6 | rs2734335 | 32001923 | 2 | 0.4672 | 0.4976 | 1 | 4.175 | 0.04103 | 0.8852 |
| 6 | rs7746553 | 32003952 | 1 | 0.1669 | 0.1652 | 2 | 0.02402 | 0.8768 | 1.012 |
| 6 | rs4151667 | 32022003 | 1 | 0.02591 | 0.05368 | 2 | 23.4 | 1.31E-06 | 0.469 |
| 6 | rs2072633 | 32027557 | 1 | 0.3967 | 0.425 | 2 | 3.745 | 0.05296 | 0.8894 |
| 6 | rs9501161 | 32032306 | 1 | 0.0834 | 0.08986 | 2 | 0.5947 | 0.4406 | 0.9216 |
| 6 | rs438999 | 32036285 | 2 | 0.04453 | 0.0972 | 1 | 48.9 | 2.70E-12 | 0.4329 |
| 6 | rs2280774 | 32036670 | 1 | 0.3532 | 0.3074 | 2 | 10.65 | 0.001101 | 1.231 |
| 6 | rs419788 | 32036778 | 1 | 0.294 | 0.2686 | 2 | 3.582 | 0.05839 | 1.134 |
| 6 | rs2734331 | 32038330 | 2 | 0.045 | 0.04269 | 1 | 0.1412 | 0.7071 | 1.057 |
| 6 | rs492899 | 32041497 | 2 | 0.08556 | 0.08801 | 1 | 0.08487 | 0.7708 | 0.9696 |
| 6 | rs389884 | 32048876 | 2 | 0.1126 | 0.09952 | 1 | 2.009 | 0.1564 | 1.148 |
| 6 | rs6941112 | 32054593 | 1 | 0.3456 | 0.3092 | 2 | 6.774 | 0.009252 | 1.18 |
| 6 | rs389512 | 32055573 | 1 | 0.08543 | 0.1386 | 2 | 32.67 | 1.10E-08 | 0.5803 |
| 6 | rs7774197 | 32154253 | 2 | 0.05673 | 0.05749 | 1 | 0.01214 | 0.9123 | 0.986 |
| 6 | rs396960 | 32299559 | 1 | 0.2713 | 0.2932 | 2 | 2.692 | 0.1009 | 0.8971 |
| 6 | rs416352 | 32315371 | 1 | 0.3903 | 0.3482 | 2 | 8.557 | 0.003442 | 1.198 |
| 6 | rs424232 | 32316302 | 1 | 0.3381 | 0.299 | 2 | 7.878 | 0.005005 | 1.197 |
| 6 | rs3115572 | 32328462 | 1 | 0.3595 | 0.3879 | 2 | 3.889 | 0.04862 | 0.8857 |
| 6 | rs6910071 | 32390832 | 2 | 0.1989 | 0.1966 | 1 | 0.03844 | 0.8446 | 1.015 |
| 6 | rs2395150 | 32434023 | 2 | 0.3923 | 0.4034 | 1 | 0.5767 | 0.4476 | 0.9548 |
| 6 | rs2273017 | 32445608 | 2 | 0.37 | 0.382 | 1 | 0.6887 | 0.4066 | 0.9502 |
| 6 | rs2050191 | 32446879 | 1 | 0.2138 | 0.2309 | 2 | 1.921 | 0.1657 | 0.9055 |
| 6 | rs3129944 | 32448850 | 1 | 0.3209 | 0.3076 | 2 | 0.9188 | 0.3378 | 1.064 |
| 6 | rs3817964 | 32475975 | 2 | 0.03927 | 0.04651 | 1 | 1.446 | 0.2291 | 0.838 |
| 6 | rs2395173 | 32512837 | 1 | 0.313 | 0.3246 | 2 | 0.7083 | 0.4 | 0.9476 |
| 6 | rs14004 | 32515687 | 1 | 0.4089 | 0.4063 | 2 | 0.03218 | 0.8576 | 1.011 |
| 6 | rs3129883 | 32518115 | 1 | 0.2519 | 0.242 | 2 | 0.5676 | 0.4512 | 1.054 |

FIG. 4.20

| CHR | SNP | BP | A1 | F_A | F_U | A2 | CHISQ | P | OR |
|---|---|---|---|---|---|---|---|---|---|
| 6 | rs3135388 | 32521029 | 1 | 0.1166 | 0.119 | 2 | 0.06019 | 0.8062 | 0.9776 |
| 6 | rs3129891 | 32523058 | 1 | 0.2088 | 0.2045 | 2 | 0.1264 | 0.7222 | 1.027 |
| 6 | rs6923504 | 32536164 | 2 | 0.296 | 0.2945 | 1 | 0.01159 | 0.9143 | 1.007 |
| 6 | rs4434496 | 32538486 | 1 | 0.2538 | 0.2493 | 2 | 0.1249 | 0.7238 | 1.025 |
| 6 | rs6901541 | 32550239 | 2 | 0.2142 | 0.2041 | 1 | 0.6943 | 0.4047 | 1.063 |
| 6 | rs660895 | 32685358 | 2 | 0.1942 | 0.2044 | 1 | 0.7351 | 0.3912 | 0.938 |
| 6 | rs4639334 | 32710192 | 1 | 0.1282 | 0.1223 | 2 | 0.3573 | 0.55 | 1.055 |
| 6 | rs2040410 | 32710676 | 1 | 0.1263 | 0.1141 | 2 | 1.58 | 0.2088 | 1.123 |
| 6 | rs2040406 | 32710985 | 2 | 0.1709 | 0.1429 | 1 | 6.581 | 0.0103 | 1.236 |
| 6 | rs7744001 | 32734064 | 1 | 0.3182 | 0.3353 | 2 | 1.489 | 0.2223 | 0.9254 |
| 6 | rs6457614 | 32759878 | 2 | 0.1279 | 0.1083 | 1 | 4.133 | 0.04207 | 1.208 |
| 6 | rs2856691 | 32761413 | 1 | 0.2247 | 0.2527 | 2 | 4.863 | 0.02744 | 0.8573 |
| 6 | rs1794265 | 32782715 | 1 | 0.0316 | 0.02947 | 2 | 0.173 | 0.6775 | 1.075 |
| 6 | rs2647087 | 32789027 | 2 | 0.2895 | 0.3039 | 1 | 1.119 | 0.2901 | 0.9333 |
| 6 | rs2858333 | 32789063 | 2 | 0.2895 | 0.3039 | 1 | 1.119 | 0.2901 | 0.9333 |
| 6 | rs7454108 | 32789461 | 2 | 0.1012 | 0.1077 | 1 | 0.5122 | 0.4742 | 0.9327 |
| 6 | rs4148871 | 32911294 | 1 | 0.1994 | 0.2205 | 2 | 3.045 | 0.08099 | 0.8802 |
| 6 | rs2071538 | 32926656 | 1 | 0.2245 | 0.236 | 2 | 0.8415 | 0.359 | 0.9371 |
| 6 | rs241398 | 32983428 | 1 | 0.4225 | 0.4155 | 2 | 0.228 | 0.633 | 1.029 |
| 6 | rs2071876 | 33056404 | 1 | 0.09474 | 0.08858 | 2 | 0.5113 | 0.4746 | 1.077 |
| 6 | rs206765 | 33072674 | 2 | 0.372 | 0.3707 | 1 | 0.008224 | 0.9277 | 1.006 |
| 6 | rs417812 | 33082059 | 1 | 0.4122 | 0.415 | 2 | 0.03559 | 0.8504 | 0.9886 |
| 6 | rs399604 | 33082992 | 2 | 0.4028 | 0.4092 | 1 | 0.1927 | 0.6607 | 0.9737 |
| 6 | rs412735 | 33132110 | 1 | 0.4214 | 0.4087 | 2 | 0.7474 | 0.3873 | 1.054 |
| 6 | rs12664430 | 33380655 | 1 | 0.07368 | 0.07633 | 2 | 0.1137 | 0.736 | 0.9626 |
| 6 | rs10807289 | 43817763 | 2 | 0.4765 | 0.4826 | 1 | 0.1694 | 0.6807 | 0.9757 |
| 10 | rs3124740 | 115459615 | 2 | 0.4709 | 0.4797 | 1 | 0.3528 | 0.5526 | 0.9652 |
| 10 | rs10885531 | 115804382 | 1 | 0.4846 | 0.5024 | 2 | 1.428 | 0.2322 | 0.9313 |
| 10 | rs4752255 | 116566780 | 2 | 0.5089 | 0.4802 | 1 | 3.717 | 0.05385 | 1.122 |
| 10 | rs2420098 | 117404353 | 2 | 0.4899 | 0.5014 | 1 | 0.6032 | 0.4374 | 0.9548 |
| 10 | rs4751939 | 117698858 | 2 | 0.5008 | 0.4966 | 1 | 0.07913 | 0.7785 | 1.017 |
| 10 | rs2901101 | 118000140 | 1 | 0.4754 | 0.4775 | 2 | 0.02024 | 0.8869 | 0.9915 |
| 10 | rs1681725 | 118575439 | 2 | 0.5113 | 0.4845 | 1 | 3.235 | 0.07207 | 1.113 |
| 10 | rs2275111 | 120907435 | 2 | 0.4972 | 0.4927 | 1 | 0.08841 | 0.7662 | 1.018 |
| 10 | rs928670 | 121021649 | 2 | 0.4914 | 0.5069 | 1 | 1.066 | 0.3018 | 0.9399 |
| 10 | rs7907952 | 124046764 | 2 | 0.5175 | 0.4512 | 1 | 19.76 | 8.79E-06 | 1.304 |
| 10 | rs7921520 | 126251345 | 2 | 0.4874 | 0.4783 | 1 | 0.3808 | 0.5372 | 1.037 |
| 10 | rs2454055 | 126849822 | 2 | 0.4854 | 0.4942 | 1 | 0.3482 | 0.5551 | 0.9654 |
| 10 | rs7077238 | 129573737 | 1 | 0.485 | 0.4932 | 2 | 0.3043 | 0.5812 | 0.9677 |
| 10 | rs4750779 | 131359782 | 1 | 0.4846 | 0.4497 | 2 | 5.507 | 0.01894 | 1.151 |
| 10 | rs672142 | 131801548 | 2 | 0.483 | 0.4657 | 1 | 1.351 | 0.2451 | 1.072 |
| 10 | rs2767438 | 134466631 | 1 | 0.4841 | 0.4612 | 2 | 2.364 | 0.1241 | 1.096 |
| 16 | rs9939115 | 17128060 | 1 | 0.2866 | 0.271 | 2 | 1.365 | 0.2426 | 1.081 |
| 16 | rs7193870 | 17182928 | 1 | 0.4186 | 0.4188 | 2 | 0.0001934 | 0.9889 | 0.9992 |
| 16 | rs7206838 | 17264313 | 2 | 0.4242 | 0.4425 | 1 | 1.533 | 0.2157 | 0.9282 |
| 16 | rs8046321 | 17321290 | 2 | 0.3858 | 0.411 | 1 | 2.984 | 0.08409 | 0.9002 |
| 16 | rs4781989 | 17356327 | 2 | 0.4425 | 0.4469 | 1 | 0.08812 | 0.7666 | 0.9824 |
| 16 | rs7203097 | 17387063 | 1 | 0.3749 | 0.3696 | 2 | 0.1371 | 0.7112 | 1.023 |
| 16 | rs6498697 | 17387295 | 2 | 0.4818 | 0.4874 | 1 | 0.1444 | 0.704 | 0.9776 |
| 16 | rs17663173 | 18717512 | 1 | 0.07692 | 0.0628 | 2 | 3.428 | 0.06411 | 1.244 |
| 16 | rs3751745 | 18725463 | 2 | 0.08785 | 0.09043 | 1 | 0.09176 | 0.7619 | 0.9688 |
| 16 | rs17731690 | 18769565 | 2 | 0.3907 | 0.3985 | 1 | 0.2841 | 0.594 | 0.968 |
| 16 | rs8063628 | 19145105 | 1 | 0.2231 | 0.2191 | 2 | 0.1058 | 0.745 | 1.024 |
| 16 | rs724307 | 19173482 | 2 | 0.3934 | 0.3728 | 1 | 2.021 | 0.1552 | 1.091 |

FIG. 4.21

| CHR | SNP | BP | A1 | F_A | F_U | A2 | CHISQ | P | OR |
|-----|-----|-----|-----|-----|-----|-----|-------|-----|-----|
| 16 | rs2521795 | 19421642 | 1 | 0.1561 | 0.161 | 2 | 0.2028 | 0.6525 | 0.9639 |
| 16 | rs2074171 | 19429602 | 2 | 0.0668 | 0.07826 | 1 | 2.213 | 0.1369 | 0.8431 |
| 16 | rs226877 | 19489585 | 1 | 0.09198 | 0.09469 | 2 | 0.09772 | 0.7546 | 0.9685 |
| 16 | rs6497385 | 19501552 | 2 | 0.2255 | 0.2336 | 1 | 0.4132 | 0.5203 | 0.9555 |
| 16 | rs957675 | 19587915 | 1 | 0.2918 | 0.2647 | 2 | 3.911 | 0.04796 | 1.145 |
| 16 | rs11644834 | 19629810 | 2 | 0.1768 | 0.1816 | 1 | 0.1792 | 0.672 | 0.9676 |
| 16 | rs4782286 | 19741923 | 1 | 0.2231 | 0.2145 | 2 | 0.4849 | 0.4862 | 1.052 |
| 16 | rs1350380 | 19942497 | 2 | 0.2219 | 0.2179 | 1 | 0.1044 | 0.7467 | 1.024 |
| 16 | rs11865589 | 20286358 | 1 | 0.3818 | 0.3705 | 2 | 0.6071 | 0.4359 | 1.049 |
| 16 | rs4606726 | 20291201 | 2 | 0.4968 | 0.5005 | 1 | 0.0624 | 0.8027 | 0.9852 |
| 16 | rs11646919 | 20308470 | 2 | 0.104 | 0.1141 | 1 | 1.179 | 0.2776 | 0.9015 |
| 16 | rs1845919 | 20542618 | 1 | 0.2185 | 0.1937 | 2 | 4.191 | 0.04064 | 1.163 |
| 16 | rs1013950 | 20645230 | 1 | 0.4503 | 0.4302 | 2 | 1.848 | 0.174 | 1.085 |
| 16 | rs1027457 | 20688695 | 1 | 0.1587 | 0.1348 | 2 | 5.117 | 0.0237 | 1.211 |
| 16 | rs8059938 | 20853437 | 2 | 0.2798 | 0.2795 | 1 | 0.0003777 | 0.9845 | 1.001 |
| 16 | rs3115435 | 20890392 | 2 | 0.4473 | 0.4652 | 1 | 1.453 | 0.228 | 0.9304 |
| 16 | rs858204 | 20978905 | 1 | 0.2 | 0.2029 | 2 | 0.05885 | 0.8083 | 0.9821 |
| 16 | rs16970952 | 21054176 | 2 | 0.238 | 0.2401 | 1 | 0.02624 | 0.8713 | 0.9887 |
| 16 | rs2733911 | 21194200 | 1 | 0.2551 | 0.2522 | 2 | 0.04956 | 0.8238 | 1.015 |
| 16 | rs7201436 | 22142001 | 2 | 0.3166 | 0.3005 | 1 | 1.369 | 0.2419 | 1.078 |
| 16 | rs12102329 | 22803228 | 1 | 0.4874 | 0.5029 | 2 | 1.076 | 0.2995 | 0.94 |
| 16 | rs12446153 | 22996550 | 2 | 0.3277 | 0.314 | 1 | 0.9608 | 0.327 | 1.065 |
| 16 | rs13306654 | 23132018 | 2 | 0.2211 | 0.2128 | 1 | 0.4545 | 0.5002 | 1.05 |
| 16 | rs250578 | 23334611 | 1 | 0.218 | 0.2174 | 2 | 0.002371 | 0.9612 | 1.004 |
| 16 | rs250555 | 23351769 | 2 | 0.1737 | 0.171 | 1 | 0.05624 | 0.8125 | 1.019 |
| 16 | rs531805 | 23373536 | 1 | 0.205 | 0.2086 | 2 | 0.08847 | 0.7661 | 0.9783 |
| 16 | rs8051700 | 23852213 | 2 | 0.4482 | 0.4507 | 1 | 0.02841 | 0.8662 | 0.99 |
| 16 | rs8055831 | 23857686 | 2 | 0.4011 | 0.3908 | 1 | 0.5004 | 0.4793 | 1.044 |
| 16 | rs1490754 | 23922567 | 2 | 0.3874 | 0.3844 | 1 | 0.04072 | 0.8401 | 1.012 |
| 16 | rs403018 | 24059121 | 2 | 0.3271 | 0.3391 | 1 | 0.7312 | 0.3925 | 0.9474 |
| 16 | rs410688 | 24063535 | 2 | 0.3211 | 0.3333 | 1 | 0.7722 | 0.3795 | 0.9457 |
| 16 | rs1126289 | 24080450 | 2 | 0.2069 | 0.213 | 1 | 0.2579 | 0.6115 | 0.9635 |
| 16 | rs17759268 | 24185140 | 2 | 0.2553 | 0.2689 | 1 | 1.081 | 0.2985 | 0.9319 |
| 16 | rs3815906 | 24478258 | 2 | 0.2451 | 0.2589 | 1 | 1.139 | 0.2859 | 0.9294 |
| 16 | rs200531 | 24663673 | 2 | 0.1884 | 0.2062 | 1 | 2.253 | 0.1334 | 0.8937 |
| 16 | rs2059134 | 24723993 | 2 | 0.2603 | 0.2911 | 1 | 5.356 | 0.02065 | 0.8571 |
| 16 | rs274098 | 24819373 | 2 | 0.2413 | 0.2599 | 1 | 2.081 | 0.1491 | 0.9056 |
| 16 | rs7193600 | 24921000 | 2 | 0.3028 | 0.2971 | 1 | 0.1762 | 0.6747 | 1.028 |
| 16 | rs7206132 | 24931339 | 2 | 0.4024 | 0.3888 | 1 | 0.866 | 0.3521 | 1.058 |
| 16 | rs277892 | 25088400 | 2 | 0.2249 | 0.2253 | 1 | 0.001365 | 0.9705 | 0.9974 |
| 16 | rs4310858 | 25669011 | 2 | 0.08833 | 0.0913 | 1 | 0.1219 | 0.7269 | 0.9643 |
| 16 | rs7197707 | 25682282 | 1 | 0.4289 | 0.4228 | 2 | 0.1709 | 0.6793 | 1.025 |
| 16 | rs7195333 | 25694235 | 1 | 0.2611 | 0.2602 | 2 | 0.005596 | 0.9404 | 1.005 |
| 16 | rs10163260 | 25770009 | 2 | 0.2957 | 0.3116 | 1 | 1.346 | 0.2459 | 0.9276 |
| 16 | rs6497898 | 25785018 | 1 | 0.1285 | 0.1234 | 2 | 0.2674 | 0.6051 | 1.048 |
| 16 | rs12926546 | 25931384 | 2 | 0.3529 | 0.3633 | 1 | 0.5267 | 0.468 | 0.9559 |
| 16 | rs7201424 | 26010080 | 2 | 0.4968 | 0.4879 | 1 | 0.3506 | 0.5538 | 1.036 |
| 16 | rs3024570 | 27265285 | 1 | 0.08057 | 0.08567 | 2 | 0.3858 | 0.5345 | 0.9352 |
| 16 | rs6498026 | 27339182 | 2 | 0.2962 | 0.2966 | 1 | 0.0009848 | 0.975 | 0.998 |
| 16 | rs8050273 | 27488590 | 1 | 0.1563 | 0.1731 | 2 | 2.328 | 0.127 | 0.8847 |
| 16 | rs734652 | 27622087 | 1 | 0.1545 | 0.1705 | 2 | 2.132 | 0.1443 | 0.8888 |
| 16 | rs933661 | 27760315 | 2 | 0.3316 | 0.3179 | 1 | 0.9637 | 0.3263 | 1.064 |
| 16 | rs715337 | 28082909 | 1 | 0.3165 | 0.3085 | 2 | 0.33 | 0.5657 | 1.038 |
| 16 | rs7193402 | 28493628 | 2 | 0.3202 | 0.3599 | 1 | 7.913 | 0.004908 | 0.8379 |

FIG. 4.22

| CHR | SNP | BP | A1 | F_A | F_U | A2 | CHISQ | P | OR |
|-----|-----|-----|-----|-----|-----|-----|-----|-----|-----|
| 16 | rs1057451 | 29740989 | 1 | 0.1479 | 0.1367 | 2 | 1.149 | 0.2837 | 1.096 |
| 16 | rs4787486 | 29885063 | 1 | 0.4538 | 0.4526 | 2 | 0.006927 | 0.9337 | 1.005 |
| 16 | rs4465620 | 30339604 | 2 | 0.3652 | 0.3614 | 1 | 0.07139 | 0.7893 | 1.017 |
| 16 | rs1046276 | 30822127 | 1 | 0.3405 | 0.3483 | 2 | 0.3054 | 0.5805 | 0.9659 |
| 16 | rs2305884 | 30878242 | 2 | 0.3602 | 0.3667 | 1 | 0.2029 | 0.6524 | 0.9725 |
| 16 | rs4889606 | 30918684 | 2 | 0.4028 | 0.3923 | 1 | 0.5165 | 0.4723 | 1.045 |
| 16 | rs741810 | 31101443 | 1 | 0.2805 | 0.2976 | 2 | 1.575 | 0.2095 | 0.92 |
| 16 | rs4889640 | 31171768 | 2 | 0.302 | 0.3114 | 1 | 0.4669 | 0.4944 | 0.9568 |
| 16 | rs9937837 | 31206440 | 2 | 0.2786 | 0.2773 | 1 | 0.008629 | 0.926 | 1.006 |
| 16 | rs12928810 | 31219216 | 1 | 0.1232 | 0.1295 | 2 | 0.4046 | 0.5247 | 0.9446 |
| 16 | rs11645653 | 31220356 | 2 | 0.2431 | 0.2534 | 1 | 0.6392 | 0.424 | 0.9463 |
| 16 | rs7193268 | 31248498 | 1 | 0.1667 | 0.1614 | 2 | 0.2315 | 0.6304 | 1.04 |
| 16 | rs4597342 | 31251270 | 1 | 0.2934 | 0.2952 | 2 | 0.01647 | 0.8979 | 0.9916 |
| 16 | rs3925075 | 31255249 | 1 | 0.4615 | 0.4585 | 2 | 0.04225 | 0.8372 | 1.012 |
| 16 | rs4075052 | 31255734 | 1 | 0.2938 | 0.2952 | 2 | 0.01076 | 0.9174 | 0.9932 |
| 16 | rs11861974 | 31672721 | 1 | 0.07206 | 0.06812 | 2 | 0.2688 | 0.6041 | 1.062 |
| 16 | rs12921028 | 31678224 | 1 | 0.3088 | 0.2974 | 2 | 0.6872 | 0.4071 | 1.055 |
| 16 | rs808783 | 45405529 | 2 | 0.1478 | 0.1401 | 1 | 0.5376 | 0.4634 | 1.064 |
| 16 | rs4967621 | 45499113 | 1 | 0.1244 | 0.1074 | 2 | 3.172 | 0.07491 | 1.181 |
| 16 | rs2341648 | 45542876 | 2 | 0.05835 | 0.05459 | 1 | 0.2975 | 0.5855 | 1.073 |
| 16 | rs16952126 | 45673685 | 2 | 0.04858 | 0.02947 | 1 | 10.75 | 0.001043 | 1.682 |
| 16 | rs13331250 | 46872339 | 1 | 0.1599 | 0.1628 | 2 | 0.06921 | 0.7925 | 0.9789 |
| 16 | rs11076564 | 46937666 | 1 | 0.09393 | 0.08357 | 2 | 1.485 | 0.2229 | 1.137 |
| 16 | rs989070 | 47159744 | 1 | 0.3 | 0.3101 | 2 | 0.5472 | 0.4595 | 0.9533 |
| 16 | rs2278026 | 47990325 | 1 | 0.1491 | 0.1382 | 2 | 1.093 | 0.2958 | 1.093 |
| 16 | rs12931260 | 48098403 | 2 | 0.3478 | 0.3662 | 1 | 1.665 | 0.197 | 0.9229 |
| 16 | rs7404830 | 48353167 | 2 | 0.3825 | 0.3912 | 1 | 0.3595 | 0.5488 | 0.964 |
| 16 | rs8059133 | 48928204 | 1 | 0.1818 | 0.1813 | 2 | 0.001512 | 0.969 | 1.003 |
| 16 | rs12924696 | 49229222 | 2 | 0.2956 | 0.3188 | 1 | 2.856 | 0.09103 | 0.8966 |
| 16 | rs9933594 | 49281588 | 2 | 0.2567 | 0.2676 | 1 | 0.6993 | 0.403 | 0.9449 |
| 16 | rs3785142 | 49344648 | 2 | 0.4672 | 0.4995 | 1 | 4.712 | 0.02995 | 0.8785 |
| 16 | rs17314544 | 49377579 | 1 | 0.4408 | 0.41 | 2 | 4.381 | 0.03634 | 1.135 |
| 16 | rs11075782 | 51789460 | 1 | 0.3287 | 0.295 | 2 | 5.957 | 0.01466 | 1.171 |
| 16 | rs12927940 | 51893627 | 2 | 0.1212 | 0.1306 | 1 | 0.9082 | 0.3406 | 0.918 |
| 16 | rs16952168 | 51909830 | 2 | 0.3506 | 0.3262 | 1 | 2.98 | 0.08428 | 1.115 |
| 16 | rs7191257 | 51920802 | 2 | 0.4744 | 0.4584 | 1 | 1.159 | 0.2817 | 1.066 |
| 16 | rs13332406 | 52047206 | 1 | 0.4919 | 0.47 | 2 | 2.152 | 0.1424 | 1.091 |
| 16 | rs31070 | 53911016 | 1 | 0.2259 | 0.2198 | 2 | 0.2421 | 0.6227 | 1.036 |
| 16 | rs31082 | 53918994 | 1 | 0.3421 | 0.3377 | 2 | 0.09822 | 0.754 | 1.02 |
| 16 | rs12930259 | 54101379 | 1 | 0.4501 | 0.4498 | 2 | 0.0005998 | 0.9805 | 1.001 |
| 16 | rs6499766 | 54161629 | 2 | 0.5024 | 0.4763 | 1 | 3.069 | 0.07982 | 1.11 |
| 16 | rs1393258 | 54165533 | 2 | 0.5057 | 0.4763 | 1 | 3.882 | 0.0488 | 1.125 |
| 16 | rs879522 | 54281953 | 1 | 0.4907 | 0.5048 | 2 | 0.9018 | 0.3423 | 0.9449 |
| 16 | rs929872 | 54822906 | 1 | 0.438 | 0.4226 | 2 | 1.087 | 0.2971 | 1.065 |
| 16 | rs4783932 | 54832002 | 1 | 0.4518 | 0.4459 | 2 | 0.16 | 0.6892 | 1.024 |
| 16 | rs1478468 | 54847933 | 1 | 0.06761 | 0.07826 | 2 | 1.9 | 0.1681 | 0.8541 |
| 16 | rs11647319 | 54916989 | 1 | 0.1395 | 0.1511 | 2 | 1.214 | 0.2706 | 0.9107 |
| 16 | rs869205 | 54951536 | 1 | 0.4428 | 0.4526 | 2 | 0.4399 | 0.5072 | 0.961 |
| 16 | rs2161738 | 55099841 | 1 | 0.1389 | 0.142 | 2 | 0.09838 | 0.7599 | 0.9741 |
| 16 | rs11642892 | 55323187 | 1 | 0.291 | 0.2565 | 2 | 6.7 | 0.009643 | 1.19 |
| 16 | rs2895432 | 55414456 | 1 | 0.07895 | 0.1034 | 2 | 8.202 | 0.004183 | 0.7434 |
| 16 | rs735144 | 55441425 | 2 | 0.4481 | 0.4136 | 1 | 5.419 | 0.01992 | 1.151 |
| 16 | rs1865832 | 55442166 | 2 | 0.1665 | 0.1749 | 1 | 0.5551 | 0.4562 | 0.9427 |
| 16 | rs12921781 | 55453690 | 2 | 0.2769 | 0.2674 | 1 | 0.5139 | 0.4734 | 1.049 |

FIG. 4.23

| CHR | SNP | BP | A1 | F_A | F_U | A2 | CHISQ | P | OR |
|---|---|---|---|---|---|---|---|---|---|
| 16 | rs2289118 | 55486176 | 1 | 0.1611 | 0.1775 | 2 | 2.142 | 0.1433 | 0.8903 |
| 16 | rs11643718 | 55491020 | 1 | 0.1202 | 0.09275 | 2 | 8.855 | 0.002923 | 1.337 |
| 16 | rs12449275 | 55495356 | 1 | 0.247 | 0.2227 | 2 | 3.677 | 0.05518 | 1.145 |
| 16 | rs2289114 | 55496023 | 1 | 0.1798 | 0.1978 | 2 | 2.394 | 0.1218 | 0.8889 |
| 16 | rs3794648 | 55504047 | 1 | 0.4543 | 0.4534 | 2 | 0.003557 | 0.9524 | 1.004 |
| 16 | rs598486 | 56069126 | 2 | 0.2522 | 0.2519 | 1 | 0.0004972 | 0.9822 | 1.002 |
| 16 | rs8060347 | 56145135 | 2 | 0.4194 | 0.415 | 1 | 0.09201 | 0.7616 | 1.019 |
| 16 | rs6499906 | 56229858 | 2 | 0.4684 | 0.456 | 1 | 0.7002 | 0.4027 | 1.051 |
| 16 | rs2965790 | 56362579 | 2 | 0.2885 | 0.3129 | 1 | 3.185 | 0.07434 | 0.8905 |
| 16 | rs8061387 | 56390004 | 2 | 0.07206 | 0.09003 | 1 | 4.919 | 0.02656 | 0.785 |
| 16 | rs2242444 | 56757171 | 1 | 0.1661 | 0.1749 | 2 | 0.6128 | 0.4338 | 0.9398 |
| 16 | rs12708998 | 56860083 | 2 | 0.4243 | 0.4266 | 1 | 0.02392 | 0.8771 | 0.9907 |
| 16 | rs4784916 | 56877712 | 1 | 0.3063 | 0.3023 | 2 | 0.08472 | 0.771 | 1.019 |
| 16 | rs2280397 | 57109729 | 1 | 0.2877 | 0.2903 | 2 | 0.03865 | 0.8441 | 0.9872 |
| 16 | rs11649173 | 57181021 | 1 | 0.4537 | 0.4691 | 2 | 1.062 | 0.3028 | 0.9402 |
| 16 | rs8058151 | 57272444 | 2 | 0.1065 | 0.1179 | 1 | 1.474 | 0.2246 | 0.8918 |
| 16 | rs6993 | 57298868 | 1 | 0.3567 | 0.357 | 2 | 0.0005173 | 0.9819 | 0.9986 |
| 16 | rs11863254 | 60259576 | 1 | 0.1134 | 0.119 | 2 | 0.3439 | 0.5576 | 0.947 |
| 16 | rs4372683 | 60284864 | 2 | 0.2622 | 0.2473 | 1 | 1.299 | 0.2544 | 1.081 |
| 16 | rs11643658 | 60297271 | 2 | 0.1349 | 0.1489 | 1 | 1.82 | 0.1773 | 0.8913 |
| 16 | rs17822852 | 60406069 | 1 | 0.1896 | 0.1804 | 2 | 0.6388 | 0.4242 | 1.063 |
| 16 | rs1865808 | 60407794 | 1 | 0.4206 | 0.4101 | 2 | 0.5132 | 0.4738 | 1.044 |
| 16 | rs1978796 | 60447405 | 2 | 0.4146 | 0.4059 | 1 | 0.3507 | 0.5537 | 1.037 |
| 16 | rs9635515 | 60460224 | 2 | 0.2585 | 0.2565 | 1 | 0.02293 | 0.8796 | 1.01 |
| 16 | rs1355264 | 60490181 | 1 | 0.1623 | 0.1522 | 2 | 0.8776 | 0.3489 | 1.08 |
| 16 | rs9933599 | 60536894 | 2 | 0.368 | 0.3657 | 1 | 0.026 | 0.8719 | 1.01 |
| 16 | rs12446244 | 60632785 | 1 | 0.4236 | 0.4411 | 2 | 1.392 | 0.2381 | 0.9315 |
| 16 | rs35154 | 63535603 | 1 | 0.2707 | 0.286 | 2 | 1.318 | 0.2509 | 0.9265 |
| 16 | rs35232 | 63559853 | 1 | 0.1925 | 0.1788 | 2 | 1.389 | 0.2386 | 1.095 |
| 16 | rs2331446 | 63569897 | 1 | 0.3433 | 0.3435 | 2 | 0.0001254 | 0.9911 | 0.9993 |
| 16 | rs1520243 | 63604836 | 2 | 0.4598 | 0.4647 | 1 | 0.1092 | 0.741 | 0.9804 |
| 16 | rs7500503 | 63627189 | 1 | 0.08623 | 0.09952 | 2 | 2.372 | 0.1236 | 0.8539 |
| 16 | rs16968461 | 63684053 | 2 | 0.3246 | 0.3324 | 1 | 0.3117 | 0.5766 | 0.9652 |
| 16 | rs1520236 | 63685798 | 1 | 0.3995 | 0.3746 | 2 | 2.93 | 0.08693 | 1.111 |
| 16 | rs355971 | 65228152 | 1 | 0.06154 | 0.06618 | 2 | 0.4077 | 0.5231 | 0.9252 |
| 16 | rs12600097 | 65315704 | 2 | 0.4189 | 0.4101 | 1 | 0.362 | 0.5474 | 1.037 |
| 16 | rs363184 | 65360598 | 1 | 0.06478 | 0.06957 | 2 | 0.4132 | 0.5203 | 0.9264 |
| 16 | rs363172 | 65402525 | 1 | 0.3632 | 0.3536 | 2 | 0.445 | 0.5047 | 1.042 |
| 16 | rs7193713 | 65811920 | 2 | 0.06478 | 0.0697 | 1 | 0.436 | 0.5091 | 0.9245 |
| 16 | rs8051587 | 66353816 | 2 | 0.1741 | 0.1729 | 1 | 0.01024 | 0.9194 | 1.008 |
| 16 | rs7192187 | 66689610 | 2 | 0.1456 | 0.1348 | 1 | 1.085 | 0.2975 | 1.094 |
| 16 | rs2290699 | 66846560 | 1 | 0.1713 | 0.1776 | 2 | 0.3036 | 0.5816 | 0.9574 |
| 16 | rs16957831 | 66935842 | 1 | 0.1175 | 0.1135 | 2 | 0.1741 | 0.6765 | 1.04 |
| 16 | rs2279539 | 66952595 | 2 | 0.4623 | 0.4743 | 1 | 0.6536 | 0.4188 | 0.9528 |
| 16 | rs1465468 | 66955791 | 1 | 0.2936 | 0.3008 | 2 | 0.2779 | 0.5981 | 0.9662 |
| 16 | rs9937560 | 66992526 | 2 | 0.2235 | 0.2382 | 1 | 1.37 | 0.2418 | 0.9206 |
| 16 | rs9940250 | 67324314 | 2 | 0.1219 | 0.1014 | 1 | 4.698 | 0.0302 | 1.229 |
| 16 | rs7203337 | 67332301 | 2 | 0.4219 | 0.4159 | 1 | 0.1621 | 0.6872 | 1.025 |
| 16 | rs12930371 | 67360437 | 1 | 0.2873 | 0.2926 | 2 | 0.1486 | 0.6999 | 0.975 |
| 16 | rs12919719 | 67379842 | 1 | 0.2897 | 0.2959 | 2 | 0.211 | 0.646 | 0.9704 |
| 16 | rs10500548 | 67731642 | 1 | 0.06721 | 0.04879 | 2 | 6.899 | 0.008624 | 1.405 |
| 16 | rs9938937 | 67795665 | 1 | 0.1522 | 0.1465 | 2 | 0.2883 | 0.5913 | 1.046 |
| 16 | rs7192863 | 67816005 | 2 | 0.3142 | 0.2868 | 1 | 4.017 | 0.04504 | 1.139 |
| 16 | rs8047297 | 68016503 | 2 | 0.1818 | 0.1852 | 1 | 0.08603 | 0.7693 | 0.9777 |

FIG. 4.24

| CHR | SNP | BP | A1 | F_A | F_U | A2 | CHISQ | P | OR |
|-----|-----|-----|-----|-----|-----|-----|-------|---|-----|
| 16 | rs11075721 | 68055311 | 1 | 0.4327 | 0.4527 | 2 | 1.811 | 0.1784 | 0.9224 |
| 16 | rs7187634 | 68220872 | 2 | 0.4364 | 0.4335 | 1 | 0.03787 | 0.8457 | 1.012 |
| 16 | rs9924953 | 68262622 | 2 | 0.1709 | 0.1512 | 1 | 3.202 | 0.07353 | 1.157 |
| 16 | rs11641233 | 68292064 | 1 | 0.1844 | 0.1942 | 2 | 0.712 | 0.3988 | 0.9379 |
| 16 | rs4985418 | 68394966 | 1 | 0.3524 | 0.371 | 2 | 1.692 | 0.1933 | 0.9225 |
| 16 | rs2088747 | 68425844 | 1 | 0.1389 | 0.1169 | 2 | 4.837 | 0.02785 | 1.218 |
| 16 | rs9940315 | 68433665 | 1 | 0.429 | 0.4224 | 2 | 0.2018 | 0.6532 | 1.027 |
| 16 | rs7204326 | 68460682 | 2 | 0.07901 | 0.08607 | 1 | 0.7436 | 0.3885 | 0.9109 |
| 16 | rs5014388 | 68615369 | 2 | 0.2184 | 0.2174 | 1 | 0.00666 | 0.935 | 1.006 |
| 16 | rs8063990 | 69084446 | 1 | 0.4278 | 0.4372 | 2 | 0.4035 | 0.5253 | 0.9625 |
| 16 | rs16970408 | 69227089 | 1 | 0.3598 | 0.3483 | 2 | 0.6502 | 0.4201 | 1.052 |
| 16 | rs2902830 | 69334083 | 2 | 0.1782 | 0.168 | 1 | 0.8166 | 0.3662 | 1.074 |
| 16 | rs6416703 | 69440839 | 1 | 0.09651 | 0.1042 | 2 | 0.7314 | 0.3924 | 0.9187 |
| 16 | rs1424137 | 69783066 | 2 | 0.3262 | 0.328 | 1 | 0.0174 | 0.8951 | 0.9917 |
| 16 | rs3096381 | 69875502 | 1 | 0.1749 | 0.1681 | 2 | 0.3641 | 0.5462 | 1.049 |
| 16 | rs16970994 | 69887620 | 2 | 0.1312 | 0.1261 | 1 | 0.2596 | 0.6104 | 1.046 |
| 16 | rs6499500 | 69905916 | 2 | 0.4347 | 0.4438 | 1 | 0.381 | 0.5371 | 0.9636 |
| 16 | rs12922830 | 70004771 | 1 | 0.1652 | 0.1614 | 2 | 0.1208 | 0.7282 | 1.028 |
| 16 | rs7202662 | 70102989 | 1 | 0.2931 | 0.2928 | 2 | 0.0007195 | 0.9786 | 1.002 |
| 16 | rs9926110 | 70123083 | 1 | 0.3128 | 0.323 | 2 | 0.5417 | 0.4617 | 0.954 |
| 16 | rs7203606 | 70148012 | 2 | 0.1619 | 0.1456 | 1 | 2.316 | 0.1281 | 1.134 |
| 16 | rs4788548 | 70212862 | 1 | 0.1609 | 0.1652 | 2 | 0.1569 | 0.6921 | 0.9686 |
| 16 | rs4788828 | 70281591 | 2 | 0.1619 | 0.1662 | 1 | 0.1478 | 0.7007 | 0.9696 |
| 16 | rs951939 | 70367035 | 1 | 0.3275 | 0.3395 | 2 | 0.7211 | 0.3958 | 0.9477 |
| 16 | rs8050651 | 70438945 | 1 | 0.2565 | 0.2512 | 2 | 0.1653 | 0.6843 | 1.028 |
| 16 | rs11075910 | 70494234 | 2 | 0.4672 | 0.4511 | 1 | 1.173 | 0.2788 | 1.067 |
| 16 | rs7188591 | 70510523 | 1 | 0.2777 | 0.2964 | 2 | 1.924 | 0.1654 | 0.9127 |
| 16 | rs1559399 | 70568762 | 1 | 0.2211 | 0.2253 | 2 | 0.1193 | 0.7298 | 0.9756 |
| 16 | rs7203426 | 70644056 | 1 | 0.4551 | 0.4579 | 2 | 0.03736 | 0.8467 | 0.9885 |
| 16 | rs9928157 | 70674574 | 1 | 0.4312 | 0.4348 | 2 | 0.05974 | 0.8069 | 0.9854 |
| 16 | rs3852781 | 70681057 | 2 | 0.4165 | 0.4101 | 1 | 0.1853 | 0.6669 | 1.026 |
| 16 | rs12325142 | 70695613 | 1 | 0.3709 | 0.3582 | 2 | 0.7791 | 0.3774 | 1.056 |
| 16 | rs12449073 | 70728505 | 1 | 0.4545 | 0.4361 | 2 | 1.546 | 0.2137 | 1.078 |
| 16 | rs17604676 | 70779485 | 1 | 0.09757 | 0.103 | 2 | 0.3682 | 0.544 | 0.9416 |
| 16 | rs11646048 | 70849233 | 2 | 0.1791 | 0.182 | 1 | 0.06404 | 0.8002 | 0.9806 |
| 16 | rs9936793 | 70931099 | 2 | 0.1814 | 0.1838 | 1 | 0.0426 | 0.8365 | 0.9842 |
| 16 | rs4788632 | 70955141 | 1 | 0.1271 | 0.1362 | 2 | 0.8189 | 0.3655 | 0.9234 |
| 16 | rs16970908 | 71010480 | 2 | 0.1745 | 0.1739 | 1 | 0.002641 | 0.959 | 1.004 |
| 16 | rs17677616 | 71094539 | 2 | 0.1182 | 0.1213 | 1 | 0.09867 | 0.7534 | 0.9716 |
| 16 | rs11075944 | 71298954 | 2 | 0.06042 | 0.07005 | 1 | 1.721 | 0.1896 | 0.8537 |
| 16 | rs12708935 | 71516528 | 1 | 0.2858 | 0.3106 | 2 | 3.316 | 0.06861 | 0.8882 |
| 16 | rs16971426 | 71533680 | 1 | 0.06929 | 0.05942 | 2 | 1.809 | 0.1787 | 1.178 |
| 16 | rs6420387 | 71860714 | 1 | 0.3822 | 0.3854 | 2 | 0.05019 | 0.8227 | 0.9863 |
| 16 | rs8063705 | 71963007 | 1 | 0.2927 | 0.2879 | 2 | 0.1223 | 0.7265 | 1.023 |
| 16 | rs8060956 | 71983248 | 2 | 0.3279 | 0.3174 | 1 | 0.5724 | 0.4493 | 1.049 |
| 16 | rs8048857 | 72262552 | 2 | 0.313 | 0.3063 | 1 | 0.2346 | 0.6281 | 1.032 |
| 16 | rs12919733 | 72266804 | 2 | 0.1186 | 0.1024 | 1 | 2.993 | 0.08365 | 1.18 |
| 16 | rs328334 | 72341985 | 2 | 0.0417 | 0.03478 | 1 | 1.454 | 0.2278 | 1.208 |
| 16 | rs2729613 | 72446098 | 1 | 0.4457 | 0.4734 | 2 | 3.476 | 0.06227 | 0.8945 |
| 16 | rs11150242 | 72528122 | 2 | 0.3526 | 0.3454 | 1 | 0.2583 | 0.6113 | 1.032 |
| 16 | rs2526045 | 72646986 | 2 | 0.1799 | 0.1929 | 1 | 1.264 | 0.2609 | 0.9176 |
| 16 | rs886502 | 72668873 | 1 | 0.1486 | 0.1488 | 2 | 0.0003897 | 0.9843 | 0.9983 |
| 16 | rs12917948 | 72715273 | 1 | 0.05146 | 0.05314 | 2 | 0.0643 | 0.7998 | 0.9666 |
| 16 | rs7200699 | 72763563 | 2 | 0.4785 | 0.4845 | 1 | 0.1632 | 0.6862 | 0.9762 |

FIG. 4.25

| CHR | SNP | BP | A1 | F_A | F_U | A2 | CHISQ | P | OR |
|---|---|---|---|---|---|---|---|---|---|
| 16 | rs10500583 | 72847378 | 2 | 0.1478 | 0.1456 | 1 | 0.04438 | 0.8331 | 1.018 |
| 16 | rs17336700 | 72892705 | 2 | 0.1381 | 0.1338 | 1 | 0.1722 | 0.6782 | 1.037 |
| 16 | rs2303281 | 73074656 | 1 | 0.3279 | 0.3353 | 2 | 0.2732 | 0.6012 | 0.9675 |
| 16 | rs7185026 | 73158768 | 2 | 0.3344 | 0.3502 | 1 | 1.254 | 0.2627 | 0.9321 |
| 16 | rs4887776 | 73193133 | 1 | 0.3595 | 0.3758 | 2 | 1.293 | 0.2555 | 0.9322 |
| 16 | rs3169335 | 73659291 | 2 | 0.1394 | 0.1301 | 1 | 0.8338 | 0.3612 | 1.083 |
| 16 | rs12599058 | 73694387 | 2 | 0.07166 | 0.07302 | 1 | 0.03093 | 0.8604 | 0.98 |
| 16 | rs11642394 | 73748994 | 1 | 0.1356 | 0.13 | 2 | 0.3146 | 0.5749 | 1.051 |
| 16 | rs11644063 | 73788800 | 2 | 0.2028 | 0.1905 | 1 | 1.078 | 0.2992 | 1.081 |
| 16 | rs4737 | 73795604 | 2 | 0.2085 | 0.1971 | 1 | 0.9037 | 0.3418 | 1.073 |
| 16 | rs17674216 | 73890307 | 2 | 0.1841 | 0.1891 | 1 | 0.1842 | 0.6678 | 0.9677 |
| 16 | rs8046109 | 73917597 | 1 | 0.3971 | 0.429 | 2 | 4.731 | 0.02962 | 0.8767 |
| 16 | rs3851735 | 73929593 | 1 | 0.1271 | 0.1527 | 2 | 6.143 | 0.0132 | 0.8084 |
| 16 | rs8048816 | 73979702 | 2 | 0.1837 | 0.211 | 1 | 5.238 | 0.0221 | 0.8415 |
| 16 | rs37590 | 74027136 | 2 | 0.06275 | 0.06763 | 1 | 0.4414 | 0.5064 | 0.923 |
| 16 | rs4149504 | 74123346 | 1 | 0.1937 | 0.179 | 2 | 1.594 | 0.2067 | 1.102 |
| 16 | rs2550913 | 74123397 | 1 | 0.2859 | 0.2633 | 2 | 2.88 | 0.08969 | 1.12 |
| 16 | rs2195430 | 74913417 | 1 | 0.3344 | 0.357 | 2 | 2.545 | 0.1107 | 0.9049 |
| 16 | rs12596963 | 74943286 | 1 | 0.1 | 0.1043 | 2 | 0.2325 | 0.6297 | 0.9537 |
| 16 | rs17766957 | 74968826 | 1 | 0.1309 | 0.1326 | 2 | 0.03006 | 0.8624 | 0.9848 |
| 16 | rs13336784 | 75021909 | 1 | 0.1101 | 0.1113 | 2 | 0.01659 | 0.8975 | 0.9878 |
| 16 | rs1506822 | 75023881 | 2 | 0.4247 | 0.4372 | 1 | 0.718 | 0.3968 | 0.9503 |
| 16 | rs1506819 | 75029600 | 1 | 0.4296 | 0.4097 | 2 | 1.829 | 0.1763 | 1.085 |
| 16 | rs1002975 | 75059110 | 2 | 0.4732 | 0.4807 | 1 | 0.2497 | 0.6173 | 0.9706 |
| 16 | rs11643145 | 75092843 | 1 | 0.3067 | 0.3039 | 2 | 0.04345 | 0.8349 | 1.014 |
| 16 | rs6564350 | 75139103 | 1 | 0.2773 | 0.2756 | 2 | 0.01625 | 0.8986 | 1.009 |
| 16 | rs17687224 | 75894521 | 2 | 0.13 | 0.1291 | 1 | 0.007198 | 0.9324 | 1.008 |
| 16 | rs17769937 | 75899478 | 1 | 0.1223 | 0.1213 | 2 | 0.01076 | 0.9174 | 1.01 |
| 16 | rs937584 | 75952706 | 1 | 0.4136 | 0.4024 | 2 | 0.5852 | 0.4443 | 1.048 |
| 16 | rs7197341 | 75958463 | 2 | 0.2174 | 0.2116 | 1 | 0.2258 | 0.6347 | 1.035 |
| 16 | rs12925015 | 75995134 | 2 | 0.1614 | 0.1557 | 1 | 0.2725 | 0.6017 | 1.044 |
| 16 | rs4888627 | 75997406 | 2 | 0.2648 | 0.272 | 1 | 0.2978 | 0.5852 | 0.964 |
| 16 | rs12934271 | 76011693 | 2 | 0.3117 | 0.3106 | 1 | 0.006506 | 0.9357 | 1.005 |
| 16 | rs9921266 | 76467066 | 1 | 0.2765 | 0.3022 | 2 | 3.626 | 0.0569 | 0.8824 |
| 16 | rs2344920 | 76480275 | 1 | 0.3838 | 0.3821 | 2 | 0.01345 | 0.9077 | 1.007 |
| 16 | rs591415 | 76558299 | 1 | 0.2696 | 0.2725 | 2 | 0.0456 | 0.8309 | 0.9858 |
| 16 | rs2735401 | 76614604 | 2 | 0.2538 | 0.2807 | 1 | 4.151 | 0.0416 | 0.8719 |
| 16 | rs7500694 | 76626911 | 1 | 0.3761 | 0.3585 | 2 | 1.51 | 0.2191 | 1.079 |
| 16 | rs3764299 | 76738266 | 2 | 0.2017 | 0.1986 | 1 | 0.06986 | 0.7915 | 1.02 |
| 16 | rs2303190 | 76755737 | 1 | 0.1704 | 0.1575 | 2 | 1.375 | 0.2409 | 1.099 |
| 16 | rs4888776 | 76882512 | 1 | 0.4271 | 0.4242 | 2 | 0.03904 | 0.8434 | 1.012 |
| 16 | rs11639904 | 76964024 | 2 | 0.3808 | 0.3733 | 1 | 0.2672 | 0.6052 | 1.032 |
| 16 | rs2667657 | 77054377 | 1 | 0.1588 | 0.1605 | 2 | 0.02449 | 0.8757 | 0.9873 |
| 16 | rs8051225 | 77069814 | 2 | 0.2547 | 0.2469 | 1 | 0.364 | 0.5463 | 1.042 |
| 16 | rs2859636 | 77085653 | 2 | 0.1587 | 0.1527 | 1 | 0.3129 | 0.5759 | 1.047 |
| 16 | rs11150082 | 77094198 | 1 | 0.2223 | 0.2191 | 2 | 0.06762 | 0.7948 | 1.019 |
| 16 | rs1877285 | 77105299 | 2 | 0.3142 | 0.3184 | 1 | 0.09135 | 0.7625 | 0.9808 |
| 16 | rs1124434 | 77238834 | 2 | 0.4571 | 0.4401 | 1 | 1.314 | 0.2517 | 1.071 |
| 16 | rs1072898 | 77372481 | 1 | 0.3181 | 0.3191 | 2 | 0.006019 | 0.9382 | 0.995 |
| 16 | rs6564595 | 77399849 | 1 | 0.1623 | 0.1496 | 2 | 1.393 | 0.238 | 1.102 |
| 16 | rs6564604 | 77421147 | 1 | 0.1718 | 0.1614 | 2 | 0.883 | 0.3474 | 1.078 |
| 16 | rs1079636 | 77467963 | 2 | 0.2874 | 0.2986 | 1 | 0.6707 | 0.4128 | 0.9478 |
| 16 | rs10451158 | 77480860 | 1 | 0.4441 | 0.4477 | 2 | 0.06034 | 0.806 | 0.9854 |
| 16 | rs13333229 | 77533552 | 1 | 0.1089 | 0.1193 | 2 | 1.213 | 0.2707 | 0.902 |

FIG. 4.26

| CHR | SNP | BP | A1 | F_A | F_U | A2 | CHISQ | P | OR |
|---|---|---|---|---|---|---|---|---|---|
| 16 | rs2656633 | 77628989 | 1 | 0.104 | 0.09469 | 2 | 1.099 | 0.2945 | 1.11 |
| 16 | rs8053895 | 77741961 | 2 | 0.2619 | 0.2585 | 1 | 0.0712 | 0.7896 | 1.018 |
| 16 | rs9972791 | 77757091 | 2 | 0.4465 | 0.4415 | 1 | 0.1106 | 0.7394 | 1.02 |
| 16 | rs6564653 | 77764739 | 1 | 0.4474 | 0.4477 | 2 | 0.0005788 | 0.9808 | 0.9986 |
| 19 | rs10424046 | 50227876 | 2 | 0.4826 | 0.4956 | 1 | 0.7694 | 0.3804 | 0.949 |

FIG. 4.27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5449754 A
- US 6180351 B
- WO 9841531 A
- US 6242266 B
- US 6232072 B
- US 6171797 B
- EP 0799897 A
- WO 9729212 A
- WO 9727317 A
- EP 0785280 A
- WO 9702357 A
- US 5593839 A
- US 5578832 A
- EP 0728520 A
- US 5599695 A
- EP 0721016 A
- US 5556752 A
- WO 9522058 A
- US 5631734 A
- US 7206438 B

### Non-patent literature cited in the description

- LI, M. et al. *Nat. Genet.,* 2006, vol. 38, 1049-1054
- LOCKHART, D. et al. *Nat. Biotech.,* 1996, vol. 14, 1675-1680
- CHEE, M. et al. *Science,* 1996, vol. 274, 610-614
- HACIA, J. et al. *Nat. Genet.,* 1996, vol. 14, 441-447
- KOZAL, M. et al. *Nat. Med.,* 1996, vol. 2, 753-759
- HADDAD, S. et al. *Surv. Ophthalmol.,* 2006, vol. 50, 306-363
- AREDS. *Arch. Ophthalmol.,* 2001, vol. 119, 1417-1436
- *Arch. Ophthalmol.,* vol. 125, 1225-1232
- *Arch. Ophthalmol.,* 2007, vol. 125, 671-679
- *Arch. Ophthalmol.,* 2005, vol. 112, 533-539
- FISHER, S. et al. *Hum. Mol. Genet.,* 2005, vol. 14, 2257-2264
- KLEIN, R. et al. *Science,* 2005, vol. 308, 385-389
- HAINES, J. et al. *Science,* 2005, vol. 308, 419-421
- EDWARDS et al. *Science,* 2005, 421-424
- HAGEMAN, G. et al. *Proc. Natl. Acad. Sci. USA,* 2005, vol. 102, 7227-7232
- RIVERA, A. et al. *Hum. Mol. Genet.,* 2005, vol. 14, 3227-3236
- GOLD, B. et al. *Nat. Genet.,* 2006, vol. 38, 458-462
- MALLER, J. et al. *Nat. Genet.,* 2006, vol. 38, 1055-1059
- MALLER, J. et al. *Nat. Genet.,* 2007, vol. 39, 1200-1201
- *Science,* 2007, vol. 316, 1331-1336
- EASTON, D. et al. *Nature,* 2007, vol. 447, 1087-1093
- SAMANI, N. et al. *N. Engl. J. Med.,* 2007, vol. 357, 443-453
- SEDDON, J. et al. *Ophthalmol.,* 2006, vol. 113, 260-266
- WILTSHIRE, S. et al. *Eur. J. Hum. Genet.,* 2006, vol. 14, 1209-1214
- BARRETT, J. et al. *Bioinformatics,* 2005, vol. 21, 263-265
- PURCELL, S. et al. *Am. J. Hum. Genet.,* 2007, vol. 81, 559-575
- VYSE, T. et al. *Genomics,* 1994, vol. 24, 90-98
- CATTERALL, C. et al. *Biochem. J.,* 1987, vol. 242, 849-856
- JHA, P. et al. *Mol. Immunol.,* 2004, vol. 44, 3997-4003